# EUROPEAN PATENT APPLICATION

(11) **EP 1 283 055 A1**
(43) Date of publication of application: **12.02.2003**
(21) Application number: 01919795.3
(22) Date of filing: 05.04.2001
(51) Int. Cl.: A61K 45/00, A61K 31/4706, A61K 31/4709, C07D 215/42, C07D 215/44, C07D 401/12, C07D 405/12, C07D 409/12, C07D 413/12, A61P 43/00, A61P 25/00, A61P 25/28, A61P 25/16

(54) **SOLUBLE BETA AMYLOID PRECURSOR PROTEIN SECRETION PROMOTERS**

(30) Priority: 07.04.2000 JP 2000111912
(71) Applicant: Takeda Chemical Industries, Ltd., Osaka-shi, Osaka 541-0045 (JP)
(72) Inventor: KAKIHANA, Mitsuru, Kobe-shi, Hyogo 651-1212 (JP); KATO, Kaneyoshi, Kawanishi-shi, Hyogo 666-0152 (JP); MORI, Masaaki, Tsukuba-shi, Ibaraki 305-0821 (JP); YAMASHITA, Toshiro, Tsukuba-shi, Ibaraki 305-0821 (JP)
(74) Representative: Lewin, John Harvey
(86) International application number: JP0102961
(87) International publication number: WO01076629

(57) **Abstract**

According to the present invention, there are provided compounds represented by formula (I): 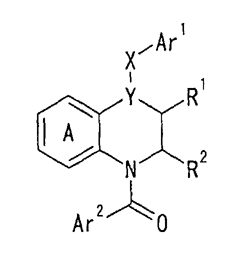 [wherein R¹ and R² represent hydrogen atom, a lower alkyl group, etc.; ring A is an optionally substituted benzene ring, X is oxygen atom, etc.; and Y represents CH or N] or salts thereof, or prodrugs thereof, and use thereof as well as processes of manufacturing these compounds. The compounds of the present invention and the like possess a potent soluble beta amyloid precursor protein secretion promoting activity and suppress the functional disorders or apoptosis of cells, in particular neurons, mediated by the secreted soluble beta amyloid precursor proteins having a neurotrophic factor like property.

## Description

### TECHNICAL FIELD

The present invention relates to pharmaceuticals, in particular, soluble beta amyloid precursor protein secretion promoters, which are useful for the treatment of neurodegenerative diseases such as Alzheimer's disease, Parkinson's disease, Down's syndrome, amyotrophic lateral sclerosis, prion disease (Creutzfeldt-Jakob's disease), Huntington's disease, neuropathy (preferably, diabetic neuropathy, etc.), etc., and further neuronal injuries associated with cerebrovascular disorders, as well as pharmaceutical compositions having both soluble beta amyloid precursor protein secretion promoting and apoptosis inhibiting activities, and the like.

### BACKGROUND ART

Drugs that prevent nerve cell death in neurodegenerative diseases such as Alzheimer's disease, Parkinson's disease, etc. to arrest the diseases have not been developed.

Alzheimer's disease is a neurodegenerative disease, which is characterized by the degeneration and loss of neuronal cells accompanied by the formation of senile plaques and neurofibrillary tangles. Senile plaques that are the most characteristic pathological finding of the brain in Alzheimer's disease are extracellular deposits of β amyloid proteins (hereinafter sometimes referred to as Aβ), which are metabolites of β amyloid precursor proteins (hereinafter sometimes referred to as βAPP) (Biochem. Biophys. Res. Commun., 122, 1311 (1984); Proc. Natl. Acad. Sci. USA, 82, 4245 (1985); J. Neurochem., 46, 1820 (1986); Neuron, 6, 487 (1991)). It is known that Aβ comprised of 40 or 42 amino acids is toxic to neurons (Science, 250, 279 (1990); Brain Res., 563, 311 (1991); J. Neurosci. 12, 376 (1992)) and induces neurofibrillary changes (Proc. Natl. Acad. Sci. USA, 90, 7789-7793 (1993)).

βAPP is not only metabolized to Aβ but also metabolized not to produce Aβ, and its metabolite or a soluble beta amyloid precursor protein (soluble APP, which is sometimes referred to as sAPP) is extracellularly secreted (Science, 248, 492 (1990); Science 248, 1122(1990)). Many studies have been reported on the neurotrophic effect of this sAPP using primary culture neurons, neuroblastoma PC 12 cells, or the like, that is, sAPP promotes extension of nerve fibers or prevents nerve cell death (Neuron, 9, 129 (1992); Neuron, 10, 243(1993); Physiol. Rev., 77, 1081 (1997)). It is also reported that sAPP level in the cerebrospinal fluid of the patient with Alzheimer's disease reduces (Lancet, 340, 453 (1992); Ann. Neurol., 32, 215 (1992); Proc. Natl. Acad. Sci. USA, 89, 2551 (1992); Alzheimer Disease and Associated Disorders, 11, 201 (1997)). Based on these findings, it is inferred that the metabolism of βAPP would tend to produce Aβ in Alzheimer's disease, not toward the production of sAPP, i.e., increased cytotoxic Aβ and its extracellular accumulation and reduction in neurotrophic factor sAPP would play extremely important role in onset of the disease. Compounds that enhance the production/secretion level of sAPP in tissues of the nerve system repair damaged nerve cells by various causes or suppress nerve cell death, via the neurotrophic factor activity of sAPP, and are thus expected to become preventive or therapeutic drugs for various neurodegenerative diseases. Besides, these compounds can also suppress the production of neurotoxic Aβ and are expected to be preventive or therapeutic drugs, in particular, for Alzheimer's disease (J. Biol. Chem., 268, 22959 (1993); Ann. New York Acad. Sci., 777, 175 (1997)).

In fact, it is known that phorbor esters, which are activators of protein kinase C (sometimes referred to as PKC), not only have a potent sAPP secretion promoting activity but also suppress the production/secretion of Aβ (Proc. Natl. Acad. Sci. USA, 87, 6003 (1990); Proc. Natl. Acad. Sci. USA, 90, 9195 (1993); J. Neurochem., 61, 2326 (1993); J. Biol. Chem. 269, 8376 (1994)).

Thus, it has been attempted to increase sAPP using various cells or brain tissue slices. In addition to the phorbol esters described above, the sAPP secretion promoting activity has been reported using, e.g., M1 muscarinic receptor agonist (Science, 258, 304 (1992); Proc. Natl. Acad. Sci. USA, 89, 10075 (1992); J. Neurosci., 15, 7442 (1995)), glutamate agonist (Proc. Natl. Acad. Sci. USA, 92, 8083 (1995), furthermore, serotonin agonist (J. Biol. Chem., 23, 4188 (1996)), etc. These agonists are considered to activate PKC, tyrosine kinase or phospholipase A2 via the corresponding receptors,

From such a viewpoint, it has been expected to discover some sAPP secretion promoters substitutable for the compounds described above.

### DISCLOSURE OF THE INVENTION

As a result of extensive studies, the present inventors have found that tetrahydro-4-quinolinamine derivatives exhibit a potent activity of promoting the sAPP secretion and inhibiting nerve cell death.

Unexpectedly, the inventors have further discovered that compounds represented by formula (I): 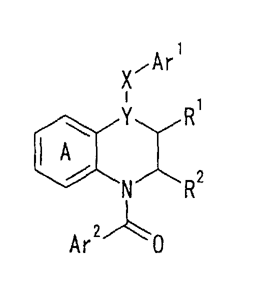 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; ring A represents a benzene ring which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y represents CH or N] or salts thereof, or prodrugs thereof, possess a soluble beta amyloid precursor protein secretion promoting activity, and have found that these compounds or the like have excellent activities of inhibiting apoptosis and improving the neuronal dysfunction. That is, they have found the presence of a pharmaceutical composition having both a soluble beta amyloid precursor protein secretion promoting activity and an apoptosis inhibiting activity. Moreover, the inventors have found that because of these activities, these compounds are useful as agents for the prevention/treatment of neurodegenerative diseases such as Alzheimer's disease, Parkinson's disease, Down's syndrome, prion disease, neuropathy (preferably, diabetic neuropathy, etc.), etc., cerebrovascular dementia, or neuronal injuries associated with cerebrovascular disorders. Based on these findings, the present invention has been accomplished.

Thus, the present invention relates to:
(1) A pharmaceutical composition comprising a compound having a soluble beta amyloid precursor protein secretion promoting activity and an apoptosis inhibiting activity, or a salt thereof, or a prodrug thereof;
(2) The pharmaceutical composition according to (1), comprising a compound containing:
   1) a partial structure represented by formula (a): 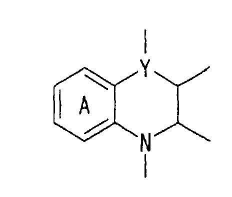 [wherein ring A represents a benzene ring which may optionally be substituted, and Y represents CH or N];
   2) a partial structure represented by formula (b): 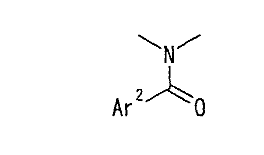 [wherein Ar² represents an aromatic group which may optionally be substituted]; or,
   3) a partial structure represented by formula (c): 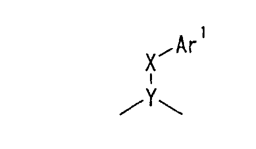 [wherein Ar¹ represents an aromatic group which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y has the same significance as defined above]; or a salt thereof, or a prodrug thereof;
(3) An agent for promoting soluble beta amyloid precursor protein secretion comprising a compound represented by formula (I): 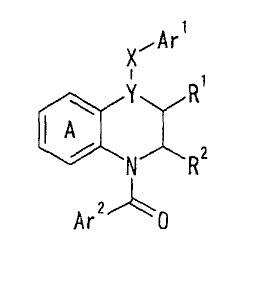 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; ring A represents a benzene ring which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y represents CH or N] or a salt thereof, or a prodrug thereof;
(4) The agent according to (3), wherein R¹ is hydrogen atom;
(5) The agent according to (3), wherein R² is hydrogen atom or a lower alkyl group which may optionally be substituted;
(6) The agent according to (3), wherein R² is hydrogen atom or a C₁₋₆ alkyl group;
(7) The agent according to (3), wherein Ar¹ is a phenyl group which may optionally be substituted or a quinolyl group which may optionally be substituted;
(8) The agent according to (3), wherein Ar¹ is a phenyl group which may optionally be substituted;
(9) The agent according to (3), wherein Ar¹ is a phenyl group which may optionally be substituted with a halogen atom, a C₁₋₃ alkylenedioxy group, an optionally halogenated C₁₋₆ alkyl group or an optionally halogenated C₁₋₆ alkoxy group;
(10) The agent according to (3), wherein Ar² is a monocyclic aromatic group which may optionally be substituted;
(11) The agent according to (3), wherein Ar² is a phenyl, furyl, thienyl or pyridyl group, each of which may optionally be substituted;
(12) The agent according to (3), wherein Ar² is a phenyl, furyl, thienyl or pyridyl group, each of which may optionally be substituted with a halogen atom, a C₁₋₃ alkylenedioxy group, an optionally halogenated C₁₋₆ alkyl group or an optionally halogenated C₁₋₆ alkoxy group;
(13) The agent according to (3), wherein the ring A is unsubstituted benzene ring;
(14) The agent according to (3), wherein X is NR⁵ (R⁵ has the same significance as defined in (3) above) and Y is CH;
(15) The agent according to (3), wherein R¹ is hydrogen atom, R² is methyl, Ar¹ is a phenyl group which may optionally be substituted, Ar² is 3,4-dimethoxyphenyl group, the ring A is unsubstituted benzene ring, X is NR⁵ (R⁵ has the same significance as defined in (3) above) and Y is CH;
(16) An agent for inhibiting apoptosis comprising a compound represented by formula (I): 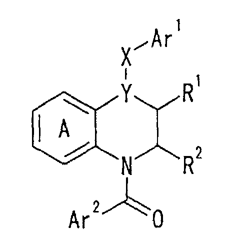 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; ring A represents a benzene ring which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y represents CH or N] or a salt thereof, or a prodrug thereof;
(17) The agent according to (3) or (16), which is an agent for improving neuronal dysfunction;
(18) The agent according to (3) or (16), which is an agent for the prevention/treatment of a neurodegenerative disease;
(19) The agent according to (18), wherein the neurodegenerative disease is Alzheimer's disease, Parkinson's disease, prion disease or neuropathy;
(20) Use of a compound having a soluble beta amyloid precursor protein secretion promoting activity and an apoptosis inhibiting activity, or a salt thereof, or a prodrug thereof, for the production of a pharmaceutical having an activity of improving neuronal dysfunction;
(21) Use of a compound having a soluble beta amyloid precursor protein secretion promoting activity and an apoptosis inhibiting activity, or a salt thereof, or a prodrug thereof, for the production of an agent for the prevention/treatment of a neurodegenerative disease;
(22) Use of a compound having a soluble beta amyloid precursor protein secretion promoting activity and an apoptosis inhibiting activity, or a salt thereof, or a prodrug thereof, for the production of an agent for the prevention/treatment of Alzheimer's disease, Parkinson's disease, prion disease or neuropathy;
(23) Use of a compound containing:
   1) a partial structure represented by formula (a): 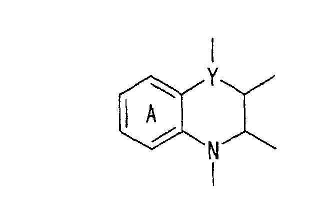 [wherein ring A represents a benzene ring which may optionally be substituted and Y represents CH or N];
   2) a partial structure represented by formula (b): 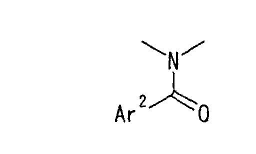 [wherein Ar² represents an aromatic group which may optionally be substituted]; or,
   3) a partial structure represented by formula (c): 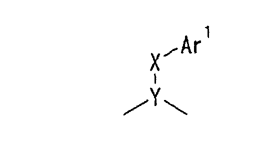 [wherein Ar¹ represents an aromatic group which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y has the same significance as defined above]; or a salt thereof, or a prodrug thereof, for the production of a pharmaceutical having a soluble beta amyloid precursor protein secretion promoting activity and an apoptosis inhibiting activity;
(24) Use of a compound represented by formula (I): 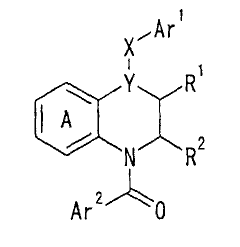 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; ring A represents a benzene ring which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y represents CH or N] or a salt thereof, or a prodrug thereof, for the production of a pharmaceutical having a soluble beta amyloid precursor protein secretion promoting activity and/or an apoptosis inhibiting activity;
(25) A method of improving neuronal dysfunction, which comprises administering to a mammal a compound having a soluble beta amyloid precursor protein secretion promoting activity and an apoptosis inhibiting activity, or a salt thereof, or a prodrug thereof;
(26) A method for the prevention/treatment of a neurodegenerative disease, which comprises administering to a mammal a compound having a soluble beta amyloid precursor protein secretion promoting activity and an apoptosis inhibiting activity, or a salt thereof, or a prodrug thereof;
(27) The method for the prevention/treatment according to (26), wherein the neurodegenerative disease is Alzheimer's disease, Parkinson's disease, prion disease or neuropathy;
(28) A method of promoting soluble beta amyloid precursor protein secretion and/or inhibiting apoptosis, which comprises administering to a mammal a compound containing:
   1) a partial structure represented by formula (a): 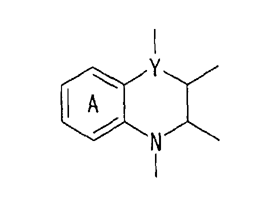 [wherein ring A represents an optionally substituted benzene ring and Y represents CH or N];
   2) a partial structure represented by formula (b): 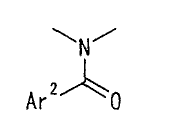 [wherein Ar² represents an aromatic group which may optionally be substituted]; or,
   3) a partial structure represented by formula (c): 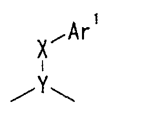 [wherein Ar¹ represents an aromatic group which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y has the same significance as defined above]; or a salt thereof, or a prodrug thereof;
(29) A method of promoting soluble beta amyloid precursor protein secretion and/or inhibiting apoptosis, which comprises administering to a mammal a compound represented by formula (I): 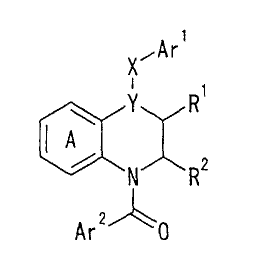 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; ring A represents a benzene ring which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y represents CH or N] or a salt thereof, or a prodrug thereof;
(30) A compound represented by formula (II): 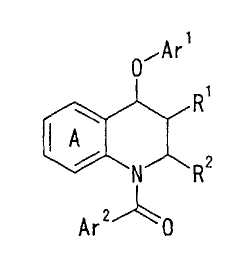 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; and ring A represents a benzene ring which may optionally be substituted] or a salt thereof, or a prodrug thereof;
(31) The compound or a salt thereof, or a prodrug thereof, according to (30), wherein R¹ is hydrogen atom, R² is methyl, Ar¹ is a phenyl group which may optionally be substituted, Ar² is 3,4-dimethoxyphenyl group, and the ring A is unsubstituted benzene ring;
(32) A pharmaceutical composition comprising the compound or a salt thereof, or a prodrug thereof, according to (30);
(33) A process of manufacturing a compound represented by formula (II): 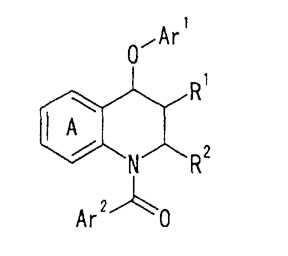 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; and ring A represents a benzene ring which may optionally be substituted] or a salt thereof, or a prodrug thereof, which comprises condensation-reacting a compound represented by formula: 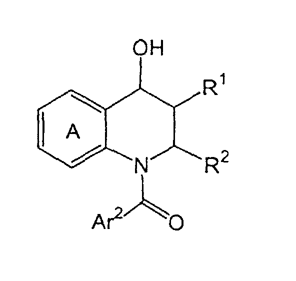 [wherein R¹, R², Ar² and ring A have the same significance as defined above] or a salt thereof, with a compound represented by formula:

   Ar¹OH

   [wherein Ar¹ has the same significance as defined above] or a salt thereof;
(34) A compound represented by formula (III): 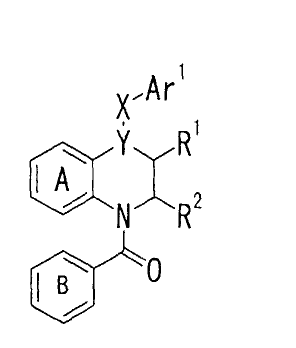 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; Ar¹ represents an aromatic group which may optionally be substituted; ring A represents a benzene ring which may optionally be substituted; ring B represents a benzene ring, which may optionally be substituted with an optionally halogenated C₁₋₆ alkoxy group, hydroxy group or a C₁₋₃ alkylenedioxy group; X represents CR³R⁴ or NR⁵ (wherein R³, R⁴ or R⁵, which may be the same or different, each represents hydrogen atom or a lower alkyl group which may optionally be substituted); and Y represents CH or N (provided that the compounds wherein X is NH, Y is CH and Ar¹ is unsubstituted phenyl group are excluded)] or a salt thereof, or a prodrug thereof;
(35) The compound or a salt thereof, or a prodrug thereof, according to (34), wherein R¹ is hydrogen atom;
(36) The compound or a salt thereof, or a prodrug thereof, according to (34), wherein R² is hydrogen atom or a lower alkyl group which may optionally be substituted;
(37) The compound or a salt thereof, or a prodrug thereof, according to (34), wherein Ar¹ is a monocyclic aromatic group which may optionally be substituted;
(38) The compound or a salt thereof, or a prodrug thereof, according to (34), wherein Ar¹ is a phenyl group which may optionally be substituted;
(39) The compound or a salt thereof, or a prodrug thereof, according to (34), wherein ring A is unsubstituted benzene ring;
(40) The compound or a salt thereof, or a prodrug thereof, according to (34), wherein X is NR⁵ (wherein R⁵ has the same significance as defined in (34) above) and Y is CH;
(41) The compound or a salt thereof, or a prodrug thereof, according to (34), wherein R¹ is hydrogen atom, R² is methyl, Ar¹ is a phenyl group which may optionally be substituted, ring A is unsubstituted benzene ring, X is NR⁵ (wherein R⁵ has the same significance as defined in (34) above) and Y is CH N (provided that those wherein X is NH, Y is CH and Ar¹ is unsubstituted phenyl group are excluded);
(42) A pharmaceutical composition comprising the compound or a salt thereof, or a prodrug thereof, according to (34);
(43) A process of manufacturing a compound represented by formula: 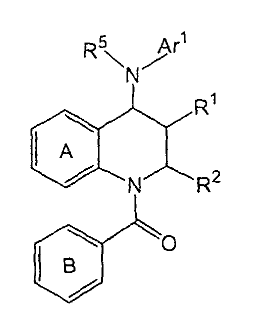 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; R⁵ represents hydrogen atom or a lower alkyl group which may optionally be substituted; ring A represents a benzene ring which may optionally be substituted; ring B represents a benzene ring, which may optionally be substituted with an optionally halogenated C₁₋₆ alkoxy group, hydroxy group or a C₁₋₃ alkylenedioxy group; and, Ar¹ represents an aromatic group which may optionally be substituted (provided that the compounds wherein R⁵ is H and Ar¹ is unsubstituted phenyl group are excluded)] or a salt thereof, or a prodrug thereof, which comprises substituting a compound represented by formula: 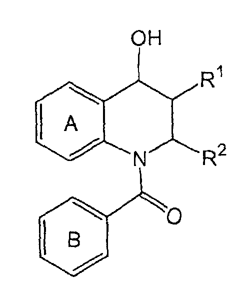 [wherein R¹, R², ring A and ring B have the same significance as defined above] or a salt thereof, with a compound represented by formula:

   Ar¹ - NR⁵H

   [wherein Ar¹ and R⁵ have the same significance as defined above] or a salt thereof;
(44) A process of manufacturing a compound represented by formula: 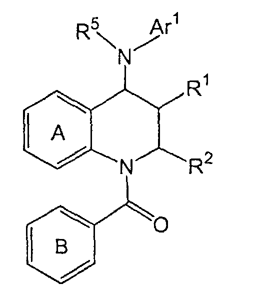 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; R⁵ represents hydrogen atom or a lower alkyl group which may optionally be substituted; ring A represents a benzene ring which may optionally be substituted; ring B represents a benzene ring, which may optionally be substituted with an optionally halogenated C₁₋₆ alkoxy group, hydroxy group or a C₁₋₃ alkylenedioxy group; and Ar¹ represents an aromatic group which may optionally be substituted (provided that the compounds wherein R⁵ is H and Ar¹ is unsubstituted phenyl group are excluded)] or a salt thereof, or a prodrug thereof, which comprises reacting a compound represented by formula: 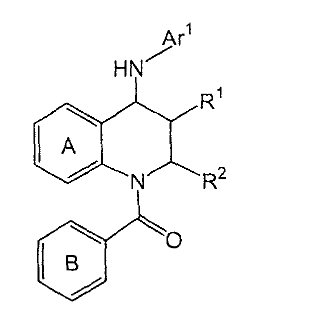 [wherein R¹, R², Ar¹, ring A and ring B have the same significance as defined above] or a salt thereof, with a compound represented by formula:

   R⁵Z

   [wherein R⁵ has the same significance as defined above and Z represents a reactive substituent] or a salt thereof;
(45) A process of manufacturing a compound represented by formula (III): 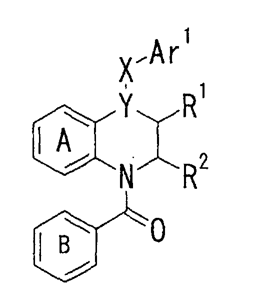 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; Ar¹ represents an aromatic group which may optionally be substituted; X represents CR³R⁴ or NR⁵ (wherein R³, R⁴ or R⁵, which may be the same or different, each represents hydrogen atom or a lower alkyl group which may optionally be substituted); Y represents CH or N (provided that the compounds wherein X is NH, Y is CH and Ar¹ is unsubstituted phenyl group are excluded); ring A represents a benzene ring which may optionally be substituted; and ring B represents a benzene ring, which may optionally be substituted with an optionally halogenated C₁₋₆ alkoxy group, hydroxy group or a C₁₋₃ alkylenedioxy group] or a salt thereof, or a prodrug thereof, which comprises reacting a compound represented by formula: 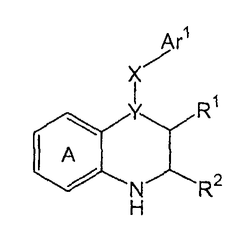 [wherein R¹, R², Ar¹, X, Y and ring A have the same significance as defined above] or a salt thereof, with a compound represented by formula: 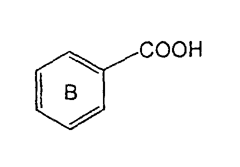 [wherein ring B has the same significance as defined above] or a salt thereof;
(46) A process of manufacturing a compound represented by formula: 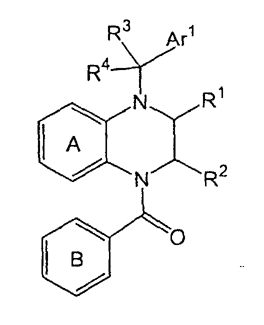 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; R³ and R⁴, which may be the same or different, each represents hydrogen atom or a lower alkyl group which may optionally be substituted; ring A represents a benzene ring which may optionally be substituted; ring B represents a benzene ring, which may optionally be substituted with an optionally halogenated C₁₋₆ alkoxy group, hydroxy group or a C₁₋₃ alkylenedioxy group; and Ar¹ represents an aromatic group which may optionally be substituted] or a salt thereof, or a prodrug thereof, which comprises reacting a compound represented by formula: 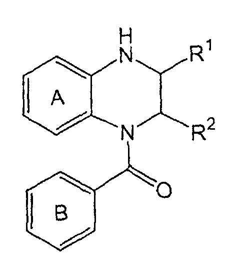 [wherein R¹, R², ring A and ring B have the same significance as defined above] or a salt thereof, with a compound represented by formula:

   Ar¹CR³R⁴Z

   [wherein Ar¹, R³ and R⁴ have the same significance as defined above; and Z represents a reactive substituent] or a salt thereof; and the like.

### BRIEF DESCRIPTION OF THE DRAWINGS

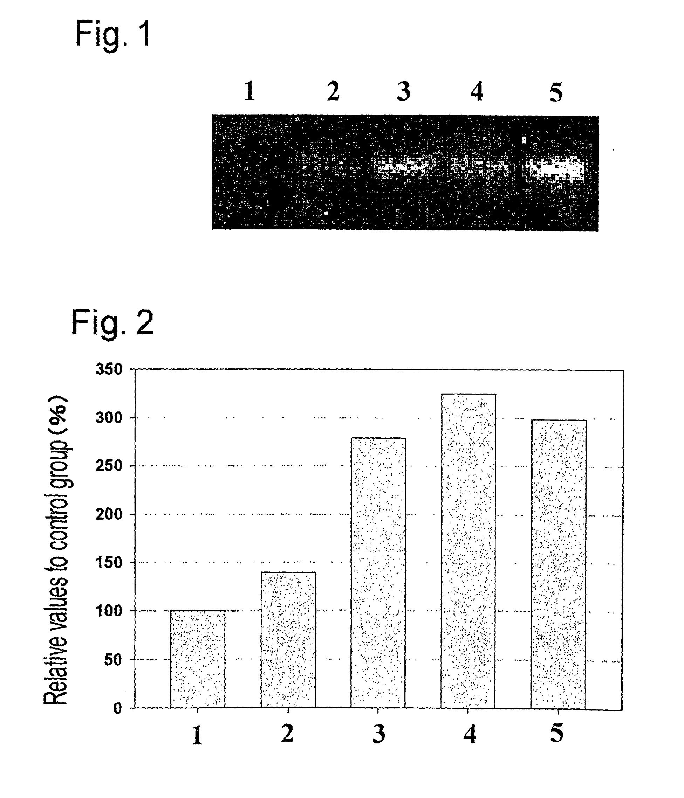

FIG. 1 shows the results of the compounds of the present invention assayed in terms of the soluble beta amyloid precursor protein secretion promoting activity by western blotting, wherein lanes 1, 2, 3, 4 and 5 designate the group added with no compound, the group added with Compound 1-2, the group added with Compound 2-2, the group added with Compound 1-3 and the group added with Compound 2-3, respectively.
FIG. 2 is a graph showing the results of the compounds of the present invention on the soluble beta amyloid precursor protein secretion promoting activity (a mean value in 3 wells), wherein the numerals on the X-axis correspond to the respective lanes in FIG. 1.

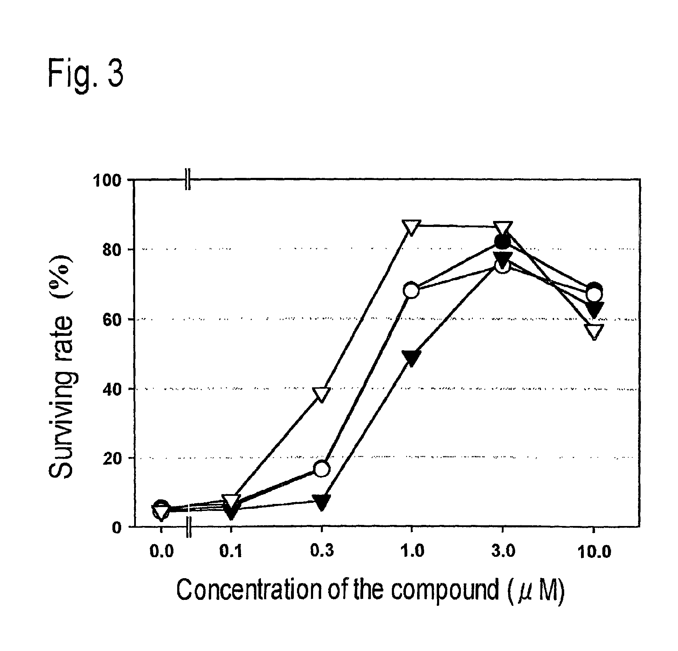

FIG. 3 is a graph showing concentration dependency of glutamate-induced cell death (a mean value in 3 wells) by the compounds of the present invention in PC12h cells, wherein -•-, -○-, -▼- and -∇- designate the group added with Compound 1-2, the group added with Compound 2-2, the group added with Compound 1-3 and the group added with Compound 2-3, respectively.

### PREFERRED EMBODIMENTS OF THE INVENTION

The pharmaceutical composition of the present invention comprises compounds having the soluble beta amyloid precursor protein secretion promoting activity and apoptosis inhibiting activity, or salts thereof, or prodrugs thereof.

Preferably, compounds containing the partial structures represented by the formulae (a), (b) and (c) described above are employed as these compounds.

In the partial structure represented by the formula (c) above, the "lower alkyl group" in the optionally substituted lower alkyl group shown by R³, R⁴ and R⁵ includes a C₁₋₆ alkyl (e.g., methyl, ethyl, propyl, isopropyl, butyl, isobutyl, sec-butyl, tert-butyl, pentyl, hexyl, etc.) and the like. Preferred examples are methyl, ethyl and propyl.

For R³, R⁴ and R⁵, hydrogen atom is preferably used.

In the "optionally substituted lower alkyl group" shown by R³, R⁴ and R⁵, examples of "substituents" are a halogen atom (e.g., fluorine, chlorine, bromine, iodine, etc.), a C₁₋₃ alkylenedioxy (e.g., methylenedioxy, ethylenedioxy, etc.), nitro, cyano, a C₁₋₆ alkyl-C₆₋₁₀ aryl-C₂₋₆ alkenyl (e.g., methylphenylethenyl, etc.), an optionally halogenated C₃₋₆ cycloalkyl, an optionally halogenated C₁₋₆ alkoxy, an optionally halogenated C₁₋₆ alkylthio, an optionally substituted C₆₋₁₀ aryloxy, a C₆₋₁₀ aryl-C₇₋₁₆ aralkyloxy (e.g., phenylbenzyloxy, etc.), amino, a mono-C₁₋₆ alkylamino (e.g., methylamino, ethylamino, propylamino, isopropylamino, butylamino, etc.), a di-C₁₋₆ alkylamino (e.g., dimethylamino, diethylamino, dipropylamino, dibutylamino, ethylmethylamino, etc.), an optionally substituted 5- to 7-membered saturated cyclic amino, acyl, acylamino, acyloxy, etc. Among others, preferred are a halogen atom, a C₁₋₃ alkylenedioxy, a C₁₋₆ alkyl-C₆₋₁₀ aryl-C₂₋₆ alkenyl, an optionally halogenated C₁₋₆ alkoxy, an optionally substituted C₆₋₁₀ aryloxy, acyl, acyloxy, etc.

In the "optionally substituted lower alkyl group" shown by R³, R⁴ and R⁵, the "lower alkyl group" may have, e.g., 1 to 13, preferably 1 to 5 substituents described above at the position where the lower alkyl group can be substituted. When the number of the substituents are 2 or more, the respective substituents may be the same or different.

In the "optionally substituted C₆₋₁₀ aryloxy" described above, examples of the "C₆₋₁₀ aryloxy" include phenyloxy, naphthyloxy, etc.

Examples of the "substituent" include 1 to 5 substitutions selected from a halogen atom (e.g., fluorine, chlorine, bromine, iodine, etc.), a C₁₋₃ alkylenedioxy (e.g., methylenedioxy, ethylenedioxy, etc.), nitro, cyano, an optionally halogenated C₁₋₆ alkyl, an optionally halogenated C₃₋₆ cycloalkyl, an optionally halogenated C₁₋₆ alkoxy, an optionally halogenated C₁₋₆ alkylthio, hydroxy, amino, a mono-C₁₋₆ alkylamino (e.g., methylamino, ethylamino, propylamino, isopropylamino, butylamino, etc.), a di-C₁₋₆ alkylamino (e.g., dimethylamino, diethylamino, dipropylamino, dibutylamino, ethylmethylamino, etc.), formyl, carboxy, carbamoyl, an optionally halogenated C₁₋₆ alkyl-carbonyl, a C₁₋₆ alkoxy-carbonyl (e.g., methoxycarbonyl, ethoxycarbonyl, propoxycarbonyl, tert-butoxycarbonyl, etc.), a mono-C₁₋₆ alkyl-carbamoyl (e.g., methylcarbamoyl, ethylcarbamoyl, etc.), a di-C₁₋₆ alkyl-carbamoyl (e.g., dimethylcarbamoyl, diethylcarbamoyl, ethylmethylcarbamoyl, etc.), an optionally halogenated C₁₋₆ alkylsulfonyl, formylamino, an optionally halogenated C₁₋₆ alkyl-carboxamido, a C₁₋₆ alkoxy-carboxamido (e.g., methoxycarboxamido, ethoxycarboxamido, propoxycarboxamido, butoxycarboxamido, etc.), a C₁₋₆ alkylsulfonylamino (e.g., methylsulfonylamino, ethylsulfonylamino, etc.), a C₁₋₆ alkyl-carbonyloxy (e.g., acetoxy, propanoyloxy, etc.), a C₁₋₆ alkoxy-carbonyloxy (e.g., methoxycarbonyloxy, ethoxycarbonyloxy, propoxycarbonyloxy, butoxycarbonyloxy, etc.), a mono-C₁₋₆ alkyl-carbamoyloxy (e.g., methylcarbamoyloxy, ethylcarbamoyloxy, etc.), a di-C₁₋₆ alkyl-carbamoyloxy (e.g., dimethylcarbamoyloxy, diethylcarbamoyloxy, etc.), and the like.

In the "optionally substituted 5- or 7-membered saturated cyclic amino" described above, examples of the "5- or 7-membered saturated cyclic amino" include morpholino, thiomorpholine, piperadin-1-yl, piperidino, pyrrolidin-1-yl, hexamethylenimin-1-yl, etc.

In the "optionally substituted 5- or 7-membered saturated cyclic amino" described above, examples of "substituents" include I to 3 substitutions selected from a C₁₋₆ alkyl, an optionally substituted C₆₋₁₄ aryl, an optionally substituted C₇₋₁₉ aralkyl, an optionally substituted 5- to 10-membered aromatic heterocyclic group, an optionally substituted C₆₋₁₀ aryl-carbonyl, an optionally halogenated C₁₋₆ alkyl-carbonyl, an optionally halogenated C₁₋₆ alkylsulfonyl, etc.

In the "optionally substituted C₆₋₁₄ aryl," examples of "C₆₋₁₄ aryl" include phenyl, a naphthyl such as 1-naphthyl, 2-naphthyl, etc., an indenyl such as 2-indenyl, etc., an anthryl such as 2-anthryl, etc., with phenyl or the like being preferred.

In the "optionally substituted C₇₋₁₉ aralkyl," examples of "C₇₋₁₉ aralkyl" include benzyl, phenethyl, diphenylmethyl, triphenylmethyl, 1-naphthylmethyl, 2-naphthylmethyl, 2,2-diphenylethyl, 3-phenylpropyl, 4-phenylbutyl, 5-phenylpentyl, etc. Benzyl or the like is preferred.

In the "optionally substituted 5- or 10-membered aromatic heterocyclic group" examples of the "5- or 10-membered aromatic heterocyclic group" include a pyridyl such as 2-, 3- or 4-pyridyl, etc., an indolyl such as 1-, 2- or 3-indolyl, etc., a thienyl such as 2-or 3-thienyl, etc. Preferably, the heterocyclic group is a pyridyl such as 2-, 3- or 4-pyridyl, etc.

In the "optionally substituted C₇₋₁₉ aralkyl," examples of the "C₆₋₁₀ aryl-carbonyl" include benzoyl, 1-naphthoyl, 2-naphthoyl, etc.

In each of these "optionally substituted C₆₋₁₄ aryl," "optionally substituted C₇₋₁₉ aralkyl," "optionally substituted 5- or 10-membered aromatic heterocyclic group" and "optionally substituted C₆₋₁₀ aryl-carbonyl," examples of the "substituents" include 1 to 5 substituents selected from a halogen atom (e.g., fluorine, chlorine, bromine, iodine, etc.), a C₁₋₃ alkylenedioxy (e.g., methylenedioxy, ethylenedioxy, etc.), nitro, cyano, an optionally halogenated C₁₋₆ alkyl, an optionally halogenated C₃₋₆ cycloalkyl, an optionally halogenated C₁₋₆ alkoxy, an optionally halogenated C₁₋₆ alkylthio, hydroxy, amino, a mono-C₁₋₆ alkylamino (e.g., methylamino, ethylamino, propylamino, isopropylamino, butylamino, etc.), a di-C₁₋₆ alkylamino (e.g., dimethylamino, diethylamino, dipropylamino, dibutylamino, ethylmethylamino, etc.), formyl, carboxy, carbamoyl, an optionally halogenated C₁₋₆ alkyl-carbonyl, a C₁₋₆ alkoxy-carbonyl (e.g., methoxycarbonyl, ethoxycarbonyl, propoxycarbonyl, tert-butoxycarbonyl, etc.), a mono-C₁₋₆ alkyl-carbamoyl (e.g., methylcarbamoyl, ethylcarbamoyl, etc.), a di-C₁₋₆ alkyl-carbamoyl (e.g., dimethylcarbamoyl, diethylcarbamoyl, ethylmethylcarbamoyl, etc.), an optionally halogenated C₁₋₆ alkylsulfonyl, formylamino, an optionally halogenated C₁₋₆ alkyl-carboxamido, a C₁₋₆ alkoxy-carboxamido (e.g., methoxycarboxamido, ethoxycarboxamido, propoxycarboxamido, butoxycarboxamido, etc.), a C₁₋₆ alkylsulfonylamino (e.g., methylsulfonylamino, ethylsulfonylamino, etc.), a C₁₋₆ alkyl-carbonyloxy (e.g., acetoxy, propanoyloxy, etc.), a C₁₋₆ alkoxy-carbonyloxy (e.g., methoxycarbonyloxy, ethoxycarbonyloxy, propoxycarbonyloxy, butoxycarbonyloxy, etc.), a mono-C₁₋₆ alkyl-carbamoyloxy (e.g., methylcarbamoyloxy, ethylcarbamoyloxy, etc.), a di-C₁₋₆ alkyl-carbamoyloxy (e.g., dimethylcarbamoyloxy, diethylcarbamoyloxy, etc.), and the like.

In the "optionally substituted lower alkyl group" shown by R³, R⁴ and R⁵ described above, the "acyl" as its "substituent" and the "acyl" in "acylamino" and "acyloxy" include formyl, carboxy, carbamoyl, an optionally halogenated C₁₋₆ alkyl-carbonyl, a C₁₋₆ alkoxy-carbonyl (e.g., methoxycarbonyl, ethoxycarbonyl, propoxycarbonyl, tert-butoxycarbonyl, etc.), an optionally substituted C₆₋₁₀ aryl-carbonyl, an optionally substituted C₆₋₁₀ aryloxy-carbonyl, an optionally substituted C₇₋₁₆ aralkyloxy-carbonyl, an optionally substituted 5- or 6-membered heterocyclic carbonyl, a mono-C₁₋₆ alkyl-carbamoyl, a di-C₁₋₆ alkyl-carbamoyl (e.g., dimethylcarbamoyl, diethylcarbamoyl, ethylmethylcarbamoyl, etc.), an optionally substituted C₆₋₁₀ aryl-carbamoyl, an optionally substituted 5- or 6-membered heterocyclic carbamoyl, an optionally halogenated C₁₋₆ alkylsulfonyl, an optionally substituted C₆₋₁₀ arylsulfonyl, etc. Among others, preferred are an optionally halogenated C₁₋₆ alkyl-carbonyl, a C₁₋₆ alkoxy-carbonyl (e.g., ethoxycarbonyl, etc.), an optionally substituted C₆₋₁₀ aryl-carbonyl, an optionally substituted C₆₋₁₀ arylsulfonyl, and the like.

In the "optionally substituted C₆₋₁₀ aryl-carbonyl" described above, examples of the "C₆₋₁₀ aryl-carbonyl" include benzoyl, 1-naphthoyl, 2-naphthoyl, etc.

In the "optionally substituted C₆₋₁₀ aryloxy-carbonyl" described above, examples of the "C₆₋₁₀ aryloxy-carbonyl" include phenoxycarbonyl, etc.

In the "optionally substituted C₇₋₁₆ aralkyloxy-carbonyl" described above, examples of the "C₇₋₁₆ aralkyloxy-carbonyl" include benzyloxycarbonyl, phenethyloxycarbonyl, etc.

In the "optionally substituted 5- or 6-membered heterocyclic carbonyl" described above, examples of the "5- or 6-membered heterocyclic carbonyl" include nicotinoyl, isonicotinoyl, a thenoyl such as 2-thenoyl or 3-thenoyl, etc., a furoyl such as 2-furoyl or 3-furoyl, etc., morpholinocarbonyl, piperidinocarbonyl, pyrrolidin-1-ylcarbonyl, etc.

In the "optionally substituted C₆₋₁₀ aryl-carbamoyl" described above, examples of the "C₆₋₁₀ aryl-carbamoyl" include phenylcarbamoyl, 1-phenylcarbamoyl, 1-naphthylcarbamoyl, 2-naphthylcarbamoyl, etc.

In the "optionally substituted 5- or 6-membered heterocyclic carbamoyl" described above, examples of the "5- or 6-membered heterocyclic carbamoyl" include a pyridylcarbamoyl such as 2-pyridylcarbamoyl, 3-pyridylcarbamoyl or 4-pyridylcarbamoyl, etc., a thienylcarbamoyl such as 2-thienylcarbamoyl or 3-thienylcarbamoyl, etc.

In the "optionally substituted C₆₋₁₀ arylsulfonyl" described above, examples of the "C₆₋₁₀ arylsulfonyl" include benzenesulfonyl, 1-naphthalenesulfonyl, 2-naphthalenesulfonyl, etc.

In these "optionally substituted C₆₋₁₀ arylcarbonyl," "optionally substituted C₆₋₁₀ aryloxycarbonyl," "optionally substituted C₇₋₁₆ aralkyloxy-carbonyl," "optionally substituted 5- or 6-membered carbonyl," "optionally substituted C₆₋₁₀ arylcarbamoyl," "optionally substituted 5- or 6-membered heterocyclic carbamoyl" and "optionally substituted C₆₋₁₀ arylsulfonyl," examples of the "substituent" are 1 to 5 substituents, preferably 1 to 3 substituents selected from a halogen atom, a C₁₋₃ alkylenedioxy, nitro, cyano, an optionally halogenated C₁₋₆ alkyl, an optionally halogenated C₁₋₆ alkoxy, an optionally halogenated C₁₋₆ alkylthio, hydroxy, amino, a mono-C₁₋₆ alkylamino, a di-C₁₋₆ alkylamino, formyl, carboxy, carbamoyl, an optionally halogenated C₁₋₆ alkyl-carbonyl, a C₁₋₆ alkoxy-carbonyl, a mono-C₁₋₆ alkyl-carbamoyl, di-C₁₋₆ alkyl-carbamoyl, an optionally halogenated C₁₋₆ alkylsulfonyl, formylamino, an optionally halogenated C₁₋₆ alkyl-carboxamido, a C₁₋₆ alkoxycarboxamido, a C₁₋₆ alkylsulfonylamino, a C₁₋₆ alkyl-carbonyloxy, a C₁₋₆ alkoxy-carbonyloxy, a mono-C₁₋₆ alkyl-carbamoyloxy and a di-C₁₋₆ alkyl-carbamoyloxy. Among others, a halogen atom, an optionally halogenated C₁₋₆ alkyl, an optionally halogenated C₁₋₆ alkoxy, and the like are preferred.

In the "lower alkyl group which may optionally be substituted" shown by R³, R⁴ and R⁵ described above, the "acylamino" as its "substituent" includes an amino substituted with 1 or 2 "acyl" groups described in detail, e.g., on the "substituents" for the "aromatic group which may optionally be substituted" described above, and preferred examples are formylamino, an optionally halogenated C₁₋₆ alkyl-carboxamido, an optionally substituted C₆₋₁₀ aryl-carboxamido (e.g., phenylcarboxamido, naphthylcarboxamido, etc.), a C₁₋₆ alkoxy-carboxamido (e.g., methoxycarboxamido, ethoxycarboxamido, propoxycarboxamido, butoxycarboxamido, etc.), a C₁₋₆ alkylsulfonylamino (e.g., methylsulfonylamino, ethylsulfonylamino, etc.), and the like.

In the "lower alkyl group which may optionally be substituted" shown by R³, R⁴ and R⁵ described above, the "acyloxy" as its "substituent" includes an oxy substituted with one "acyl" described in detail, e.g., on the "substituents" for the "optionally substituted aromatic group" described above, and preferred examples include a C₁₋₆ alkyl-carbonyloxy (e.g., acetoxy, propanoyloxy, etc.), an optionally substituted C₆₋₁₀ aryl-carbonyloxy (e.g., benzoyloxy, 1-naphthoyloxy, 2-naphthoyloxy, etc.), a C₁₋₆ alkoxy-carbonyloxy (e.g., methoxycarbonyloxy, ethoxycarbonyloxy, propoxycarbonyloxy, butoxycarbonyloxy, etc.), a mono-C₁₋₆ alkyl-carbamoyloxy (e.g., methylcarbamoyloxy, ethylcarbamoyloxy, etc.), a di-C₁₋₆ alkyl-carbamoyloxy (e.g., dimethylcarbamoyloxy, diethylcarbamoyloxy, etc.), an optionally substituted C₆₋₁₀ aryl-carbamoyloxy (e.g., phenylcarbamoyloxy, naphthylcarbamoyloxy, etc.), nicotinoyloxy, and the like.

The "aromatic group" for the "aromatic group which may optionally be substituted" shown by Ar² in the partial structure represented by formula (b) above and Ar¹ in the partial structure represented by formula (c) above include, for example, a monocyclic aromatic group, a ring-assembled aromatic group, a ring-fused aromatic group, etc.

Examples of the "monocyclic aromatic group" described above include a monovalent group formed by removing one optional hydrogen atom from benzene ring, 5-or 6-membered aromatic heterocyclic ring, and the like.

The "5- or 6-membered aromatic heterocyclic ring" includes a 5- or 6-membered aromatic heterocyclic ring containing carbon atoms and at least one (e.g., 1 to 3) of other hetero atoms selected from nitrogen, sulfur and oxygen atoms. Specific examples are thiophene, furan, pyrrole, imidazole, pyrazole, thiazole, oxazole, pyridine, pyrazine, pyrimidine, pyridazine, etc.

Specific examples of the "monocyclic aromatic group" are phenyl; a thienyl such as 2- or 3-thienyl, etc.; a furyl such as 2- or 3-furyl, etc.; a pyrrolyl such as 1-, 2- or 3-pyn-olyl, etc.; an imidazolyl such as 2- or 4-imidazolyl, etc.; a pyrazolyl such as 3- or 4-pyrazolyl, etc.; a thiazolyl such as 2-, 4- or 5-thiazolyl, etc.; an oxazolyl such as 2-4- or 5-oxazolyl, etc.; a pyridyl such as 2-. 3- or 4-pyridyl, etc.; a pyrazinyl such as 2-pyrazinyl, etc.; a pyrimidinyl such as 2-4- or 5-pyrimidinyl; a pyridazinyl such as 3- or 4-pyridazinyl, etc.

The "ring-assembled aromatic group" described above includes a group formed by removing one optional hydrogen atom from an aromatic ring assembly, in which 2 or more (preferably 2 or 3) aromatic rings are directly connected with each other by single bond(s) and the number of such direct ring bonds is less by one than the number of the aromatic rings involved.

The "aromatic ring" includes an aromatic hydrocarbon, an aromatic heterocyclic ring, etc.

Examples of the "aromatic hydrocarbon" include a monocyclic or fused polycyclic (bi- or tri-cyclic) aromatic hydrocarbon having 6 to 14 carbon atoms (e.g., benzene, naphthalene, indene, anthracene, etc.), and the like.

The "aromatic heterocyclic ring" includes, for example, 5- to 14-membered, preferably 5- to 10-membered aromatic heterocyclic rings, etc. containing 1 or more (e.g., 1 to 4) hetero atoms selected from the group consisting of nitrogen, sulfur and oxygen atoms in addition to carbon atoms. Specifically, there are an aromatic heterocyclic ring such as thiophene, benzothiophene, benzofuran, benzimidazole, benzoxazole, benzothiazole, benzisothiazole, naphtho[2,3-b]thiophene, furan, phenoxathiin, pyrrole, imidazole, pyrazole, oxazole, isoxazole, an oxadiazole such as 1,2,4-oxadiazole or 1,3,4-oxadiazole, etc., a thiadizole such as 1,2,4-thiadiazole or 1,3,4-thiadiazole, etc., pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, furazan, phenoxazine, phthalimide, etc.; a ring formed by fusion of these rings (preferably a monocyclic ring) to 1 or more (preferably 1 or 2) aromatic rings (e.g., benzene ring, etc.), and the like.

The aromatic ring assembly wherein these aromatic rings are directly bonded to each other via a single bond includes, for example, an aromatic ring assembly composed of 2 or 3 (preferably 2) aromatic rings selected from benzene ring, naphthalene ring and 5-to 10-membered (preferably 5- or 6-membered) aromatic heterocyclic ring. Preferred examples of the aromatic ring assembly are the aromatic ring assembly composed of 2 or 3 aromatic rings selected from the group consisting of benzene, naphthalene, pyridine, pyrimidine, thiophene, furan, thiazole, isothiazole, oxazole, an oxadiazole such as 1,2,4-oxadiazole or 1,3,4-oxadiazole, etc., a thiadiazole such as 1,2,4-thiadiazole or 1,3,4-thiadiazole, etc., quinoline, isoquinoline, indole, benzothiophene, benzoxazole, benzothiazole, and benzofuran.

Specific examples include a biphenylyl such as 2-, 3- or 4-biphenylyl, etc.; a naphthyl-substituted oxadiazolyl such as 3-(1-naphthyl)-1,2,4-oxadiazol-5-yl or 3-(2-naphthyl)-1,2,4-oxadiazol-5-yl, etc.; a benzofuranyl-substituted oxadiazolyl such as 3-(2-benzofuranyl)-1,2,4-oxadiazol-5-yl, etc.; a phenyl-substituted oxadiazolyl such as 3-phenyl-1,2,4-oxadiazol-5-yl or 5-phenyl-1,3,4-oxadiazol-2-yl, etc.; a benzoxazolyl-substituted oxadiazolyl such as 3-(2-benzoxazolyl)-1,2,4-oxadiazol-2-yl, etc.; an indolyl-substituted oxadiazolyl such as 3-(3-indolyl)-1,2,4-oxadiazol-2-yl or 3-(2-indolyl)-1,2,4-oxadiazol-2-yl, etc.; a phenyl-substituted thiazolyl such as 4-phenylthiazol-2-yl, etc.; a benzofuranyl-substituted thiazolyl such as 4-(2-benzofuranyl)-thiazol-2-yl, etc.; a phenyl-substituted oxazolyl such as 4-phenyl-1,3-oxazol-5-yl or 5-phenyloxazol-2-yl, etc.; a phenyl-substituted isothiazolyl such as 5-phenyl-isothiazol-4-yl, etc.; a thienyl-substituted phenyl such as 4-(2-thienyl)phenyl or 4-(3-thienyl)phenyl, etc.; a pyridyl-substituted phenyl such as 3-(3-pyridyl)phenyl or 4-(3-pyridyl)phenyl, etc.; a phenyl-substituted pyridyl such as 6-phenyl-3-pyridyl, etc.; a naphthyl-substituted phenyl such as 4-(2-naphthyl)phenyl, etc.; a benzofuranyl-substituted phenyl such as 4-(2-benzofuranyl)phenyl, etc.; a terphenylyl such as 4,4'-terphenylyl, etc.

The "fused aromatic ring" described above includes, for example, a monovalent group formed by removing one optional hydrogen atom from a fused polycyclic (preferably bi- to tetra-cyclic, preferably bi- or tri-cyclic) aromatic ring. The "fused polycyclic aromatic ring" includes, for example, a fused polycyclic aromatic hydrocarbon, a fused polycyclic aromatic heterocyclic ring, etc.

The "fused polycyclic aromatic hydrocarbon" includes, for example, a fused polycyclic (bi- or tri-cyclic) aromatic hydrocarbon having 9 to 14 carbon atoms (e.g., naphthalene, indene, anthracene, etc.).

The "fused polycyclic aromatic heterocyclic ring" includes, for example, 9- to 14-membered, preferably 9- or 10-membered fused polycyclic aromatic heterocyclic rings containing 1 or more (e.g., 1 to 4) hetero atoms selected from the group consisting of nitrogen, sulfur and oxygen atoms, in addition to carbon atoms, etc. Specifically, there are an aromatic heterocyclic ring such as benzofuran, benzimidazole, benzoxazole, benzothiazole, benzisothiazole, naphtho[2,3-b]thiophene, isoquinoline, quinoline, indole, quinoxaline, phenanthridine, phenothiazine, phenoxazine, phthalimide, and the like.

Specific examples of the "fused aromatic group" described above include a naphthyl such as 1-naphthyl or 2-naphthyl, etc.; a quinolyl such as 2-quinolyl, 3-quinolyl or 4-qunolyl, etc.; a benzofuranyl such as 2-benzofuranyl, etc.; a benzothiazolyl such as 2-benzothiazolyl, etc.; a benzimidazolyl such as 2-benzimidazolyl, etc.; an indolyl such as 1-indolyl, 2-indolyl or 3-indolyl, etc.

Among the above groups, those preferred as the aromatic groups shown by Ar¹ and Ar² are:
(1) monocyclic aromatic groups such as phenyl; a thienyl such as 2- or 3-thienyl, etc.; a furyl such as 2- or 2-furyl, etc.; a pyridyl such as 2-, 3- or 4-pyridyl, etc.;
(2) ring-assembled aromatic groups, e.g., a biphenylyl such as 2-, 3- or 4-biphenylyl, etc.; a naphthyl-substituted oxadiazolyl such as 3-(1-naphthyl)-1,2,4-oxadiazol-5-yl or 3-(2-naphthyl)-1,2,4-oxadiazol-5-yl, etc.; a benzofuranyl-substituted oxadiazolyl such as 3-(2-benzofuranyl)-1,2,4-oxadiazol-5-yl, etc.; a phenyl-substituted oxadiazolyl such as 3-phenyl-1,2,4-oxadiazol-5-yl, etc.; a benzoxazolyl-substituted oxadiazolyl such as 3-(2-benzoxazolyl)-1,2,4-oxadiazol-2-yl, etc.; an indolyl-substituted oxadiazolyl such as 3-(3-indolyl)-1,2,4-oxadiazol-2-yl or 3-(2-indolyl)-1,2,4-oxadiazol-2-yl, etc.; a phenyl-substituted thiazolyl such as 4-phenylthiazol-2-yl, etc.; a benzofuranyl-substituted thiazolyl such as 4-(2-benzofuranyl)-thiazol-2-yl, etc.; a phenyl-substituted oxazolyl such as 4-phenyl-1,3-oxazol-5-yl, etc.; a thienyl-substituted phenyl such as 4-(2-thienyl)phenyl, etc.; a pyridyl-substituted phenyl such as 4-(3-pyridyl)phenyl, etc.; a naphthyl-substituted phenyl such as 4-(2-naphthyl)phenyl, etc.; a terphenylyl such as 4,4'-terphenylyl, etc.; and,
(3) fused aromatic groups, e.g., a quinolyl such as 2-, 3- or 4-quinolyl, etc.; an indolyl such as 1-, 2- or 3-indolyl, etc.

For the aromatic group shown by Ar¹, more preferred are monocyclic aromatic groups, e.g., phenyl, etc., and fused aromatic groups such as 2-quinolyl, 3-quinolyl or 4-quinolyl, etc. Phenyl is particularly preferably used.

For the aromatic group shown by Ar², more preferred are monocyclic aromatic groups, e.g., phenyl, a thienyl such as 2- or 3-thienyl, etc., a furyl such as 2- or 3-furyl, etc., a pyridyl such as 2-, 3- or 4-pyridyl, etc.

The "optionally halogenated C₁₋₆ alkyl" in the present specification includes, for example, a C₁₋₆ alkyl (e.g., methyl, ethyl, propyl, isopropyl, butyl, isobutyl, sec-butyl, tert-butyl, pentyl, hexyl, etc.) optionally having 1 to 5, preferably 1 to 3 halogen atoms (e.g., fluorine, chlorine, bromine, iodine, etc.). Specific examples are methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, etc.

The "optionally halogenated C₃₋₆ cycloalkyl" in the present specification includes, for example, a C₃₋₆ cycloalkyl (e.g., cyclopropyl, cyclobutyl, cyclopentyl, cyclohexyl, etc.) optionally having 1 to 5, preferably 1 to 3 halogen atoms (e.g., fluorine, chlorine, bromine, iodine, etc.). Specific examples are cyclopropyl, cyclobutyl, cyclopentyl, cyclohexyl, 4,4-dichlorocyclohexyl, 2,2,3,3-tetrafluorocyclopentyl, 4-chlorocyclohexyl, etc.

The "optionally halogenated C₁₋₆ alkoxy" in the present specification includes, for example, a C₁₋₆ alkoxy (e.g., methoxy, ethoxy, propoxy, butoxy, pentyloxy, etc.) optionally having 1 to 5, preferably 1 to 3 halogen atoms (e.g., fluorine, chlorine, bromine, iodine, etc.). Specific examples are methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy, etc.

The "optionally halogenated C₁₋₆ alkylthio" in the present specification includes, for example, a C₁₋₆ alkylthio (e.g., methylthio, ethylthio, propylthio, isopropylthio, butylthio, sec-butylthio, tert-butylthio, etc.) optionally having 1 to 5, preferably 1 to 3 halogen atoms (e.g., fluorine, chlorine, bromine, iodine, etc.). Specific examples are methylthio, difluoromethylthio, trifluoromethylthio, ethylthio, propylthio, isopropylthio, butylthio, 4,4,4-trifluorobutylthio, pentylthio, hexylthio, etc.

The "optionally halogenated C₁₋₆ alkyl-carbonyl" in the present specification includes, for example, a C₁₋₆ alkyl-carbonyl (e.g., acetyl, propanoyl, butanoyl, pentanoyl, hexanoyl, etc.) optionally having 1 to 5, preferably 1 to 3 halogen atoms (e.g., fluorine, chlorine, bromine, iodine, etc.). Specific examples are acetyl, monochloroacetyl, trifluoroacetyl, trichloroacetyl, propanoyl, butanoyl, pentanoyl, hexanoyl, etc.

The "optionally halogenated C₁₋₆ alkylsulfonyl" in the present specification includes, for example, a C₁₋₆ alkylsulfonyl (e.g., methylsulfonyl, ethylsulfonyl, propylsulfonyl, isopropylsulfonyl, butylsulfonyl, sec-butylsulfonyl, tert-butylsulfonyl, etc.) optionally having 1 to 5, preferably 1 to 3 halogen atoms (e.g., fluorine, chlorine, bromine, iodine, etc.). Specific examples are methylsulfonyl, difluoromethylsulfonyl, trifluoromethylsulfonyl, ethylsulfonyl, propylsulfonyl, isopropylsulfonyl, butylsulfonyl, 4,4,4-trifluorobutylsulfonyl, pentylsulfonyl, hexylsulfonyl, etc.

The "optionally halogenated C₁₋₆ alkyl-carboxamido" in the present specification includes, for example, a C₁₋₆ alkyl-carboxamido (e.g., acetamide, etc.) optionally having 1 to 5, preferably 1 to 3 halogen atoms (e.g., fluorine, chlorine, bromine, iodine, etc.). Specific examples are acetamido, trifluoroacetamido, propanamido, butanamido, etc.

In the formulae above, preferred examples of the "substituents" in the "aromatic group which may optionally be substituted," "optionally substituted monocyclic aromatic group," "optionally substituted phenyl group" shown by Ar¹ and Ar², the "optionally substituted phenyl group or optionally substituted quinolyl group" shown by Ar¹, the "optionally substituted phenyl, furyl, thienyl or pyridyl group" shown by Ar² and the "optionally substituted benzene ring" shown by the ring A include a halogen atom (e.g., fluorine, chlorine, bromine, iodine, etc.), a C₁₋₃ alkylenedioxy (e.g., methylenedioxy, ethylenedioxy, etc.), nitro, cyano, an optionally halogenated C₁₋₆ alkyl, a C₆₋₁₀ aryloxy-C₁₋₆ alkyl (e.g., phenoxymethyl, etc.), a C₁₋₆ alkyl-C₆₋₁₀ aryl-C₂₋₆ alkenyl (e.g., methylphenylethenyl, etc.), an optionally halogenated C₃₋₆ cycloalkyl, an optionally substituted C₇₋₁₆ aralkyl, an optionally halogenated C₁₋₆ alkoxy, an optionally halogenated C₁₋₆ alkylthio, hydroxy, an optionally substituted C₆₋₁₀ aryloxy, a C₆₋₁₀ aryl-C₇₋₁₆ aralkyloxy (e.g., phenylbenzyloxy, etc.), amino, a mono-C₁₋₆ alkylamino (e.g., methylamino, ethylamino, propylamino, isopropylamino, butylamino, etc.), an optionally substituted 5- to 7-membered saturated cyclic amino, acyl, acylamino, acyloxy, etc. Among them, preferred are a halogen atom, a C₁₋₃ alkylenedioxy, an optionally halogenated C₁₋₆ alkyl, a C₆₋₁₀ aryloxy-C₁₋₆ alkyl, a C₁₋₆ alkyl-C₆₋₁₀ aryl-C₂₋₆ alkenyl, an optionally substituted C₇₋₁₆ aralkyl, an optionally halogenated C₁₋₆ alkoxy, hydroxy, an optionally substituted C₆₋₁₀ aryloxy, acyl, acyloxy, etc.

In the "aromatic group which may optionally be substituted" shown by Ar¹ and Ar², the "aromatic group" may have 1 to 5, preferably 1 to 3 of the above substituents at substitutable positions on the aromatic group. When the number of the substituents is 2 or more, those substituents may be the same or different. Also, when the substituents are cyclic, the aromatic ring of the aromatic group may be bonded to the substituents by spiro-union.

Further, in the "benzene ring which may optionally be substituted" shown by the ring A, the "benzene ring" may have 1 to 4, preferably 1 to 2 of the above substituents at substitutable positions on the aromatic group. When the numbers of substituents are 2 or more, those substituents may be the same or different. Also, when the substituents are cyclic, the benzene ring may be bonded to the substituents by spiro-union.

The "C₇₋₁₆ aralkyl" for the "C₇₋₁₆ aralkyl which may optionally be substituted" described above includes, for example, benzyl, phenethyl, naphthylmethyl, etc.

The "C₆₋₁₀ aryloxy" for the "C₆₋₁₀ aryloxy which may be substituted" includes, for example, phenyloxy, naphthyloxy, etc.

The "substituents" for these "optionally substituted C₇₋₁₆ aralkyl" and "optionally substituted C₆₋₁₀ aryloxy" include, for example, 1 to 5 substituents selected from a halogen atom (e.g., fluorine, chlorine, bromine, iodine, etc.), a C₁₋₃ alkylenedioxy (e.g., methylenedioxy, ethylenedioxy, etc.), nitro, cyano, an optionally halogenated C₁₋₆ alkyl, an optionally halogenated C₃₋₆ cycloalkyl, an optionally halogenated C₁₋₆ alkoxy, an optionally halogenated C₁₋₆ alkylthio, hydroxy, amino, a mono-C₁₋₆ alkylamino (e.g., methylamino, ethylamino, propylamino, isopropylamino, butylamino, etc.), a di-C₁₋₆ alkylamino (e.g., dimethylamino, diethylamino, dipropylamino, dibutylamino, ethylmethylamino, etc.), formyl, carboxy, carbamoyl, an optionally halogenated C₁₋₆ alkyl-carbonyl, a C₁₋₆ alkoxy-carbonyl (e.g., methoxycarbonyl, ethoxycarbonyl, propoxycarbonyl, tert-butoxycarbonyl, etc.), a mono-C₁₋₆ alkyl-carbamoyl (e.g., methylcarbamoyl, ethylcarbamoyl, etc.), a di-C₁₋₆ alkyl-carbamoyl (e.g., dimethylcarbamoyl, diethylcarbamoyl, ethylmethylcarbamoyl, etc.), an optionally halogenated C₁₋₆ alkylsulfonyl, formylamino, an optionally halogenated C₁₋₆ alkyl-carboxamido, a C₁₋₆ alkoxy-carboxamido (e.g., methoxycarboxamido, ethoxycarboxamido, propoxycarboxamido, butoxycarboxamido, etc.), a C₁₋₆ alkylsulfonylamino (e.g., methylsulfonylamino, ethylsulfonylamino, etc.), a C₁₋₆ alkyl-carbonyloxy (e.g., acetoxy, propanoyloxy, etc.), a C₁₋₆ alkoxy-carbonyloxy (e.g., methoxycarbonyloxy, ethoxycarbonyloxy, propoxycarbonyloxy, butoxycarbonyloxy, etc.), a mono-C₁₋₆ alkyl-carbamoyloxy (e.g., methylcarbamoyloxy, ethylcarbamoyloxy, etc.), a di-C₁₋₆ alkyl-carbamoyloxy (e.g., dimethylcarbamoyloxy, diethylcarbamoyloxy, etc.), and the like.

In the "5- to 7-membered saturated cyclic amino which may optionally be substituted" above, examples of the "5- to 7-membered saturated cyclic amino" include morpholino, thiomorpholino, piperazin-1-yl, piperidino, pyrrolidin-1-yl, hexamethylenimin-1-yl, etc.

In the "5- to 7-membered saturated cyclic amino which may optionally be substituted," the "substituent" includes, for example, 1 to 3 substituents selected from a C₁₋₆ alkyl, an optionally substituted C₆₋₁₄aryl, an optionally substituted C₇₋₁₉aralkyl, an optionally substituted 5- to 10-membered aromatic heterocyclic group, an optionally substituted C₆₋₁₀ aryl-carbonyl, an optionally halogenated C₁₋₆ alkylsulfonyl, etc.

In the "optionally substituted C₆₋₁₄ aryl," the "C₆₋₁₄ aryl" includes, for example, phenyl, a naphthyl such as 1-naphthyl, 2-naphthyl, etc.; an indenyl such as 2-indenyl, etc., an anthryl such as 2-anthryl, etc. Preferred is phenyl or the like.

In the "optionally substituted C₇₋₁₉ aralkyl," the "C₇₋₁₉ aralkyl" includes, for example, benzyl, phenethyl, diphenylmethyl, triphenylmethyl, 1-naphthylmethyl, 2-naphthylmethyl, 2,2-diphenylmethyl, 3-phenylpropyl, 4-phenylbutyl, 5-phenylpentyl, etc. Preferred is benzyl or the like.

In the "optionally substituted 5- to 10-membered aromatic heterocyclic group," the "5- to 10-membered aromatic heterocyclic group" includes, for example, a pyridyl such as 2-, 3- or 4-pyridyl, etc.; an indolyl such as 1-, 2- or 3-indolyl, etc.; a thienyl such as 2- or 3-thienyl, etc. Preferred is a pyridyl such as 2-, 3- or 4-pyridyl, etc.

In the "optionally substituted C₆₋₁₀ aryl-carbonyl," the "C₆₋₁₀ aryl-carbonyl" includes, for example, benzoyl, 1-naphthoyl, 2-naphthoyl, etc.

In each of these "optionally substituted C₆₋₁₄ aryl," "optionally substituted C₇₋₁₉ aralkyl," "optionally substituted 5- to 10-membered aromatic heterocyclic group" and "optionally substituted C₆₋₁₀ aryl-carbonyl," the "substituent" includes, for example, 1 to 5 substituents selected from a halogen atom (e.g., fluorine, chlorine, bromine, iodine, etc.), a C₁₋₃ alkylenedioxy (e.g., methylenedioxy, ethylenedioxy, etc.), nitro, cyano, an optionally halogenated C₁₋₆ alkyl, an optionally halogenated C₃₋₆ cycloalkyl, an optionally halogenated C₁₋₆ alkoxy, an optionally halogenated C₁₋₆ alkylthio, hydroxy, amino, a mono-C₁₋₆ alkylamino (e.g., methylamino, ethylamino, propylamino, isopropylamino, butylamino, etc.), a di-C₁₋₆ alkylamino (e.g., dimethylamino, diethylamino, dipropylamino, dibutylamino, ethylmethylamino, etc.), formyl, carboxy, carbamoyl, an optionally halogenated C₁₋₆ alkyl-carbonyl, a C₁₋₆ alkoxy-carbonyl (e.g., methoxycarbonyl, ethaxycarbonyl, propoxycarbonyl, tert-butoxycarbonyl, etc.), a mono-C₁₋₆ alkyl-carbamoyl (e.g., methylcarbamoyl, ethylcarbamoyl, etc.), a di-C₁₋₆ alkyl-carbamoyl (e.g., dimethylcarbamoyl, diethylcarbamoyl, ethylmethylcarbamoyl, etc.), an optionally halogenated C₁₋₆ alkylsulfonyl, formylamino, an optionally halogenated C₁₋₆ alkyl-carboxamido, a C₁₋₆ alkoxy-carboxamido (e.g., methoxycarboxamido, ethoxycarboxamido, propoxycarboxamido, butoxycarboxamido, etc.), a C₁₋₆ alkylsulfonylamino (e.g., methylsulfonylamino, ethylsulfonylamino, etc.), a C₁₋₆ alkyl-carbonyloxy (e.g., acetoxy, propanoyloxy, etc.), a C₁₋₆ alkoxy-carbonyloxy (e.g., methoxycarbonyloxy, ethoxycarbonyloxy, propoxycarbonyloxy, butoxycarbonyloxy, etc.), a mono-C₁₋₆ alkyl-carbamoyloxy (e.g., methylcarbamoyloxy, ethylcarbamoyloxy, etc.), a di-C₁₋₆ alkyl-carbamoyloxy (e.g., dimethylcarbamoyloxy, diethylcarbamoyloxy, etc.), and the like.

In the "optionally substituted aromatic group" shown by Ar¹ and Ar² described above and "optionally substituted benzene ring," the "acyl" as its "substituent" and the "acyl" in "acylamino" and "acyloxy" include formyl, carboxy, carbamoyl, an optionally halogenated C₁₋₆ alkyl-carbonyl, a C₁₋₆ alkoxy-carbonyl (e.g., methoxycarbonyl, ethoxycarbonyl, propoxycarbonyl, tert-butoxycarbonyl, etc.), an optionally substituted C₆₋₁₀ aryl-carbonyl, an optionally substituted C₆₋₁₀ aryloxy-carbonyl, an optionally substituted C₇₋₁₆ aralkyloxy-carbonyl, an optionally substituted 5- or 6-membered heterocyclic carbonyl, a mono-C₁₋₆ alkyl-carbamoyl, a di-C₁₋₆ alkyl-carbamoyl (e.g., dimethylcarbamoyl, diethylcarbamoyl, ethylmethylcarbamoyl, etc.), an optionally substituted C₆₋₁₀ aryl-carbamoyl, an optionally substituted 5- or 6-membered heterocyclic carbamoyl, an optionally halogenated C₁₋₆ alkylsulfonyl, an optionally substituted C₆₋₁₀ arylsulfonyl, etc. Among them, preferred are an optionally halogenated C₁₋₆ alkyl-carbonyl, a C₁₋₆ alkoxy-carbonyl (e.g., ethoxycarbonyl, etc.), an optionally substituted C₆₋₁₀ aryl-carbonyl, an optionally substituted C₆₋₁₀ arylsulfonyl, and the like.

In the "optionally substituted C₆₋₁₀ aryl-carbonyl" described above, examples of the "C₆₋₁₀ aryl-carbonyl" include benzoyl, 1-naphthoyl, 2-naphthoyl, etc.

In the "optionally substituted C₆₋₁₀ aryloxy-carbonyl" described above, examples of the "C₆₋₁₀ aryloxy-carbonyl" include phenoxycarbonyl, etc.

In the "optionally substituted C₇₋₁₆ aralkyloxy-carbonyl" described above, examples of the "C₇₋₁₆ aralkyloxy-carbonyl" include benzyloxycarbonyl, phenethyloxycarbonyl, etc.

In the "optionally substituted 5- or 6-membered heterocyclic carbonyl" described above, examples of the "5- or 6-membered heterocyclic carbonyl" include nicotinoyl, isonicotinoyl, a thenoyl such as 2-thenoyl or 3-thenoyl, etc., a furoyl such as 2-furoyl or 3-furoyl, etc., morpholinocarbonyl, piperidinocarbonyl, pyrrolidin-1-ylcarbonyl, etc.

In the "optionally substituted C₆₋₁₀ aryl-carbamoyl" described above, examples of the "C₆₋₁₀ aryl-carbamoyl" include phenylcarbamoyl, 1-phenylcarbamoyl, 1-naphthylcarbamoyl, 2-naphthylcarbamoyl, etc.

In the "optionally substituted 5- or 6-membered heterocyclic carbamoyl" described above, examples of the "5- or 6-membered heterocyclic carbamoyl" include a pyridylcarbamoyl such as 2-pyridylcarbamoyl, 3-pyridylcarbamoyl or 4-pyridylcarbamoyl, etc., a thienylcarbamoyl such as 2-thienylcarbamoyl or 3-thienylcarbamoyl, etc.

In the "optionally substituted C₆₋₁₀ arylsulfonyl" described above, examples of the "C₆₋₁₀ arylsulfonyl" include benzenesulfonyl, 1-naphthalenesulfonyl, 2-naphthalenesulfonyl, etc.

In these "optionally substituted C₆₋₁₀ arylcarbonyl," "optionally substituted C₆₋₁₀ aryloxycarbonyl," "optionally substituted C₇₋₁₆ aralkyloxy-carbonyl," "optionally substituted 5- or 6-membered carbonyl," "optionally substituted C₆₋₁₀ arylcarbamoyl," "optionally substituted 5- or 6-membered heterocyclic carbamoyl" and "optionally substituted C₆₋₁₀ arylsulfonyl," examples of the "substituents" include 1 to 5 substituents, preferably 1 to 3 substituents selected from a halogen atom, a C₁₋₃ alkylenedioxy, nitro, cyano, an optionally halogenated C₁₋₆ alkyl, an optionally halogenated C₁₋₆ alkoxy, an optionally halogenated C₁₋₆ alkylthio, hydroxy, amino, a mono-C₁₋₆ alkylamino, a di-C₁₋₆ alkylamino, formyl, carboxy, carbamoyl, an optionally halogenated C₁₋₆ alkyl-carbonyl, a C₁₋₆ alkoxy-carbonyl, a mono-C₁₋₆ alkyl-carbamoyl, di-C₁₋₆ alkyl-carbamoyl, an optionally halogenated C₁₋₆ alkylsulfonyl, formylamino, an optionally halogenated C₁₋₆ alkyl-carboxamido, a C₁₋₆ alkoxycarboxamido, a C₁₋₆ alkylsulfonylamino, a C₁₋₆ alkyl-carbonyloxy, a C₁₋₆ alkoxy-carbonyloxy, a mono-C₁₋₆ alkyl-carbamoyloxy and a di-C₁₋₆ alkyl-carbamoyloxy. Among them, preferred are a halogen atom, an optionally halogenated C₁₋₆ alkyl, an optionally halogenated C₁₋₆ alkoxy, and the like.

In the "aromatic group which may optionally be substituted" shown by Ar¹ and Ar² and the "optionally substituted benzene ring" shown by the ring A described above, the "acylamino" as the "substituent" includes an amino substituted with 1 or 2 "acyl" groups described in detail, e.g., on the "substituents" for the "aromatic group which may optionally be substituted" above, and preferred examples are formylamino, an optionally halogenated C₁₋₆ alkyl-carboxamido, an optionally substituted C₆₋₁₀ aryl-carboxamido (e.g., phenylcarboxamido, naphthylcarboxamido, etc.), a C₁₋₆ alkoxy-carboxamido (e.g., methoxycarboxamido, ethoxycarboxamido, propoxycarboxamido, butoxycarboxamido, etc.), a C₁₋₆ alkylsulfonylamino (e.g., methylsulfonylamino, ethylsulfonylamino, etc.), and the like.

In the "aromatic group which may optionally be substituted" shown by Ar¹ and Ar² and the "optionally substituted benzene ring" shown by the ring A described above, the "acyloxy" as the "substituent" includes an oxy substituted with one "acyl" described in detail, e.g., on the "substituents" for the "optionally substituted aromatic group" described above, and preferred are a C₁₋₆ alkyl-carbonyloxy (e.g., acetoxy, propanoyloxy, etc.), an optionally substituted C₆₋₁₀ aryl-carbonyloxy (e.g., benzoyloxy, 1-naphthoyloxy, 2-naphthoyloxy, etc.), a C₁₋₆ alkoxy-carbonyloxy (e.g., methoxycarbonyloxy, ethoxycarbonyloxy, propoxycarbonyloxy, butoxycarbonyloxy, etc.), a mono-C₁₋₆ alkyl-carbamoyloxy (e.g., methylcarbamoyloxy, ethylcarbamoyloxy, etc.), a di-C₁₋₆ alkyl-carbamoyloxy (e.g., dimethylcarbamoyloxy, diethylcarbamoyloxy, etc.), an optionally substituted C₆₋₁₀ aryl-carbamoyloxy (e.g., phenylcarbamoyloxy, naphthylcarbamoyloxy, etc.), nicotinoyloxy, and the like.

In the compounds described above, an optionally substituted phenyl group or an optionally substituted quinolyl group are preferred for Ar¹, an optionally substituted phenyl group is more preferred, and most preferred is a phenyl group which may optionally be substituted with a halogen atom, a C₁₋₃ alkylenedioxy group, an optionally halogenated C₁₋₆ alkyl group or an optionally halogenated C₁₋₆ alkoxy group.

For Ar², an optionally substituted monocyclic aromatic group is preferred, a phenyl, furyl, thienyl or pyridyl group, each of which may optionally be substituted, is more preferred, much more preferred is a phenyl, furyl, thienyl or pyridyl group, each of which may optionally be substituted with a halogen atom, a C₁₋₃ alkylenedioxy group, an optionally halogenated C₁₋₆ alkyl group or an optionally halogenated C₁₋₆ alkoxy group, and most preferred is 3,4-dimethoxyphenyl group.

For the ring A, unsubstituted benzene ring is preferred.

For X, NR⁵ (wherein R⁵ has the same significance as defined above) is preferred.

For Y, CH is preferred.

As the salts of the compounds described above, pharmaceutically acceptable salts are preferred, and examples are salts with inorganic bases, salts with organic bases, salts with inorganic acids, salts with organic acids, salts with basic or acidic amino acids, etc.

Preferred examples of salts with inorganic bases include alkali metal salts such as sodium salts, potassium salts, etc.; alkaline earth metal salts such as calcium salts, magnesium salts, etc.; aluminum salts, ammonium salts, etc.

Preferred examples of salts with organic bases include salts with trimethylamine, triethylamine, pyridine, picoline, ethanolamine, diethanolamine, triethanolamine, dicyclohexylamine, N',N'-dibenzylethylenediamine, etc.

Preferred examples of the salts with inorganic acids are salts with hydrochloric acid, hydrobromic acid, nitric acid, sulfuric acid, phosphoric acid, etc.

Preferred examples of the salts with organic acids are salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.

Preferred examples of the salts with basic amino acids are salts with arginine, lysine, omithine, etc., and preferred examples of the salts with acidic amino acids are salts with aspartic acid, glutamic acid, etc.

The prodrugs of the compounds described above refer to compounds that are converted into the compounds described above through reactions associated with enzymes, gastric acid, etc. in vivo under the physiological conditions, that is, compounds which are converted into the compounds described above by oxidation, reduction, hydrolysis, etc. caused enzymatically; or compounds which are converted into the compounds described above through hydrolysis by gastric acid, etc. Examples of the prodrug of the compounds above include a compound wherein an amino group of the compound above is acylated, alkylated or phosphorylated (e.g., a compound wherein an amino group of the compound described above is substituted with eicosanoyl, alanyl, pentylaminocarbonyl, (5-methyl-2-oxo-1,3-dioxolen-4-yl)methoxycarbonyl, tetrahydrofuranyl, tetrahydropyranyl, pyrrolidylmethyl, pivaloyloxymethyl, tert-butyl, etc.); a compound wherein a hydroxy group of the compound described above is acylated, alkylated, phosphorylated or borated (e.g., a compound wherein a hydroxy group of the compound described above is substituted with acetyl, palmitoyl, propanoyl, pivaloyl, succinyl, fumaryl, alanyl, dimethylaminomethylcarbonyl, tetrahydropyranyl, etc.); a compound wherein a carboxyl group of the compound described above is esterified or amidated (e.g., a compound wherein a carboxyl group of the compound (I) is converted into the ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); and the like. These compounds can be manufactured from the compounds described above by publicly known methods.

The compounds described above may also be labeled with an isotope (e.g., ³H, ¹⁴C, ³⁵S, ¹²⁵I, etc.) or the like.

The compounds described above may also be in the form of anhydrides or hydrates thereof.

The processes of manufacturing the compounds described above are stated below.

The following description of the manufacturing processes are applied not only to the compounds described above but also to salts thereof and prodrugs thereof, but in the following description they are sometimes merely referred to as compounds. Likewise, the respective compounds used to manufacture the compounds described above include salts thereof as well, even when they are simply referred to as, e.g., Compound (II).

The compounds described above can be manufactured, e.g., by the methods given below, using publicly known procedures.

The thus obtained compounds described above can be isolated and purified from reaction solutions by any publicly known procedures, for example, through solvent extraction, concentration, neutralization, filtration, crystallization, recrystallization, column chromatography, high performance liquid chromatography, recrystallization, etc., whereby the product can be isolated and purified in a high purity.

The compounds described above may also be in the form of hydrates or anhydrides thereof. The hydrates include monohydrates, 0.5 hydrates, dihydrates, etc.

Also, the compounds described above may be labeled with an isotope (e.g., ³H, ¹⁴C, ³⁵S, ¹²⁵I, etc.) or the like.

Where the compounds of the present invention described above or salts thereof contain an asymmetric carbon, the resulting optically active mixture (racemate) can be resolved to the respective optically active compounds by conventional optical resolution means, for example, through formation of salts with optically active acids (e.g., camphor sulfonic acid, etc.) or optically active bases (e.g., 1-methylbenzylamine, etc.), or by separation means such as various chromatographies (e.g., liquid chromatography using an optically active column, etc.), fractional recrystallization, etc.

The "room temperature" means normally 0 to 30°C.

Symbols in the chemical structures described in the manufacturing processes have the same significance as defined above, unless otherwise indicated.

In the present specification, the "splitting-off group" refers to a halogen atom (e.g., chloro, bromo, iodo, etc.), an optionally halogenated C₁₋₆ alkylsulfonyloxy (e.g., methanesulfonyloxy, ethanesulfonyloxy, trifluoromethanesulfonyloxy, etc.), an optionally substituted C₆₋₁₀ arylsulfonyloxy, hydroxy, etc.

The "substituent" for the "optionally substituted C₆₋₁₀ arylsulfonyloxy" includes 1 to 3 substituents selected from a halogen atom, an optionally halogenated C₁₋₆ alkyl or C₁₋₆ alkoxy, etc. Specific examples of the "optionally substituted C₆₋₁₀ arylsulfonyloxy" are benzenesulfonyloxy, p-toluenesulfonyloxy, 1-naphthalenesulfonyloxy, 2-naphthalenesulfonyloxy, etc.

In the present specification, the "base" includes;
1) for example, strong bases such as alkali metal or alkaline earth metal hydrides (e.g., lithium hydride, sodium hydride, potassium hydride, calcium hydride, etc.), alkali metal or alkaline earth metal amides (e.g., lithium amide, sodium amide, lithium diisopropylamide, lithium dicyclohexylamide, lithium hexamethyldisilazide, sodium hexamethyldisilazide, potassium hexamethyldisilazide, etc.), alkali metal or alkaline earth metal lower alkoxides (e.g., sodium methoxide, sodium ethoxide, potassium tert-butoxide, etc.), and the like.;
2) for example, inorganic bases such as alkali metal or alkaline earth metal hydroxides (e.g., sodium hydroxide, potassium hydroxide, lithium hydroxide, barium hydroxide, etc.), alkali metal or alkaline earth metal carbonates (e.g., sodium carbonate, potassium carbonate, cesium carbonate, etc.), alkali metal or alkaline earth metal hydrogencarbonates (e.g., sodium hydrogencarbonate, potassium hydrogencarbonate, etc.), and the like;
3) for example, organic bases such as amines, e.g., triethylamine, diisopropylethylamine, N-methylmorpholine, dimethylaminopyridine, DBU (1,8-diazabicyclo[5.4.0]-7-undec-7-ene), DBN (1,5-diazabicyclo[4.3.0]non-5-ene), etc., basic heterocyclic compounds, e.g., pyridine, imidazole, 2,6-lutidine, etc.

The reactions described in the present specification, for example, "alkylation," "hydrolysis," "amination," "esterification," "amidation," "esterification," "etherification," "oxidation," "reduction," etc. may be carried out by publicly known methods, for example, those described in ORGANIC FUNCTIONAL GROUP PREPARATIONS, 2nd Ed., Academic Press Inc., 1989, etc.

According to the present invention, the compounds represented by formula (I) above, or salts thereof, or prodrugs thereof are employed.

In the lower alkyl group which may optionally be substituted, which is shown by R¹, R², R³, R⁴ and R⁵, the "lower alkyl group" includes a C₁₋₆ alkyl (e.g., methyl, ethyl, propyl, isopropyl, butyl, isobutyl, sec-butyl, tert-butyl, pentyl, hexyl, etc.) and the like. Preferred are methyl, ethyl and propyl.

In the compounds represented by formula (I) described above, salts thereof, or prodrugs thereof, the same description on the "substituent" for the "lower alkyl group which may optionally be substituted" shown by R³, R⁴ and R⁵, which was made on the compounds above having the soluble beta amyloid precursor protein secretion promoting activity and apoptosis inhibiting activity, salts thereof, or prodrugs thereof, applies to the "substituent" for the "lower alkyl group which may optionally be substituted" shown by R¹, R², R³, R⁴ and R⁵, in the formula (I) described above.

In the formula (I) described above, the compounds wherein R¹ and R² are combined to form a 4- to 7-membered ring together with the adjacent carbon atoms include: 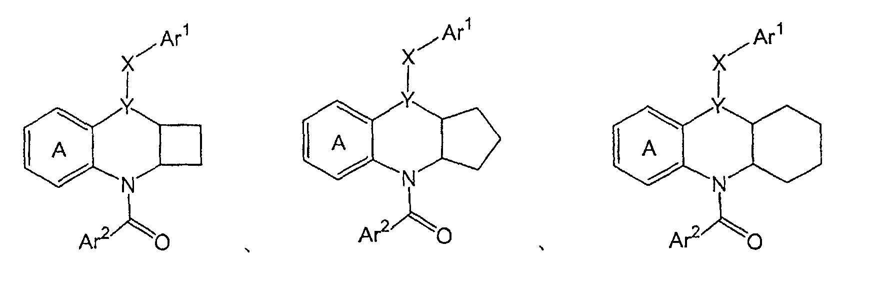 or 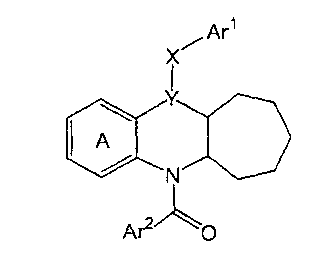 [wherein the symbols have the same significance as defined above], and the like.

For R¹, hydrogen atom is preferred; and for R², hydrogen atom or an optionally substituted lower alkyl group is preferred, more preferably, a C₁₋₆ alkyl group, and most preferably, methyl, etc.

For X, NR⁵ (wherein R⁵ has the same significance as defined above); and CH is preferably used for Y.

Each of Ar¹ and Ar² represents an aromatic group which may optionally be substituted.

In the compounds represented by formula (I) described above, salts thereof, or prodrugs thereof, the same description on the "aromatic group" for the "aromatic group which may optionally be substituted" shown by Ar¹ and Ar², which was made on the compounds above having the soluble beta amyloid precursor protein secretion promoting activity and apoptosis inhibiting activity, salts thereof, or prodrugs thereof, applies to the "aromatic group" for the "aromatic group which may optionally be substituted" shown by Ar¹ and Ar², in the formula (I) described above.

In the aromatic groups shown by Ar¹ and Ar² described above, preferred examples are:
(1) monocyclic aromatic groups such as phenyl; a thienyl such as 2- or 3-thienyl, etc.; a furyl such as 2- or 2-furyl, etc.; a pyridyl such as 2-, 3- or 4-pyridyl, etc.;
(2) ring-assembled aromatic groups, e.g., a biphenylyl such as 2-, 3- or 4-biphenylyl, etc.; a naphthyl-substituted oxadiazolyl such as 3-(1-naphthyl)-1,2,4-oxadiazol-5-yl or 3-(2-naphthyl)-1,2,4-oxadiazol-5-yl, etc.; a benzofuranyl-substituted oxadiazolyl such as 3-(2-benzofuranyl)-1,2,4-oxadiazol-5-yl, etc.; a phenyl-substituted oxadiazolyl such as 3-phenyl-1,2,4-oxadiazol-5-yl, etc.; a benzoxazolyl-substituted oxadiazolyl such as 3-(2-benzoxazolyl)-1,2,4-oxadiazol-2-yl, etc.; an indolyl-substituted oxadiazolyl such as 3-(3-indolyl)-1,2,4-oxadiazol-2-yl or 3-(2-indolyl)-1,2,4-oxadiazol-2-yl, etc.; a phenyl-substituted thiazolyl such as 4-phenylthiazol-2-yl, etc.; a benzofuranyl-substituted thiazolyl such as 4-(2-benzofuranyl)thiazol-2-yl, etc.; a phenyl-substituted oxazolyl such as 4-phenyl-1,3-oxazol-5-yl, etc.; a thienyl-substituted phenyl such as 4-(2-thienyl)phenyl, etc.; a pyridyl-substituted phenyl such as 4-(3-pyridyl)phenyl, etc.; a naphthyl-substituted phenyl such as 4-(2-naphthyl)phenyl, etc.; a terphenylyl such as 4,4'-terphenylyl, etc.; and,
(3) fused aromatic groups, e.g., a quinolyl such as 2-, 3- or 4-quinolyl, etc.; an indolyl such as 1-, 2- or 3-indolyl, etc.

For the aromatic group shown by Ar¹, more preferred are monocyclic aromatic groups, e.g., phenyl, etc., and fused aromatic groups such as 2-quinolyl, 3-quinolyl or 4-quinolyl, etc. Phenyl is particularly preferably used.

For the aromatic group shown by Ar², more preferred are monocyclic aromatic groups, e.g., phenyl, a thienyl such as 2- or 3-thienyl, etc., a furyl such as 2- or 3-furyl, etc., a pyridyl such as 2-, 3- or 4-pyridyl, etc.

In the compounds represented by formula (I) described above, salts thereof, or prodrugs thereof, the same description on the "substituent" for the "aromatic group which may optionally be substituted," "optionally substituted monocyclic aromatic group" and "optionally substituted phenyl group" shown by Ar¹ and Ar², for the "optionally substituted phenyl group or optionally substituted quinolyl group" shown by Ar¹, for the "optionally substituted phenyl, furyl, thienyl or pyridyl group" shown by Ar², and for the "optionally substituted benzene ring" shown by the ring A, which was made on the compounds above having the soluble beta amyloid precursor protein secretion promoting activity and apoptosis inhibiting activity, salts thereof, or prodrugs thereof, applies to the "substituent" for the "aromatic group which may optionally be substituted," "optionally substituted monocyclic aromatic group" and "optionally substituted phenyl group" shown by Ar¹ and Ar², for the "optionally substituted phenyl group or optionally substituted quinolyl group" shown by Ar¹, for the "optionally substituted phenyl, furyl, thienyl or pyridyl group" shown by Ar², and for the "optionally substituted benzene ring" shown by the ring A, in the formula (I) described above.

In the compounds represented by formula (I), hydrogen atom is preferred for R¹.

For R², hydrogen atom or an optionally substituted lower alkyl group is preferred, more preferably a C₁₋₆ alkyl group, and particularly preferably methyl, etc.

For Ar¹, preferred is an optionally substituted phenyl group or an optionally substituted quinolyl group; an optionally substituted phenyl group is more preferred, and particularly preferred is a phenyl group which may optionally be substituted with a halogen atom, a C₁₋₃ alkylenedioxy group, an optionally halogenated C₁₋₆ alkyl group or an optionally halogenated C₁₋₆ alkoxy group.

For Ar², an optionally substituted monocyclic aromatic group is preferred; a phenyl, furyl, thienyl or pyridyl group, each of which may optionally be substituted, is more preferred, much more preferred is a phenyl, furyl, thienyl or pyridyl group, each of which may optionally be substituted with a halogen atom, a C₁₋₃ alkylenedioxy group, an optionally halogenated C₁₋₆ alkyl group or an optionally halogenated C₁₋₆ alkoxy group, and most preferred is 3,4-dimethoxyphenyl group.

For the ring A, unsubstituted benzene ring is preferred.

For X, NR⁵ (wherein R⁵ has the same significance as defined above) is preferred.

For Y, CH is preferred.

Also, in the compound shown by formula (I) described above, in particular, preferred are the compound wherein R¹ is hydrogen atom, R² is methyl group, Ar¹ is optionally substituted phenyl group, Ar² is 3,4-dimethoxyphenyl group, the ring A is unsubstituted benzene ring, X is NR⁵ (wherein R⁵ has the same significance as described above) and Y is CH.

As the salts of the compounds represented by the formula (I) described above (hereinafter sometimes simply referred to as the compound (I)), pharmaceutically acceptable salts are preferred, and examples are salts with inorganic bases, salts with organic bases, salts with inorganic acids, salts with organic acids, salts with basic or acidic amino acids, etc.

Preferred examples of salts with inorganic bases include alkali metal salts such as sodium salts, potassium salts, etc.; alkaline earth metal salts such as calcium salts, magnesium salts, etc.; aluminum salts, ammonium salts, etc.

Preferred examples of salts with organic bases include salts with trimethylamine, triethylamine, pyridine, picoline, ethanolamine, diethanolamine, triethanolamine, dicyclohexylamine, N',N'-dibenzylethylenediamine, etc.

Preferred examples of the salts with inorganic acids are salts with hydrochloric acid, hydrobromic acid, nitric acid, sulfuric acid, phosphoric acid, etc.

Preferred examples of the salts with organic acids are salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.

Preferred examples of the salts with basic amino acids are salts with arginine, lysine, ornithine, etc., and preferred examples of the salts with acidic amino acids are salts with aspartic acid, glutamic acid, etc.

The prodrugs of the compound (I) refer to compounds that are converted into the compound (I) through reactions associated with enzymes, gastric acid, etc. in vivo under the physiological conditions, that is, compounds which are converted into the compound (I) by oxidation, reduction, hydrolysis, etc. caused enzymatically; or compounds which are converted into the compound (I) through hydrolysis by gastric acid, etc. Examples of the prodrugs of the compound (I) include a compound wherein an amino group of the compound (I) is acylated, alkylated or phosphorylated (e.g., a compound wherein an amino group of the compound (I) is substituted with eicosanoyl, alanyl, pentylaminocarbonyl, (5-methyl-2-oxo-1,3-dioxolen-4-yl)methoxycarbonyl, tetrahydrofuranyl, tetrahydropyranyl, pyrrolidylmethyl, pivaloyloxymethyl, tert-butyl, etc.); a compound wherein a hydroxy group of the compound (I) is acylated, alkylated, phosphorylated or borated (e.g., a compound wherein a hydroxy group of the compound (I) is substituted with acetyl, palmitoyl, propanoyl, pivaloyl, succinyl, fumaryl, alanyl, dimethylaminomethylcarbonyl, tetrahydropyranyl, etc.); a compound wherein a carboxyl group of the compound (I) is esterified or amidated (e.g., a compound wherein a carboxyl group of the compound (I) is converted into the ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); and the like. These compounds can be manufactured from the compound (I) by publicly known methods.

The compound (I) may also be labeled with an isotope (e.g., ³H, ¹⁴C, ³⁵S, ¹²⁵I, etc.) or the like.

The compound (I) may also be in the form of anhydrides or hydrates thereof.

The processes of manufacturing the compound (I) are described below.

The following description of the manufacturing processes are applied not only to the compound (I) but also to its salts and prodrugs thereof, but in the following description they are sometimes merely referred to as the compound (I). Likewise, the respective compounds used to manufacture the compound (I) include salts thereof as well, even when they are simply referred to as, e.g., Compound (I').

The compound (I) can be manufactured, e.g., by the methods given below, using publicly known procedures.

The thus obtained compound (I) can be isolated and purified from reaction solutions by any publicly known procedures, for example, through solvent extraction, concentration, neutralization, filtration, crystallization, recrystallization, column chromatography, high performance liquid chromatography, recrystallization, etc., whereby the product can be isolated and purified in a high purity.

The compound (I) may also be in the form of hydrates or anhydrides thereof. The hydrates include monohydrates, 0.5 hydrates, dihydrates, etc.

Furthermore, the compound (I) may be labeled with an isotope (e.g., ³H, ¹⁴C, ³⁵S, ¹²⁵I, etc.) or the like.

Where the compound (I) of the present invention or salts thereof contain an asymmetric carbon, the resulting optically active mixture (racemate) can be resolved to the respective optically active compounds by conventional optical resolution means, for example, through formation of salts with optically active acids (e.g., camphor sulfonic acid, etc.) or optically active bases (e.g., 1-methylbenzylamine, etc.), or by separation means such as various chromatographies (e.g., liquid chromatography using an optically active column, etc.), fractional recrystallization, etc.

The "room temperature" means normally 0 to 30°C.

The symbols given in the chemical structures described in the manufacturing processes have the same significance as defined above, unless otherwise indicated.

Hereinafter the processes of manufacturing the compound (I) are specifically described.

### PROCESS 1:

The compound (I) can be manufactured normally by PROCESS 1 described below. The compound (I') can be manufactured by publicly known methods described in, e.g., Bulletin of the Chemical Society of Japan. 42, 2885-2894 (1969) or Can. J. CHEM., 52 (1974), or methods referred to in these journals. 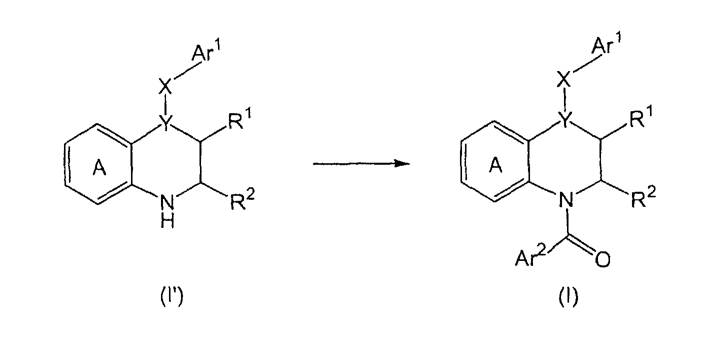 [wherein the symbols have the same significance as defined above].

The reaction described in PROCESS 1 is amidation, which can be effected, in the presence of a dehydrating and condensing agent, or by converting the carboxy once to its reactive derivative followed by condensation, or the like.

### i) Process using a dehydrating/condensing agent

The compound (I') or its salt, 1 to 5 equivalents of the compound shown by formula:

Ar² - COOH

[wherein Ar² has the same significance as defined above], and 1 to 2 equivalents of a dehydrating/condensing agent are reacted in an inert solvent at room temperature for 10 to 24 hours. The reaction may be carried out, if necessary, by adding 1 to 1.5 equivalents of 1-hydroxybenzotriazole (HOBT) and/or a catalytic amount to 5 equivalents of a base (e.g., triethylamine, 4-dimethylaminopyridine, etc.).

Examples of the "dehydrating/condensing agent" are dicyclohexylcarbodiimide (DCC), 1-ethyl-3-(3-dimethylaminopropyl)carbodiimide hydrochloride (WSC), etc. Among them, WSC is preferred.

For the inert solvent, for example, a nitrile solvent (preferably acetonitrile), an amide solvent (preferably dimethylformamide (DMF)), a halogenated hydrocarbon solvent (preferably dichloromethane), an ethereal solvent (preferably tetrahydrofuran (THF)), etc. may be used either singly or as a mixture of two or more.

### ii) Process using the reactive derivative of carboxy

The reactive derivative of the compound (I") and 1 to 5 equivalents (preferably 1 to 3 equivalents) of the compound (I') are reacted in an inert solvent at -20°C to 50°C (preferably at room temperature) for 5 minutes to 40 hours (preferably 1 to 18 hours). If necessary, the reaction may be carried out in the presence of 1 to 10 equivalents, preferably 1 to 3 equivalents of a base.

The "reactive derivative" of the compound (I") include acid halides (e.g., acid chlorides, acid bromides, etc.), mixed acid anhydrides (e.g., acid anhydrides with C₁₋₆ alkyl-carboxylic acids, C₆₋₁₀ aryl-carboxylic acids or C₁₋₆ alkyl-carbonic acids, etc.), activated esters (e.g., esters with optionally substituted phenols, 1-hydroxybenzotriazole or N-hydroxysuccinimide, etc.), and the like. The "substituent" for the "optionally substituted phenol" includes 1 to 5 substituents selected from a halogen atom, nitro, an optionally halogenated C₁₋₆ alkyl and an optionally halogenated C₁₋₆ alkoxy. Specific examples of the "optionally substituted phenol" are phenol, pentachlorophenol, pentafluorophenol, p-nitrophenol, etc. Acid halides are preferable as the reactive derivative.

Preferred examples of the "base" are sodium hydride, potassium carbonate, sodium carbonate, sodium hydroxide, potassium hydroxide, sodium hydrogencarbonate, potassium hydrogencarbonate, triethylamine, pyridine, etc.

For the inert solvent, for example, an ethereal solvent, a halogenated hydrocarbon solvent, an aromatic solvent, a nitrile solvent, an amide solvent, a ketone solvent, a sulfoxide solvent, water, etc. may be used either singly or as a mixture of two or more. Among them, pyridine, acetonitrile, THF, dichloromethane, chloroform, etc. are preferably used, more preferably, pyridine, THF, acetonitrile, etc.

### PROCESS 2:

The compound (Ia) wherein X is NR⁵ and Y is CH can be manufactured by converting the hydroxy group of the compound (IV) to its reactive substituent, e.g., in accordance with the method described in the literature (Tetrahedron Letters, 38 (15), 2673 (1997)), etc. and then substituting with the compound (IX) in the presence of a base. 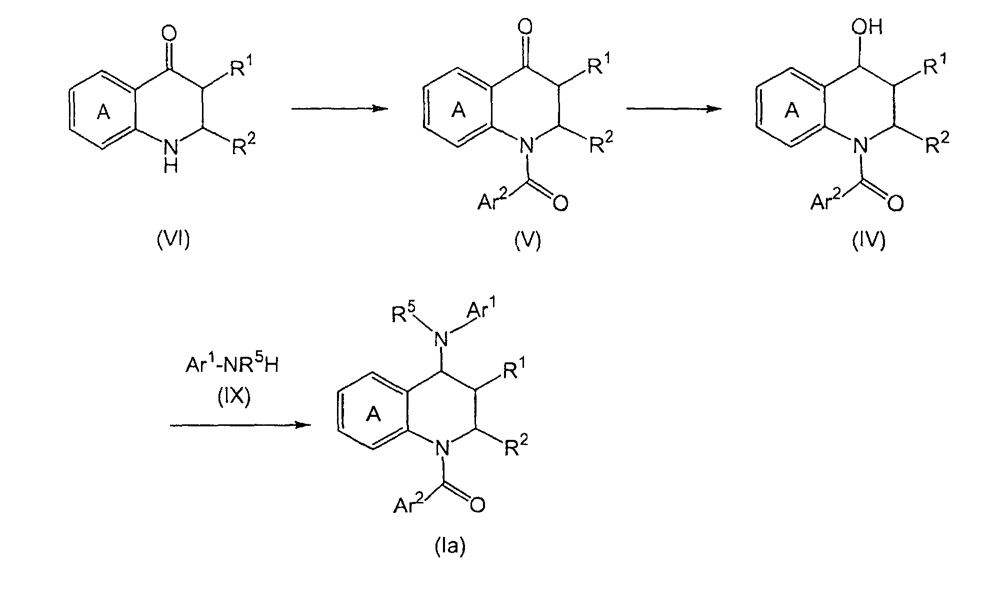 [wherein the symbols have the same significance as defined above].

The reactive substituent includes, for example, a halogen atom (e.g., chlorine, bromine, iodine, etc.), an optionally halogenated C₁₋₆ alkylsulfonyloxy (e.g., methanesulfonyloxy, ethanesulfonyloxy, trifluoromethanesulfonyloxy, etc.), an optionally substituted C₆₋₁₀ arylsulfonyloxy, etc.

The "substituent" for the "optionally substituted C₆₋₁₀ arylsulfonyloxy" includes a halogen atom, an optionally halogenated C₁₋₆ alkyl or C₁₋₆ alkoxy, etc. The number of the substituents is, e.g., 1 to 3. Specific examples of the "optionally substituted C₆₋₁₀ arylsulfonyloxy" are benzenesulfonyloxy, p-toluenesulfonyloxy, 1-naphthalenesulfonyloxy, 2-naphthalenesulfonyloxy, etc.

Preferred examples of the "reactive substituent" include a halogen atom (e.g., chlorine, bromine, iodine, etc.), methanesulfonyloxy, trifluoromethanesulfonyloxy, p-toluenesulfonyloxy, etc.

The halogenation is carried out, e.g., by reacting the compound (IV) with 1 to 10 equivalents of a halogenating agent in an inert solvent.

Examples of the halogenating agent are inorganic halides such as thionyl chloride, thionyl bromide, phosphorus trichloride, phosphorus pentachloride, phosphorus oxychloride, etc.; hydrohalogenic acids such as hydrochloric acid, hydrobromic acid, etc.; or silyl halides such as iodotrimethylsilane, etc. Preferably, iodotrimethylsilane is used.

The "inert solvent" includes an ethereal solvent, a halogenated hydrocarbon solvent, an aromatic solvent, a nitrile solvent, an amide solvent, a sulfoxide solvent, etc.

The reaction temperature is between -20°C and 200°C, preferably between 0°C and 100°C. The reaction time ranges from 0.1 to 48 hours, preferably 1 to 24 hours.

The sulfonylation is carried out, e.g., by reacting the compound (IV) and 1 to 5 equivalents of the corresponding sulfonyl halide in an inert solvent in the presence of a base.

The base is preferably potassium carbonate, sodium hydrogencarbonate, triethylamine, N-methylmorpholine, pyridine, etc. The amount of the base used is preferably from 1 to 10 equivalents.

The "inert solvent" includes an ethereal solvent, a halogenated hydrocarbon solvent, an aromatic solvent, a nitrile solvent, an amide solvent, a ketone solvent, a sulfoxide solvent, etc.

The reaction temperature is between -20°C and 200°C, preferably between 0°C and 100°C. The reaction time ranges from 0.1 to 48 hours, preferably 1 to 24 hours.

As the compound (IX), there are used commercially available anilines or anilines synthesized by the methods described in SHIN-JIKKEN KAGAKU KOZA, 14 (III), ORGANIC FUNCTIONAL GROUP PREPARATIONS, 2nd Ed., Academic Press Inc., 1989, Comprehensive Organic Transformations, VCH Publishers Inc., 1989, etc. The amount used is preferably from 1 to 10 equivalents.

As the base, those given above are used; preferred examples include potassium carbonate, barium carbonate, sodium hydrogencarbonate, triethylamine, N-methylmorpholine, pyridine, etc. The amount of the base used is preferably from 1 to 10 equivalents.

The reaction is carried out in an inert solvent. The "inert solvent" includes an ethereal solvent, a halogenated hydrocarbon solvent, an aromatic solvent, a nitrile solvent, an amide solvent, a ketone solvent, a sulfoxide solvent, etc.

The reaction temperature is between -20°C and 200°C, preferably between 0°C and 50°C. The reaction time ranges from 0.1 to 48 hours, preferably 1 to 24 hours.

The compound (IV) can be manufactured by reducing the compound (V) with an appropriate reducing agent in an inert solvent.

The "inert solvent" includes an alcoholic solvent, an ethereal solvent, a halogenated hydrocarbon solvent, an aromatic solvent, a nitrile solvent, an amide solvent, an organic acid solvent, etc. These solvents may be used by mixing two or more in a suitable ratio. Among these solvents, preferred are methanol, ethanol, etc.

Examples of the reducing agent include sodium borohydride, sodium triacetoxyborohydride, sodium cyanoborohydride, lithium aluminum hydride, etc. The amount of the reducing agent used is generally 1 to 20 equivalents, preferably 1 to 5 equivalents.

The reaction temperature is generally from -20°C to 150°C, preferably from 0°C to 50°C. The reaction time is generally from 5 minutes to 24 hours, preferably 1 to 12 hours.

The compound (V) can be manufactured by acylating the compound (VI) as in PROCESS 1.

The compound (VI) can be manufactured by publicly known methods (e.g., by the method described in Bio-Organic & Medicinal Chemistry Letters, 9, 1009 (1999), etc.).

### PROCESS 3:

The compound (Ia) can be manufactured by reacting the compound (Ib) obtained in PROCESS 1 or 2 with the compound shown by R⁵Z (VII) [wherein R⁵ is the reactive substituent described above] in an inert solvent in the presence of an appropriate base. 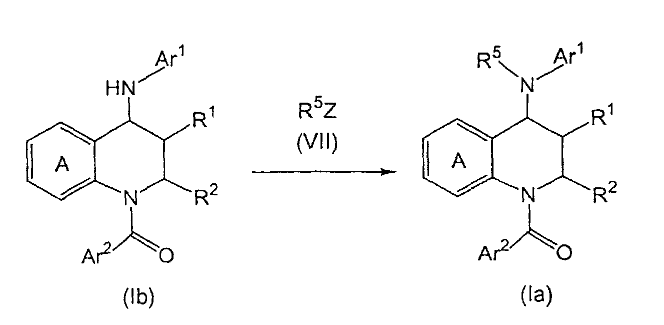 [wherein the symbols have the same significance as defined above].

For the reactive substituent, those described in PROCESS 1 are used, preferably a halogen atom, an alkylsulfonyl group, an arylsulfonyl group, etc.

For the "base," those given above are employed; preferred are potassium carbonate, sodium carbonate, sodium hydroxide, potassium hydroxide, sodium hydride, potassium hydride, etc.

The amount of the base used is preferably 1 to 10 equivalents.

As the compound (VII), there are used commercially available alkyl chlorides, alkyl bromides or alkyl iodides, or those synthesized by the methods described in SHIN-JIKKEN KAGAKU KOZA, 14 (I), ORGANIC FUNCTIONAL GROUP PREPARATIONS, 2nd Ed., Academic Press Inc., 1989; Comprehensive Organic Transformations, VCH Publishers Inc., 1989, etc.

The amount used is preferably from 1 to 20 equivalents.

The "inert solvent" includes, for example, an alcoholic solvent, an ethereal solvent, a halogenated hydrocarbon solvent, an aromatic solvent, a nitrile solvent, an amide solvent, a ketone solvent, a sulfoxide solvent, etc. These solvents may be used by mixing two or more in a suitable ratio. Among these solvents, preferred are acetonitrile, DMF, DMSO, acetone, etc.

The reaction temperature is generally from approximately -20°C to 100°C, preferably from room temperature to 80°C. The reaction time is, for example, approximately from 0.5 to 48 hours.

### PROCESS 4:

The compound (Ic) wherein X is O and Y is CH can be manufactured by subjecting the compound (IV) obtained in PROCESS 2 and the compound (VIII) to condensation. For the condensation, the Mitsunobu reaction (the method described in Synthesis, 1 (1980)), etc. are available. 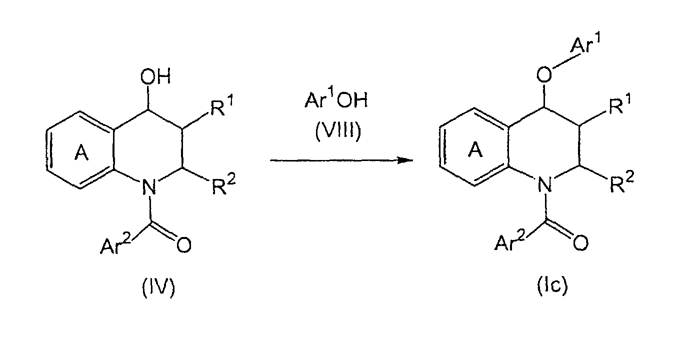 [wherein the symbols have the same significance as defined above].

As the compound Ar¹OH, there are used commercially available phenols, or phenols synthesized by the methods described in SHIN-JTKKEN KAGAKU KOZA, 14 (I), ORGANIC FUNCTIONAL GROUP PREPARATIONS, 2nd Ed., Academic Press Inc., 1989; Comprehensive Organic Transformations, VCH Publishers Inc., 1989, etc.

In the Mitsunobu reaction, the compound (IV) and 0.5 to 5 equivalents (preferably 1 to 1.5 equivalents) of the compound (VIII) are reacted in an inert solvent in the co-presence of 0.5 to 5 equivalents (preferably 1 to 1.5 equivalents) of an azodicarboxylic acid diester and a triarylphosphine or a trialkylphosphine.

As the azodicarboxylic acid diester, dimethyl azodicarboxylate, diethyl azodicarboxylate, diisopropyl azodicarboxylate, etc. are preferably employed.

As the phosphine, triphenylphosphine, tributylphosphine, etc. are preferably used.

The inert solvent includes, for example, an ethereal solvent, a halogenated hydrocarbon solvent, an aromatic solvent, a nitrile solvent, an amide solvent, a ketone solvent, a sulfoxide solvent, etc. These solvents may be used by mixing two or more in a suitable ratio. Among them, THF, acetonitrile, dichloromethane, chloroform, etc. are preferably used.

The reaction temperature is generally from -20°C to 50°C, preferably at room temperature. The reaction time is normally from 5 minutes to 40 hours, preferably 1 to 18 hours.

### PROCESS 5:

The compound (Id) wherein X is CR³R⁴ and Y is N can be manufactured from the compound (X) and the compound (XI) [wherein Z is a reactive substituent] in a manner similar to PROCESS 3. 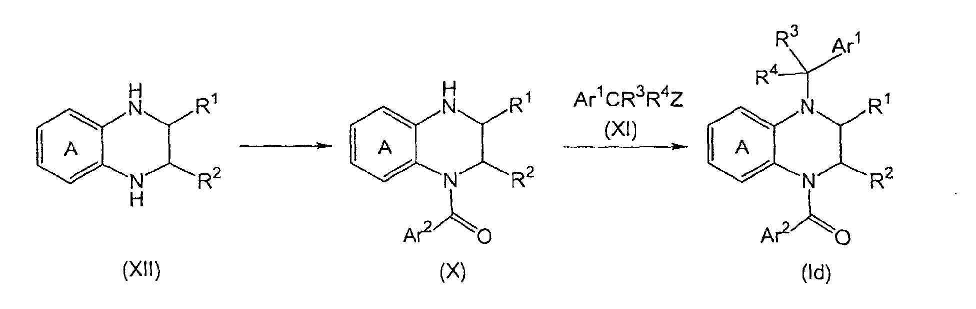 [wherein the symbols have the same significance as defined above].

As the compound (XI), there are used commercially available alkyl chlorides, alkyl bromides or alkyl iodides, or those synthesized by the methods described in SHIN-JIKKEN KAGAKU KOZA, 14(1), ORGANIC FUNCTIONAL GROUP PREPARATIONS, 2nd Ed., Academic Press Inc., 1989; Comprehensive Organic Transformations, VCH Publishers Inc., 1989, etc.

The compound (X) can be manufactured by acylating the compound (XII) as in PROCESS 1. The compound (X) can be manufactured by publicly known methods (e.g., the method described in Journal of Pharmaceutical Bulletin, 32 (6), 2421 (1984), etc.).

In the present invention, the compound represented by the formula (II) described above, its salts or prodrugs thereof are employed.

In the lower alkyl group which may optionally be substituted, which is shown by R¹ and R², the "lower alkyl group" includes a C₁₋₆ alkyl (e.g., methyl, ethyl, propyl, isopropyl, butyl, isobutyl, sec-butyl, tert-butyl, pentyl, hexyl, etc.) and the like. Preferred are methyl, ethyl and propyl.

In the compounds represented by formula (II) described above, salts thereof, or prodrugs thereof, the same description on the "substituent" for the "lower alkyl group which may optionally be substituted" shown by R³, R⁴ and R⁵, which was made on the compounds above having the soluble beta amyloid precursor protein secretion promoting activity and apoptosis inhibiting activity, salts thereof, or prodrugs thereof, applies to the "substituent" for the "lower alkyl group which may optionally be substituted" shown by R¹ and R², in the formula (II) described above.

In the formula (II) described above, the compounds wherein R¹ and R² are combined to form a 4- to 7-membered ring together with the adjacent carbon atoms include: 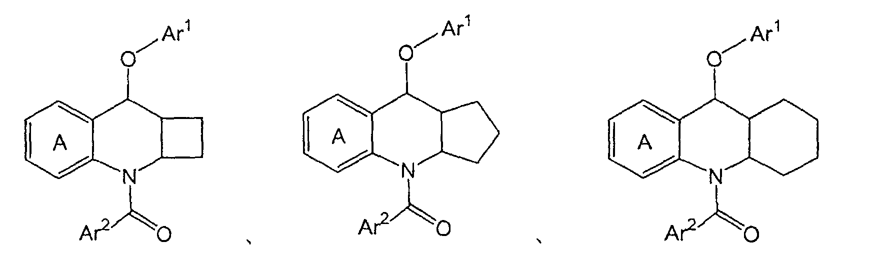 or 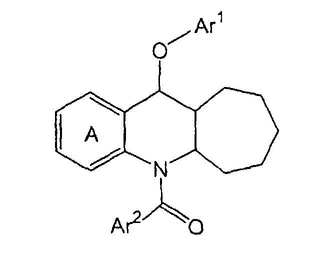 [wherein the symbols have the same significance as defined above], and the like.

For R¹, hydrogen atom is preferred; and for R², hydrogen atom or an optionally substituted lower alkyl group is preferred, more preferably, a C₁₋₆ alkyl group, and most preferably, methyl, etc.

For X, NR⁵ (wherein R⁵ has the same significance as defined above); and CH is preferably used for Y.

Each of Ar¹ and Ar² represents an aromatic group, which may optionally be substituted.

In the compounds represented by formula (II) described above, salts thereof, or prodrugs thereof, the same description on the "aromatic group" for the "aromatic group which may optionally be substituted" shown by Ar¹ and Ar², which was made on the compounds above having the soluble beta amyloid precursor protein secretion promoting activity and apoptosis inhibiting activity, salts thereof, or prodrugs thereof, applies to the "aromatic group" for the "aromatic group which may optionally be substituted" shown by Ar¹ and Ar², in the formula (II) described above.

In the aromatic groups shown by Ar¹ and Ar² described above, preferred examples are:
(1) monocyclic aromatic groups such as phenyl; a thienyl such as 2- or 3-thienyl, etc.; a furyl such as 2- or 2-furyl, etc.; a pyridyl such as 2-, 3- or 4-pyridyl, etc.;
(2) ring-assembled aromatic groups, e.g., a biphenylyl such as 2-, 3- or 4-biphenylyl, etc.; a naphthyl-substituted oxadiazolyl such as 3-(1-naphthyl)-1,2,4-oxadiazol-5-yl or 3-(2-naphthyl)-1,2,4-oxadiazol-5-yl, etc.; a benzofuranyl-substituted oxadiazolyl such as 3-(2-benzofuranyl)-1,2,4-oxadiazol-5-yl, etc.; a phenyl-substituted oxadiazolyl such as 3-phenyl-1,2,4-oxadiazol-5-yl, etc.; a benzoxazolyl-substituted oxadiazolyl such as 3-(2-benzoxazolyl)-1,2,4-oxadiazol-2-yl, etc.; an indolyl-substituted oxadiazolyl such as 3-(3-indolyl)-1,2,4-oxadiazol-2-yl or 3-(2-indolyl)-1,2,4-oxadiazol-2-yl, etc.; a phenyl-substituted thiazolyl such as 4-phenylthiazol-2-yl, etc.; a benzofuranyl-substituted thiazolyl such as 4-(2-benzofuranyl)thiazol-2-yl, etc.; a phenyl-substituted oxazolyl such as 4-phenyl-1,3-oxazol-5-yl, etc.; a thienyl-substituted phenyl such as 4-(2-thienyl)phenyl, etc.; a pyridyl-substituted phenyl such as 4-(3-pyridyl)phenyl, etc.; a naphthyl-substituted phenyl such as 4-(2-naphthyl)phenyl, etc.; a terphenylyl such as 4,4'-terphenylyl, etc.; and,
(3) fused aromatic groups, e.g., a quinolyl such as 2-, 3- or 4-quinolyl, etc.; an indolyl such as 1-, 2- or 3-indolyl, etc.

For the aromatic group shown by Ar¹, more preferred are monocyclic aromatic groups, e.g., phenyl, etc., and fused aromatic groups such as 2-quinolyl, 3-quinolyl or 4-quinolyl, etc. Phenyl is particularly preferably used.

For the aromatic group shown by Ar², more preferred are monocyclic aromatic groups, e.g., phenyl, a thienyl such as 2- or 3-thienyl, etc., a furyl such as 2- or 3-furyl, etc., a pyridyl such as 2-, 3- or 4-pyridyl, etc.

In the compounds represented by formula (II) described above, salts thereof, or prodrugs thereof, the same description on the "substituent" for the "aromatic group which may optionally be substituted," "optionally substituted monocyclic aromatic group" and "optionally substituted phenyl group" shown by Ar¹ and Ar², for the "optionally substituted phenyl group or optionally substituted quinolyl group" shown by Ar¹, for the "optionally substituted phenyl, furyl, thienyl or pyridyl group" shown by Ar², and for the "optionally substituted benzene ring" shown by the ring A, which was made on the compounds above having the soluble beta amyloid precursor protein secretion promoting activity and apoptosis inhibiting activity, salts thereof, or prodrugs thereof, applies to the "substituent" for the "aromatic group which may optionally be substituted," "optionally substituted monocyclic aromatic group" and "optionally substituted phenyl group" shown by Ar¹ and Ar², for the "optionally substituted phenyl group or optionally substituted quinolyl group" shown by Ar¹, for the "optionally substituted phenyl, furyl, thienyl or pyridyl group" shown by Ar², and for the "optionally substituted benzene ring" shown by the ring A, in the formula (II) described above.

In the compounds represented by formula (II), hydrogen atom is preferred for R¹.

For R², hydrogen atom or an optionally substituted lower alkyl group is preferred, more preferably hydrogen atom or a C₁₋₆ alkyl group, and particularly preferably methyl, etc.

For Ar¹, preferred is an optionally substituted phenyl group or an optionally substituted quinolyl group; an optionally substituted phenyl group is more preferred, and particularly preferred is a phenyl group which may optionally be substituted with a halogen atom, a C₁₋₃ alkylenedioxy group, an optionally halogenated C₁₋₆ alkyl group or an optionally halogenated C₁₋₆ alkoxy group.

For Ar², an optionally substituted monocyclic aromatic group is preferred; a phenyl, furyl, thienyl or pyridyl group, each of which may optionally be substituted, is more preferred, much more preferred is a phenyl, furyl, thienyl or pyridyl group, each of which may optionally be substituted with a halogen atom, a C₁₋₃ alkylenedioxy group, an optionally halogenated C₁₋₆ alkyl group or an optionally halogenated C₁₋₆ alkoxy group, and most preferred is 3,4-dimethoxyphenyl group.

For the ring A, unsubstituted benzene ring is preferred.

In the compounds represented by the formula (II) described above, particularly preferably employed are the compounds wherein R¹ is hydrogen atom, R² is methyl group, Ar¹ is a phenyl group which may optionally be substituted, Ar² is 3,4-dimethoxyphenyl group, and the ring A is unsubstituted benzene ring,

As the salts of the compounds represented by the formula (II) described above (hereinafter sometimes simply referred to as the compound (II)), pharmaceutically acceptable salts are preferred, and examples are salts with inorganic bases, salts with organic bases, salts with inorganic acids, salts with organic acids, salts with basic or acidic amino acids, etc.

Preferred examples of salts with inorganic bases include alkali metal salts such as sodium salts, potassium salts, etc.; alkaline earth metal salts such as calcium salts, magnesium salts, etc.; aluminum salts, ammonium salts, etc.

Preferred examples of salts with organic bases include salts with trimethylamine, triethylamine, pyridine, picoline, ethanolamine, diethanolamine, triethanolamine, dicyclohexylamine, N',N'-dibenzylethylenediamine, etc.

Preferred examples of the salts with inorganic acids are salts with hydrochloric acid, hydrobromic acid, nitric acid, sulfuric acid, phosphoric acid, etc.

Preferred examples of the salts with organic acids are salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.

Preferred examples of the salts with basic amino acids are salts with arginine, lysine, ornithine, etc., and preferred examples of the salts with acidic amino acids are salts with aspartic acid, glutamic acid, etc.

The prodrugs of the compound (II) refer to compounds that are converted into the compound (II) through reactions associated with enzymes, gastric acid, etc. in vivo under the physiological conditions, that is, compounds which are converted into the compound (II) by oxidation, reduction, hydrolysis, etc. caused enzymatically; or compounds which are converted into the compound (II) through hydrolysis by gastric acid, etc. Examples of the prodrugs of the compound (II) include a compound wherein an amino group of the compound (II) is acylated, alkylated or phosphorylated (e.g., a compound wherein an amino group of the compound (II) is substituted with eicosanoyl, alanyl, pentylaminocarbonyl, (5-methyl-2-oxo-1,3-dioxolen-4-yl)methoxycarbonyl, tetrahydrofuranyl, tetrahydropyranyl, pyrrolidylmethyl, pivaloyloxymethyl, tert-butyl, etc.); a compound wherein a hydroxy group of the compound (II) is acylated, alkylated, phosphorylated or borated (e.g., a compound wherein a hydroxy group of the compound (II) is substituted with acetyl, palmitoyl, propanoyl, pivaloyl, succinyl, fumaryl, alanyl, dimethylaminomethylcarbonyl, tetrahydropyranyl, etc.); a compound wherein a carboxyl group of the compound (II) is esterified or amidated (e.g., a compound wherein a carboxyl group of the compound (II) is converted into the ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); and the like. These compounds can be manufactured from the compound (II) by publicly known methods.

The compound (II) may also be labeled with an isotope (e.g., ³H, ¹⁴C, ³⁵S, ¹²⁵I, etc.) or the like.

The compound (II) may also be in the form of anhydrides or hydrates thereof.

The processes of manufacturing the compound (II) are described below.

The following description of the manufacturing processes are applied not only to the compound (II) but also to its salts and prodrugs thereof, but in the following description they are sometimes merely referred to as the compound (II). Likewise, the respective compounds used to manufacture the compound (II) include salts thereof as well, even when they are simply referred to as, e.g., Compound (II').

The compound (II) can be manufactured, e.g., by the methods given below, using publicly known procedures.

The thus obtained compound (II) can be isolated and purified from reaction solutions by any publicly known procedures, for example, through solvent extraction, concentration, neutralization, filtration, crystallization, recrystallization, column chromatography, high performance liquid chromatography, recrystallization, etc., whereby the product can be isolated and purified in a high purity.

The compound (II) may also be in the form of hydrates or anhydrides thereof. The hydrates include monohydrates, 0.5 hydrates, dihydrates, etc.

Furthermore, the compound (II) may be labeled with an isotope (e.g., ³H, ¹⁴C, ³⁵S, ¹²⁵I, etc.) or the like.

Where the compound (II) of the present invention or salts thereof contain an asymmetric carbon, the resulting optically active mixture (racemate) can be resolved to the respective optically active compounds by conventional optical resolution means, for example, through formation of salts with optically active acids (e.g., camphor sulfonic acid, etc.) or optically active bases (e.g., 1-methylbenzylamine, etc.), or by separation means such as various chromatographies (e.g., liquid chromatography using an optically active column, etc.), fractional recrystallization, etc.

The "room temperature" means normally 0 to 30°C.

The symbols given in the chemical structures described in the manufacturing processes have the same significance as defined above, unless otherwise indicated.

Hereinafter the processes of manufacturing the compound (II) are specifically described.

### PROCESS 6:

The compound (II) can be manufactured by subjecting the compound (IV) obtained in PROCESS 2 described above and the compound (VIII) to condensation. For the condensation, the Mitsunobu reaction (the method described in Synthesis, 1 (1980)), etc. are available. 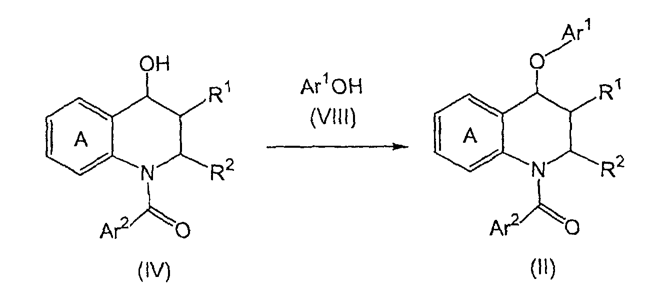 [wherein the symbols have the same significance as defined above].

As the compound Ar¹OH, there are used commercially available phenols, or phenols synthesized by the methods described in SHIN-JIKKEN KAGAKU KOZA, 14 (I), ORGANIC FUNCTIONAL GROUP PREPARATIONS, 2nd Ed., Academic Press Inc., 1989; Comprehensive Organic Transformations, VCH Publishers Inc., 1989, etc.

In the Mitsunobu reaction, the compound (IV) and 0.5 to 5 equivalents (preferably 1 to 1.5 equivalents) of the compound (VIII) are reacted in an inert solvent in the co-presence of 0.5 to 5 equivalents (preferably 1 to 1.5 equivalents) of an azodicarboxylic acid diester and a triarylphosphine or a trialkylphosphine.

As the azodicarboxylic acid diester, dimethyl azodicarboxylate, diethyl azodicarboxylate, diisopropyl azodicarboxylate, etc. are preferably employed. As the phosphine, triphenylphosphine, tributylphosphine, etc. are preferably used.

The inert solvent includes, for example, an ethereal solvent, a halogenated hydrocarbon solvent, an aromatic solvent, a nitrile solvent, an amide solvent, a ketone solvent, a sulfoxide solvent, etc. These solvents may be used by mixing two or more in a suitable ratio. Among them, THF, acetonitrile, dichloromethane, chloroform, etc. are preferably used.

The reaction temperature is generally from -20°C to 50°C, preferably at room temperature. The reaction time is normally from 5 minutes to 40 hours, preferably 1 to 18 hours.

In the present invention, the compound represented by the formula (III) described above, its salts or prodrugs thereof are employed.

In the lower alkyl group which may optionally be substituted, which is shown by R¹, R², R³, R⁴ and R⁵, the "lower alkyl group" includes a C₁₋₆ alkyl (e.g., methyl, ethyl, propyl, isopropyl, butyl, isobutyl, sec-butyl, tert-butyl, pentyl, hexyl, etc.) and the like. Preferred are methyl, ethyl and propyl.

In the compounds represented by formula (III) described above, salts thereof, or prodrugs thereof, the same description on the "substituent" for the "lower alkyl group which may optionally be substituted" shown by R³, R⁴ and R⁵, which was made on the compounds above having the soluble beta amyloid precursor protein secretion promoting activity and apoptosis inhibiting activity, salts thereof, or prodrugs thereof, applies to the "substituent" for the "lower alkyl group which may optionally be substituted" shown by R¹, R², R³, R⁴ and R⁵, in the formula (III) described above.

In the formula (III) described above, the compounds wherein R¹ and R² are combined to form a 4- to 7-membered ring together with the adjacent carbon atoms include: 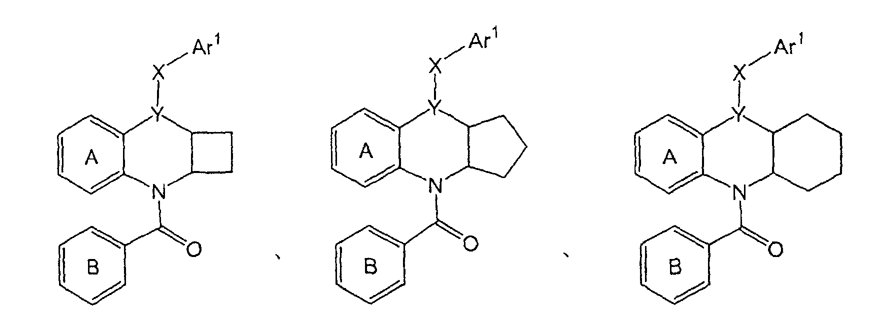 or 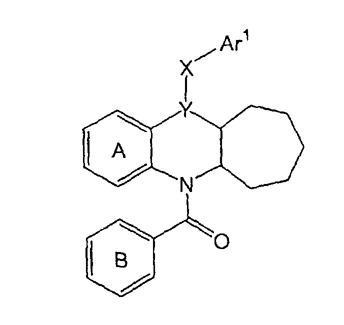 [wherein the symbols have the same significance as defined above], and the like.

For R¹, hydrogen atom is preferred; and for R², hydrogen atom or an optionally substituted lower alkyl group is preferred, more preferably, a C₁₋₆ alkyl group, and most preferably, methyl, etc.

For X, NR⁵ (wherein R⁵ has the same significance as defined above); and CH is preferably used for Y.

Ar¹ represents an aromatic group which may optionally be substituted.

In the compounds represented by formula (III) described above, salts thereof, or prodrugs thereof, the same description on the "aromatic group" for the "aromatic group which may optionally be substituted" shown by Ar¹ and Ar², which was made on the compounds above having the soluble beta amyloid precursor protein secretion promoting activity and apoptosis inhibiting activity, salts thereof, or prodrugs thereof, applies to the "aromatic group" for the "aromatic group which may optionally be substituted" shown by Ar¹, in the formula (III) described above.

In the aromatic groups shown by Ar¹ described above, preferred examples are:
(1) monocyclic aromatic groups such as phenyl; a thienyl such as 2- or 3-thienyl, etc.; a furyl such as 2- or 2-furyl, etc.; a pyridyl such as 2-, 3- or 4-pyridyl, etc.;
(2) ring-assembled aromatic groups, e.g., a biphenylyl such as 2-, 3- or 4-biphenylyl, etc.; a naphthyl-substituted oxadiazolyl such as 3-(1-naphthyl)-1,2,4-oxadiazol-5-yl or 3-(2-naphthyl)-1,2,4-oxadiazol-5-yl, etc.; a benzofuranyl-substituted oxadiazolyl such as 3-(2-benzofuranyl)-1,2,4-oxadiazol-5-yl, etc.; a phenyl-substituted oxadiazolyl such as 3-phenyl-1,2,4-oxadiazol-5-yl, etc.; a benzoxazolyl-substituted oxadiazolyl such as 3-(2-benzoxazolyl)-1,2,4-oxadiazol-2-yl, etc.; an indolyl-substituted oxadiazolyl such as 3-(3-indolyl)-1,2,4-oxadiazol-2-yl or 3-(2-indolyl)-1,2,4-oxadiazol-2-yl, etc.; a phenyl-substituted thiazolyl such as 4-phenylthiazol-2-yl, etc.; a benzofuranyl-substituted thiazolyl such as 4-(2-benzofuranyl)thiazol-2-yl, etc.; a phenyl-substituted oxazolyl such as 4-phenyl-1,3-oxazol-5-yl, etc.; a thienyl-substituted phenyl such as 4-(2-thienyl)phenyl, etc.; a pyridyl-substituted phenyl such as 4-(3-pyridyl)phenyl, etc.; a naphthyl-substituted phenyl such as 4-(2-naphthyl)phenyl, etc.; a terphenylyl such as 4,4'-terphenylyl, etc.; and,
(3) fused aromatic groups, e.g., a quinolyl such as 2-, 3- or 4-quinolyl, etc.; an indolyl such as 1-, 2- or 3-indolyl, etc.

For the aromatic group shown by Ar¹, more preferred are monocyclic aromatic groups, e.g., phenyl, etc., and fused aromatic groups such as 2-quinolyl, 3-quinolyl or 4-quinolyl, etc. Phenyl is particularly preferably used.

In the compounds represented by formula (III) described above, salts thereof, or prodrugs thereof, the same description on the "substituent" for the "aromatic group which may optionally be substituted," "optionally substituted monocyclic aromatic group" and "optionally substituted phenyl group" shown by Ar¹ and Ar², for the "optionally substituted phenyl group or optionally substituted quinolyl group" shown by Ar¹, for the "optionally substituted phenyl, furyl, thienyl or pyridyl group" shown by Ar², and for the "optionally substituted benzene ring" shown by the ring A, which was made on the compounds above having the soluble beta amyloid precursor protein secretion promoting activity and apoptosis inhibiting activity, salts thereof, or prodrugs thereof, applies to the "substituent" for the "aromatic group which may optionally be substituted," "optionally substituted monocyclic aromatic group" and "optionally substituted phenyl group" shown by Ar¹, and for the "optionally substituted benzene ring" shown by the ring A, in the formula (III) described above.

In the compounds represented by formula (III), hydrogen atom is preferred for R¹.

For R², hydrogen atom or an optionally substituted lower alkyl group is preferred, more preferably a C₁₋₆ alkyl group, and particularly preferably methyl, etc.

For Ar¹, preferred is an optionally substituted phenyl group or an optionally substituted quinolyl group; an optionally substituted phenyl group is more preferred, and particularly preferred is a phenyl group which may optionally be substituted with a halogen atom, a C₁₋₃ alkylenedioxy group, an optionally halogenated C₁₋₆ alkyl group or an optionally halogenated C₁₋₆ alkoxy group.

For the ring A, unsubstituted benzene ring is preferred.

For the ring B, preferred is a benzene ring, which may optionally be substituted with a C₁₋₆ alkoxy group, hydroxy group or a C₁₋₃ alkylenedioxy group.

For X, NR⁵ (wherein R⁵ has the same significance as defined above)

For Y, CH is preferably used.

In the compounds represented by the formula (III) described above, particularly preferably used are the compounds wherein R¹ is hydrogen atom, R² is methyl group, Ar¹ is a phenyl group which may optionally be substituted, the ring A is unsubstituted benzene ring, X is NR⁵ (wherein R⁵ has the same significance as defined above), Y is CH (provided that those wherein X is NH, Y is CH and Ar¹ is unsubstituted phenyl group).

As the salts of the compounds represented by the formula (III) described above (hereinafter sometimes simply referred to as the compound (III)), pharmaceutically acceptable salts are preferred, and examples are salts with inorganic bases, salts with organic bases, salts with inorganic acids, salts with organic acids, salts with basic or acidic amino acids, etc.

Preferred examples of salts with inorganic bases include alkali metal salts such as sodium salts, potassium salts, etc.; alkaline earth metal salts such as calcium salts, magnesium salts, etc.; aluminum salts, ammonium salts, etc.

Preferred examples of salts with organic bases include salts with trimethylamine, triethylamine, pyridine, picoline, ethanolamine, diethanolamine, triethanolamine, dicyclohexylamine, N',N'-dibenzylethylenediamine, etc.

Preferred examples of the salts with inorganic acids are salts with hydrochloric acid, hydrobromic acid, nitric acid, sulfuric acid, phosphoric acid, etc.

Preferred examples of the salts with organic acids are salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.

Preferred examples of the salts with basic amino acids are salts with arginine, lysine, ornithine, etc., and preferred examples of the salts with acidic amino acids are salts with aspartic acid, glutamic acid, etc.

The prodrugs of the compound (III) refer to compounds that are converted into the compound (III) through reactions associated with enzymes, gastric acid, etc. in vivo under the physiological conditions, that is, compounds which are converted into the compound (III) by oxidation, reduction, hydrolysis, etc. caused enzymatically; or compounds which are converted into the compound (III) through hydrolysis by gastric acid, etc. Examples of the prodrugs of the compound (III) include a compound wherein an amino group of the compound (III) is acylated, alkylated or phosphorylated (e.g., a compound wherein an amino group of the compound (III) is substituted with eicosanoyl, alanyl, pentylaminocarbonyl, (5-methyl-2-oxo-1,3-dioxolen-4-yl)methoxycarbonyl, tetrahydrofuranyl, tetrahydropyranyl, pyrrolidylmethyl, pivaloyloxymethyl, tert-butyl, etc.); a compound wherein a hydroxy group of the compound (III) is acylated, alkylated, phosphorylated or borated (e.g., a compound wherein a hydroxy group of the compound (III) is substituted with acetyl, palmitoyl, propanoyl, pivaloyl, succinyl, fumaryl, alanyl, dimethylaminomethylcarbonyl, tetrahydropyranyl, etc.); a compound wherein a carboxyl group of the compound (III) is esterified or amidated (e.g., a compound wherein a carboxyl group of the compound (III) is converted into the ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); and the like. These compounds can be manufactured from the compound (III) by publicly known methods.

The compound (III) may also be labeled with an isotope (e.g., ³H, ¹⁴C, ³⁵S, ¹²⁵I, etc.) or the like.

The compound (III) may also be in the form of anhydrides or hydrates thereof.

The processes of manufacturing the compound (III) are described below.

The following description of the manufacturing processes are applied not only to the compound (III) but also to its salts and prodrugs thereof, but in the following description they are sometimes merely referred to as the compound (III). Likewise, the respective compounds used to manufacture the compound (III) include salts thereof as well, even when they are simply referred to as, e.g., Compound (III').

The compound (III) can be manufactured, e.g., by the methods given below, using publicly known procedures.

The thus obtained compound (III) can be isolated and purified from reaction solutions by any publicly known procedures, for example, through solvent extraction, concentration, neutralization, filtration, crystallization, recrystallization, column chromatography, high performance liquid chromatography, recrystallization, etc., whereby the product can be isolated and purified in a high purity.

The compound (III) may also be in the form of hydrates or anhydrides thereof. The hydrates include monohydrates, 0.5 hydrates, dihydrates, etc.

Furthermore, the compound (III) may be labeled with an isotope (e.g., ³H, ¹⁴C, ³⁵S, ¹²⁵I, etc.) or the like.

Where the compound (III) of the present invention or salts thereof contain an asymmetric carbon, the resulting optically active mixture (racemate) can be resolved to the respective optically active compounds by conventional optical resolution means, for example, through formation of salts with optically active acids (e.g., camphor sulfonic acid, etc.) or optically active bases (e.g., 1-methylbenzylamine, etc.), or by separation means such as various chromatographies (e.g., liquid chromatography using an optically active column, etc.), fractional recrystallization, etc.

The "room temperature" means normally 0 to 30°C.

The symbols given in the chemical structures described in the manufacturing processes have the same significance as defined above, unless otherwise indicated.

Hereinafter the processes of manufacturing the compound (III) are specifically described.

### PROCESS 7:

The compound (III) can be manufactured normally by PROCESS 7 described below. The compound (III') can be manufactured by publicly known methods described in, e.g., Bulletin of the Chemical Society of Japan, 42, 2885-2894 (1969) or Can. J. CHEM., 52 (1974), or methods referred to in these journals. 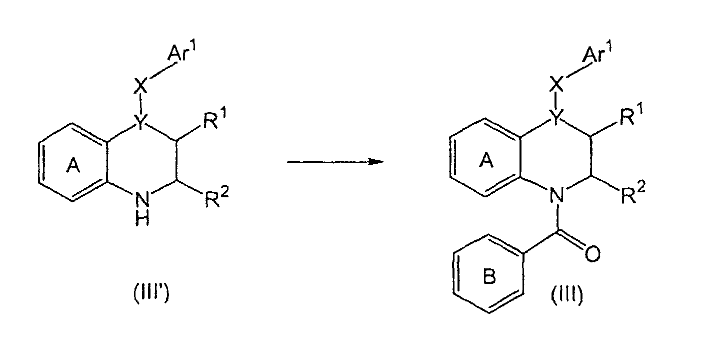 [wherein the symbols have the same significance as defined above].

The reaction described in PROCESS 7 is amidation, which can be effected, in the presence of a dehydrating and condensing agent, or by converting the carboxy once to its reactive derivative followed by condensation, or the like.
i) Process using a dehydrating/condensing agent
   The compound (III') or its salt, 1 to 5 equivalents of the compound shown by formula (III'): 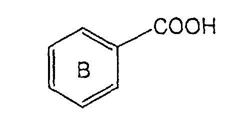 [wherein the ring B has the same significance as defined above], and 1 to 2 equivalents of a dehydrating/condensing agent are reacted in an inert solvent at room temperature for 10 to 24 hours. If necessary, the reaction may be carried out by adding 1 to 1.5 equivalents of 1-hydroxybenzotriazole (HOBT) and/or a catalytic amount to 5 equivalents of a base (e.g., triethylamine, 4-dimethylaminopyridine, etc.).
   Examples of the "dehydrating/condensing agent" are dicyclohexylcarbodiimide (DCC), 1-ethyl-3-(3-dimethylaminopropyl)carbodiimide hydrochloride (WSC), etc. 5 Among them, WSC is preferred.
   For the inert solvent, for example, a nitrile solvent (preferably acetonitrile), an amide solvent (preferably dimethylformamide (DMF)), a halogenated hydrocarbon solvent (preferably dichloromethane), an ethereal solvent (preferably tetrahydrofuran (THF)), etc. may be used either singly or as a mixture of two or more.
ii) Process using the reactive derivative of carboxy
   The reactive derivative of the compound (III") and 1 to 5 equivalents (preferably 1 to 3 equivalents) of the compound (III') are reacted in an inert solvent at -20°C to 50°C (preferably at room temperature) for 5 minutes to 40 hours (preferably 1 to 18 hours). If necessary, the reaction may be carried out in the presence of 1 to 10 equivalents, preferably 1 to 3 equivalents of a base.

The "reactive derivative" of the compound (III") include acid halides (e.g., acid chlorides, acid bromides, etc.), mixed acid anhydrides (e.g., acid anhydrides with C₁₋₆ alkyl-carboxylic acids, C₆₋₁₀ aryl-carboxylic acids or C₁₋₆ alkyl-carbonic acids, etc.), activated esters (e.g., esters with optionally substituted phenols, 1-hydroxybenzotriazole or N-hydroxysuccinimide, etc.), and the like. The "substituent" for the "optionally substituted phenol" includes 1 to 5 substituents selected from a halogen atom, nitro, an optionally halogenated C₁₋₆ alkyl and an optionally halogenated C₁₋₆ alkoxy. Specific examples of the "optionally substituted phenol" are phenol, pentachlorophenol, pentafluorophenol, p-nitrophenol, etc. Acid halides are preferable as the reactive derivative.

Preferred examples of the "base" are sodium hydride, potassium carbonate, sodium carbonate, sodium hydroxide, potassium hydroxide, sodium hydrogencarbonate, potassium hydrogencarbonate, triethylamine, pyridine, etc.

For the inert solvent, for example, an ethereal solvent, a halogenated hydrocarbon solvent, an aromatic solvent, a nitrile solvent, an amide solvent, a ketone solvent, a sulfoxide solvent, water, etc. may be used either singly or as a mixture of two or more. Among them, pyridine, acetonitrile, THF, dichloromethane, chloroform, etc. are preferably used, more preferably, pyridine, THF, acetonitrile, etc.

### PROCESS 8:

The compound (IIIa) wherein X is NR⁵ and Y is CH can be manufactured by converting the hydroxy group of the compound (IV') to its reactive substituent, e.g., in accordance with the method described in the literature (Tetrahedron Letters, 38 (15), 2673 (1997)), etc. and then substituting with the compound (IX) in the presence of a base. 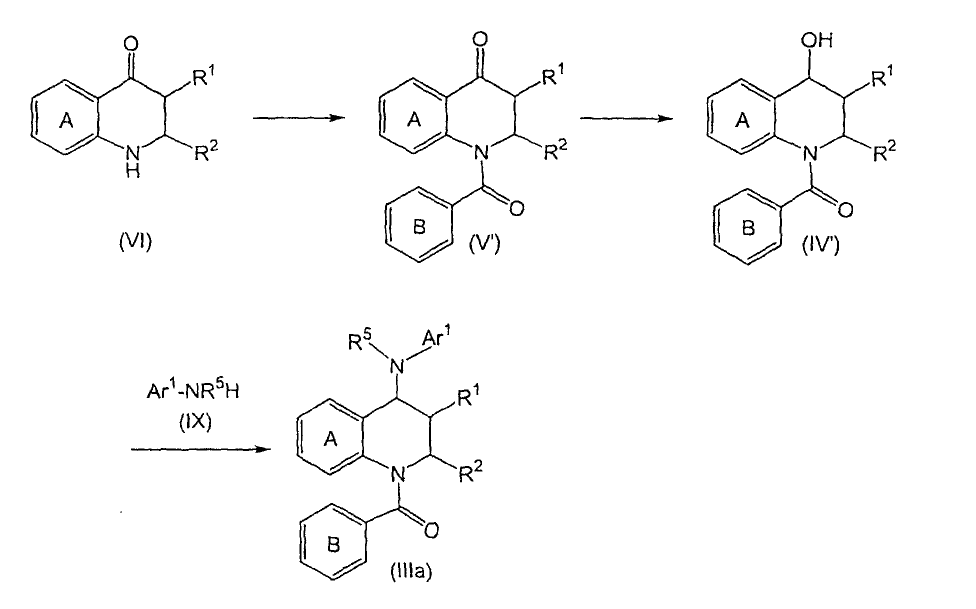 [wherein the symbols have the same significance as defined above].

The reactive substituent includes, for example, a halogen atom (e.g., chlorine, bromine, iodine, etc.), an optionally halogenated C₁₋₆ alkylsulfonyloxy (e.g., methanesulfonyloxy, ethanesulfonyloxy, trifluoromethanesulfonyloxy, etc.), an optionally substituted C₆₋₁₀ arylsulfonyloxy, etc.

The "substituent" for the "optionally substituted C₆₋₁₀ arylsulfonyloxy" includes a halogen atom, an optionally halogenated C₁₋₆ alkyl or C₁₋₆ alkoxy, etc. The number of the substituents is, e.g., 1 to 3. Specific examples of the "optionally substituted C₆₋₁₀ arylsulfonyloxy" are benzenesulfonyloxy, p-toluenesulfonyloxy, 1-naphthalenesulfonyloxy, 2-naphthalenesulfonyloxy, etc.

Preferred examples of the "reactive substituent" include a halogen atom (e.g., chlorine, bromine, iodine, etc.), methanesulfonyloxy, trifluoromethanesulfonyloxy, p-toluenesulfonyloxy, etc.

The halogenation is carried out, e.g., by reacting the compound (IV') with 1 to 10 equivalents of a halogenating agent in an inert solvent.

Examples of the halogenating agent are inorganic halides such as thionyl chloride, thionyl bromide, phosphorus trichloride, phosphorus pentachloride, phosphorus oxychloride, etc.; hydrohalogenic acids such as hydrochloric acid, hydrobromic acid, etc.; or silyl halides such as iodotrimethylsilane, etc. Preferably, iodotrimethylsilane is used.

The "inert solvent" includes an ethereal solvent, a halogenated hydrocarbon solvent, an aromatic solvent, a nitrile solvent, an amide solvent, a sulfoxide solvent, etc.

The reaction temperature is between -20°C and 200°C, preferably between 0°C and 100°C. The reaction time ranges from 0.1 to 48 hours, preferably 1 to 24 hours.

The sulfonylation is carried out, e.g., by reacting the compound (IV') and 1 to 5 equivalents of the corresponding sulfonyl halide in an inert solvent in the presence of a base.

The base is preferably potassium carbonate, sodium hydrogencarbonate, triethylamine, N-methylmorpholine, pyridine, etc. The amount of the base used is preferably from 1 to 10 equivalents.

The "inert solvent" includes an ethereal solvent, a halogenated hydrocarbon solvent, an aromatic solvent, a nitrile solvent, an amide solvent, a ketone solvent, a sulfoxide solvent, etc.

The reaction temperature is between -20°C and 200°C, preferably between 0°C and 100°C. The reaction time ranges from 0.1 to 48 hours, preferably 1 to 24 hours.

As the compound (IX), there are used commercially available anilines or anilines synthesized by the methods described in SHIN-JIKKEN KAGAKU KOZA, 14 (III), ORGANIC FUNCTIONAL GROUP PREPARATIONS, 2nd Ed., Academic Press Inc., 1989, Comprehensive Organic Transformations, VCH Publishers Inc., 1989, etc. The amount used is preferably from 1 to 10 equivalents.

As the base, those described above are used; preferred examples include potassium carbonate, barium carbonate, sodium hydrogencarbonate, triethylamine, N-methylmorpholine, pyridine, etc. The amount of the base used is preferably from 1 to 10 equivalents.

The reaction is carried out in an inert solvent. The "inert solvent" includes an ethereal solvent, a halogenated hydrocarbon solvent, an aromatic solvent, a nitrile solvent, an amide solvent, a ketone solvent, a sulfoxide solvent, etc.

The reaction temperature is between -20°C and 200°C, preferably between 0°C and 50°C. The reaction time ranges from 0.1 to 48 hours, preferably 1 to 24 hours.

The compound (IV') can be manufactured by reducing the compound (V') with an appropriate reducing agent in an inert solvent.

The "inert solvent" includes an alcoholic solvent, an ethereal solvent, a halogenated hydrocarbon solvent, an aromatic solvent, a nitrile solvent, an amide solvent, an organic acid solvent, etc. These solvents may be used by mixing two or more in a suitable ratio. Among these solvents, preferred are methanol, ethanol, etc.

Examples of the reducing agent include sodium borohydride, sodium triacetoxyborohydride, sodium cyanoborohydride, lithium aluminum hydride, etc. The amount of the reducing agent used is generally 1 to 20 equivalents, preferably 1 to 5 equivalents.

The reaction temperature is generally from -20°C to 150°C, preferably from 0°C to 50°C. The reaction time is generally from 5 minutes to 24 hours, preferably 1 to 12 hours.

The compound (V') can be manufactured by acylating the compound (VI) as in PROCESS 1.

The compound (VI) can be manufactured by publicly known methods (e.g., by the method described in Bio-Organic & Medicinal Chemistry Letters, 9, 1009 (1999), etc.).

### PROCESS 9:

The compound (IIIa) can be manufactured by reacting the compound (IIIb) obtained in PROCESS 7 or 8 with the compound shown by R⁵Z (VII) [wherein R⁵ is the reactive substituent described above] in an inert solvent in the presence of an appropriate base. 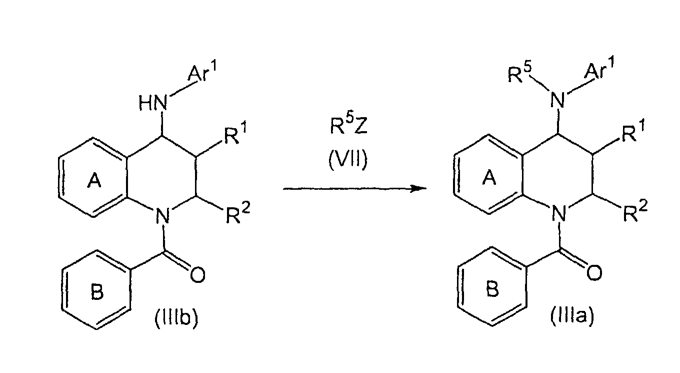 [wherein the symbols have the same significance as defined above].

For the reactive substituent, those described in PROCESS 7 are used, preferably a halogen atom, an alkylsulfonyl group, an arylsulfonyl group, etc.

For the "base," those given above are employed; preferred are potassium carbonate, sodium carbonate, sodium hydroxide, potassium hydroxide, sodium hydride, potassium hydride, etc.

The amount of the base used is preferably 1 to 10 equivalents.

As the compound (VII), there are used commercially available alkyl chlorides, alkyl bromides or alkyl iodides, or those synthesized by the methods described in SHIN-JIKKEN KAGAKU KOZA, 14 (I), ORGANIC FUNCTIONAL GROUP PREPARATIONS, 2nd Ed., Academic Press Inc., 1989; Comprehensive Organic Transformations, VCH Publishers Inc., 1989, etc.

The amount used is preferably from 1 to 20 equivalents.

The "inert solvent" includes, for example, an alcoholic solvent, an ethereal solvent, a halogenated hydrocarbon solvent, an aromatic solvent, a nitrile solvent, an amide solvent, a ketone solvent, a sulfoxide solvent, etc. These solvents may be used by mixing two or more in a suitable ratio. Among these solvents, preferred are acetonitrile, DMF, DMSO, acetone, etc.

The reaction temperature is generally from approximately -20°C to 100°C, preferably from room temperature to 80°C. The reaction time is, for example, approximately from 0.5 to 48 hours.

### PROCESS 10:

The compound (IIId) wherein X is CR³R⁴ and Y is N can be manufactured from the compound (X') and the compound (XI) [wherein Z is a reactive substituent] in a manner similar to PROCESS 9. 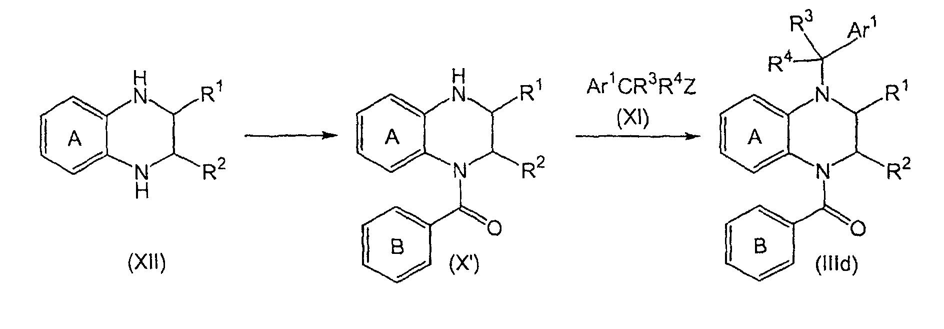 [wherein the symbols have the same significance as defined above].

As the compound (XI), there are used commercially available alkyl chlorides, alkyl bromides or alkyl iodides, or those synthesized by the methods described in SHIN-JIKKEN KAGAKU KOZA, 14 (1), ORGANIC FUNCTIONAL GROUP PREPARATIONS, 2nd Ed., Academic Press Inc., 1989; Comprehensive Organic Transformations, VCH Publishers Inc., 1989, etc.

The compound (X') can be manufactured by acylating the compound (XII) as in PROCESS 1. The compound (XII) can be manufactured by publicly known methods (e.g., the method described in Journal of Pharmaceutical Bulletin, 32 (6), 2421 (1984), etc.).

The compound of the present invention described above (hereinafter referred to also as the compound of the present invention), its salt, or a prodrug thereof possesses an excellent soluble beta amyloid precursor protein secretion promoting activity, and is low toxic. Thus, the compound of the present invention can be used as a safe soluble beta amyloid precursor protein secretion promoter for human and mammal (e.g., mouse, rate, guinea pig, rabbit, dog, cat, bovine, swine, sheep, monkey, chimpanzee, etc.), and can be used as an apoptosis inhibitor or an agent for improving neuronal dysfunction.

Therefore, the compound of the present invention, its salt or a prodrug thereof, can be safely used to human and mammal, as the agent for the prevention/treatment of neurodegenerative diseases such as Alzheimer's disease, Parkinson's disease, prion disease, neuropathy (preferably, diabetic neuropathy, etc.), etc., cerebrovascular dementia, or neuronal injuries associated with cerebrovascular disorders.

The compound of the present invention, its salts or prodrugs thereof are used as pharmaceuticals, whereby they are used as they are or prepared into a pharmaceutical composition. The pharmaceutical composition can be prepared by optionally admixing with suitable pharmaceutically acceptable carriers, excipients, diluents, etc., and can be safely administered either orally or parenterally in the form of oral dosage preparations (e.g., powders, granules, tablets, capsules (including soft capsules and microcapsules), syrup, etc.), parenteral dosage preparations (e.g., injections, topical agents (nasal spray preparations, transdermal preparations, etc.), suppositories (e.g., rectal suppositories, vaginal suppositories, etc.), pellets, etc.).

These dosage forms can be prepared by publicly known methods, for example, by those conventionally used to manufacture pharmaceutical preparations. The ratio of the compound of the present invention formulated in the preparations may vary depending upon its dosage form; for example, in the oral dosage form described above, approximately 10 to 95 wt% is preferred, and approximately 0.001 to 95 wt% is preferred for the parenteral dosage form described above.

In the case of, e.g., injections, the compound of the present invention can be prepared into injections either in the form of an aqueous injection together with solubilizing agents (e.g., β-cyclodextrins, etc.), dispersing agents (e.g., Tween 80 (manufactured by Atlas Powder, Inc., U.S.A.), HCO 60 (manufactured by Nikko Chemicals Co., Ltd.), carboxymethylcellulose, sodium alginate, etc.), preservatives (e.g., methyl paraben, propyl paraben, benzyl alcohol, chlorobutanol, etc.), isotonizing agents (e.g., sodium chloride, glycerin, sorbitol, glucose, etc.) and the like or in the form of an oily injection by appropriately dissolving, suspending or emulsifying in vegetable oil (e.g., olive oil, sesame oil, peanut oil, cotton seed oil, corn oil, etc.), propylene glycol, and the like.

The pharmaceutical composition for oral administration can be prepared by appropriately adding to the compound of the present invention, for example, excipients (e.g., lactose, sucrose, starch, etc.), disintegrating agents (e.g., starch, calcium carbonate, etc.), binders (e.g., starch, gum arabic, carboxymethylcellulose, polyvinylpyrrolidone, hydroxypropylcellulose, etc.) or lubricants (e.g., talc, magnesium stearate, polyethylene glycol 6000, etc.), and the like, which is compression-molded; if necessary, the composition is then coated by publicly known methods for the purposes of taste masking, an enteric coating or sustained release. Examples of coating agents are hydroxypropylmethylcellulose, ethylcellulose, hydroxymethylcellulose, hydroxypropylcellulose, polyoxyethylene glycol, Tween 80, pluronic F68, cellulose acetate phthalate, hydroxypropylmethylcellulose phthalate, hydroxymethylcellulose acetate succinate, Eudragit (methacrylic acid-acrylic acid copolymer; manufactured by Rohm, A.G., Germany), pigments (e.g., titanium oxide, colcothar, etc.) and the like.

The compound of the present invention may also be used in the form of a solid, semi-solid or liquid topical agent.

For solid topical agents, the compound of the present invention can be used as it is; or, the compound of the present invention can be prepared into a powdery composition by adding/mixing excipients (e.g., glycol, mannitol, starch, microcrystalline cellulose, etc.), thickeners (e.g., natural gums, cellulose derivatives, acrylic acid polymers, etc.), and the like. Semi-solid topical agents are prepared in a conventional manner and used preferably as an aqueous or oily gel or as an ointment. Liquid topical agents may also be manufactured in the form of an oily or aqueous suspension by the procedures used to prepare injections or by modifications thereof.

Also, these solid, semi-solid or liquid topical preparations may be suitably formulated with a pH adjusting agent (e.g., carbonic acid, phosphoric acid, citric acid, hydrochloric acid, sodium hydroxide, etc.), an antiseptic agent (e.g., p-oxybenzoates, chlorobutanol, benzalkonium chloride, etc.), and the like. Specifically, the compound of the present invention can be used as an ointment containing generally about 0.1 to about 100 mg per gram, using, e.g., Vaseline, lanoline, etc. as an ointment base.

The compound of the present invention may be prepared into an oily or aqueous solid, semi-solid or liquid suppository. For oily base materials in the manufacture of an ointment, there are appropriately used, for example, glycerides of higher fatty acids (e.g., cacao butter, Witepsols (manufactured by Dynamite-Nobel, Inc.), etc.), medium fatty acids (e.g., Miglyols (manufactured by Dynamite-Nobel, Inc.), etc., or vegetable oil (e.g., sesame oil, soybean oil, cotton seed oil, etc.), and the like. Polyethylene glycol, propylene glycol, etc. are suitably used for the aqueous base material and natural gums, cellulose derivatives, vinyl polymers, acrylic acid polymers, etc. for the aqueous gel base material.

The dose of the compound of the present invention or its salt varies depending on target disease, human or mammal to be administered, symptom, age, body weight, symptom, dosage form, route for administration, dosing period, etc.; for example, in oral administration for the treatment of Alzheimer's disease, the dose of the compound of the present invention or its salt is normally about 0.1 to 100 mg, preferably about 1.0 to 50 mg, more preferably about 1 to about 50 mg, and most preferably about 2.5 to 50 mg, per day for human or mammal weighing 60 kg body weight, which is administered by dividing the dose in 1 to 3 portions. As described above, of course, the dose may vary depending upon various conditions, and a dose less than the dose described above may be sufficient, or a dose exceeding the dose given may be administered as well.

### EXAMPLES

The present invention is described in detail below with reference to EXAMPLES and EXPERIMENTAL EXAMPLES, but these EXAMPLES and EXPERIMENTAL EXAMPLES are not deemed to limit the scope of the present invention thereto.

### EXAMPLE 1

### cis-(4-Anilino-2-methyl-3,4-dihydro-(2H)-quinolinyl)(2-thienyl)methanone

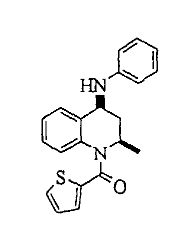

Under ice cooling, an acid chloride (0.42 g) was dropwise added to a THF (20 ml) solution of cis-N-(2-methyl-1,2,3,4-tetrahydro-4-quinolinyl)-N-phenylamine (0.66 g), and triethylamine (0.5 ml) was further added to the mixture. After stirring overnight at room temperature, water was added to the reaction solution followed by extraction with ethyl acetate. The organic phase was washed with water, dried and then concentrated. The residue was recrystallized from IPE to give the title compound (0.7 g).

The compounds of EXAMPLES 1-2 to 1-5 were synthesized by the similar procedures.

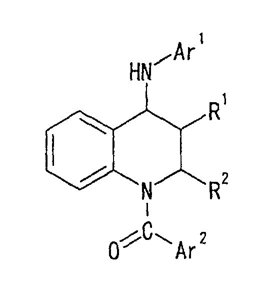

| EX. No. | Ar¹ | Ar² | R¹ | R² | Stereochemistry | Melting point (°C) | Recrystallization solvent |
|---|---|---|---|---|---|---|---|
| 1-1 | Ph | 2-thienyl | H | Me | cis | 172-173 | IPE |
| 1-2 | Ph | 2-furyl | H | Me | cis | 128-129 | IPE |
| 1-3 | Ph | 3,4-dimethoxyphenyl | H | Me | cis | 110-111 | IPE |
| 1-4 | Ph | 2-benzothienyl | H | Me | cis | 122-123 | IPE |
| 1-5 | Ph | 3,5-dimethoxyphenyl | H | Me | cis | 202-203 | Hex/EtOAc |

IPE, Hex and EtOAc denote isopropyl ether, hexane and ethyl acetate, respectively, and the stereochemistry denotes stereochemistry between NHAr¹ and R².

### EXAMPLE 2

### (4-Anilino-2-methyl-3,4-dihydro-1(2H)-quinolinyl)(4-chlorophenyl)methanone

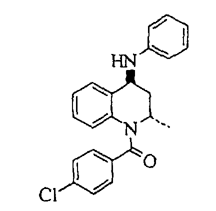

Under ice cooling, p-chlorobenzoyl chloride (0.35 g) was added to a pyridine (15 ml) solution oftrans-N-(2-methyl-1,2,3,4-tetrahydro-4-quinolinyl)-N-phenylamine (0.46 g). After stirring overnight at room temperature, ethyl acetate was added to the reaction solution followed by washing with water. After drying, the reaction mixture was concentrated. The residue was recrystallized from IPE/ethyl acetate to give the title compound (0.4 g).

The compounds of EXAMPLES 2-2 to 2-9 were synthesized by the similar procedures.

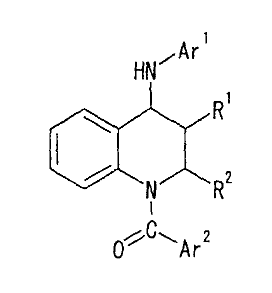

| EX. No. | Ar¹ | Ar² | R¹ | R² | Stereochemistry | Melting point (°C) | Recrystallization solvent |
|---|---|---|---|---|---|---|---|
| 2-1 | Ph | 4-chlorophenyl | H | Me | trans | 173-175 | IPE/EtOAc |
| 2-2 | Ph | 2-furyl | H | Me | trans | 122-123 | IPE |
| 2-3 | Ph | 2-thienyl | H | Me | trans | 138-139 | Hex/EtOAc |
| 2-4 | Ph | 3,4-dimethoxyphenyl | H | Me | trans | 208-209 | Hex/THF |
| 2-5 | Ph | 3,5-dimethoxyphenyl | H | Me | trans | 166-167 | IPE/EtOAc |
| 2-6 | Ph | 4-bromophenyl | H | Me | trans | 170-171 | IPE/EtOAc |
| 2-7 | Ph | 3-methoxyphenyl | H | Me | trans | 156-157 | IPE/EtOAc |
| 2-8 | Ph | 1-methylindol-2-yl | H | Me | trans | 194-195 | Hex/EtOAc |
| 2-9 | Ph | isooxazol-5-yl | H | Me | trans | 82-83 | Hex/EtOAc |

IPE, Hex and EtOAc denote isopropyl ether, hexane and ethyl acetate, respectively, and the stereochemistry denotes stereochemistry between NHAr¹ and R².

### REFERENCE EXAMPLE 1

### cis-1-(3,4-Dimethoxybenzoyl)-2-methyl-1,2,3,4-tetrahydro-4-quinolinol

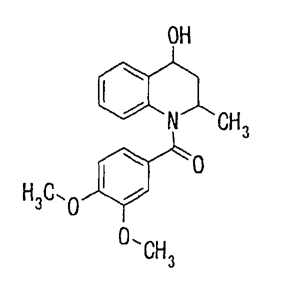

1) 1-(3,4-Dimethoxybenzoyl)-2-methyl-2,3-dihydro-4(1H)-quinolinone
After 1.19 g (7.38 mmols) of 2-methyl-2,3-dihydro-4(1H)-quinolone, which was synthesized by the method known by the literature (Bio-Organic & Medicinal Chemistry Letters, 9, 1009 (1999)), was dissolved in 15 ml of pyridine, 1.63 g (8.12 mmols) of 3,4-dimethoxybenzoyl chloride was added to the solution followed by stirring at room temperature for 16 hours. The reaction solution was concentrated, and ethyl acetate and water were added to the concentrate for extraction. The organic layer was dried and then concentrated. The residue was applied to silica gel column chromatography (developing solvent: ethyl acetate/hexane = 1/2). The eluate was recrystallized from ethyl acetate-hexane to give 1.57g (yield, 65%) of the product, cis-1-(3,4-dimethoxy-benzoyl)-2-methyl-2,3-dihydro-4 (1H)-quinolinone as white crystals.

| Elemental analysis as C₁₉H₁₉NO₄ | | | |
|---|---|---|---|
| Calcd. | C, 70.14; | H, 5.89; | N, 4.31 |
| Found | C, 70.18; | H, 5.86; | N, 4.46 |

2) cis-1-(3,4-Dimethoxybenzoyl)-2-methyl-1,2,3,4-tetrahydro-4-quinolinol
After 4.65g (14.3 mmols) of cis-1-(3,4-dimethoxybenzoyl)-2-methyl-2,3-dihydro-4(1H)-quinolinone obtained in 1) was dissolved in a solution mixture of 40 ml of methanol and 80 ml of THF, 540mg (14.3 mmols) of sodium borohydride was added to the mixture followed by stirring at room temperature for 2 hours. The reaction solution was poured onto water, which was extracted with ethyl acetate. After the organic layer was dried and concentrated, the residue was recrystallized from THF-hexane to give 3.84 g (yield, 82%) of the product, cis-1-(3,4-dimethoxybenzoyl)-2-methyl-1,2,3,4-tetra-hydro-4-quinolinol as white crystals.

| Elemental analysis as C₁₉H₂₁NO₄ | | | |
|---|---|---|---|
| Calcd. | C, 69.71; | H, 6.47; | N, 4.28 |
| Found | C, 69.88; | H, 6.51; | N, 4.18 |

### REFERENCE EXAMPLE 2

### 1-(3,4-Dimethoxybenzoyl)-1,2,3,4-tetrahydro-4-quinolinol

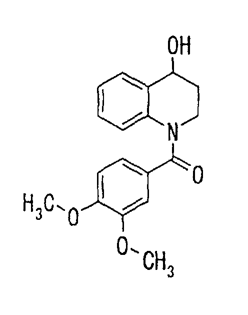

1) 1-(3,4-Dimethoxybenzoyl)-2,3-dihydro-4(1H)-quinolinone
   By the similar procedures as in REFERENCE EXAMPLE 1-1), 4.42 g (yield, 86%) of 1-(3,4-dimethoxybenzoyl)-2,3-dihydro-4(1H)-quinolinone was obtained as white crystals from 2.60 g (16.54 mmols) of 2,3-dihydro-4(1H)-quinolone.
   ¹H-NMR (CDCl₃) δ: 2.90 (2H, t, J=6.3 Hz), 3.80 (3H, s), 3.90 (3H, s), 4.35 (2H, t, J=6.3 Hz), 6.76 (1H, d, J=8.4 Hz), 6.95 (1H, d, J=7.5 Hz), 7.03-7.29 (4H, m), 8.01 (1H, dd, J=1.2 Hz, 7.8 Hz).
2) 1-(3,4-Dimethoxybenzoyl)-1,2,3,4-tetrahydro-4-quinolinol
   By the similar procedures as in REFERENCE EXAMPLE 1-2), 1.54 g (yield, 77%) of 1-(3,4-dimethoxybenzoyl)-1,2,3,4-tetrahydro-4-quinolinol was obtained as white crystals from 2.00g (14.3 mmols) of 1-(3,4-dimethoxybenzoyl)-2,3-dihydro-4(1H)-quinolinone obtained in 1).
   ¹H-NMR (CDCl₃) δ: 2.05-2.13 (1H, m), 2.21-2.27 (1H, m), 3.76 (3H, s), 3.76-3.84 (1H, m), 3.88 (3H, s), 4.09-4.20 (1H, m), 4.89-4.90 (1H, m), 6.74 (1H, d, J=7.8 Hz), 6.88 (1H, d, J=7.8 Hz), 6.96-7.12 (4H, m), 7.45 (1H, d, J= 6.0 Hz).

### REFERENCE EXAMPLE 3

### 1-(2-Furoyl)-1,2,3,4-tetrahydro-4-quinolinol

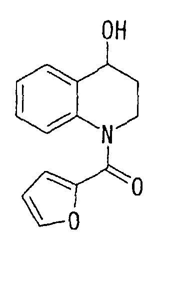

1) 1-(2-Furoyl)-2,3-dihydro-4(1H)-quinolinone
   By the similar procedures as in REFERENCE EXAMPLE 1-1), 2.90 g (yield, 95%) of 1-(2-furoyl)-2,3-dihydro 4(1H)-quinolinone was obtained as white crystals from 2.00 g (12.7 mmols) of 2,3-dihydro-4(1H)-quinolone and 3.32 g (0.025 mmol) of 2-furoylchloride.
   ¹H-NMR (CDCl₃) δ: 2.88 (2H, t, J=6.0 Hz), 4.37 (2H, t, J=6.0 Hz), 6.48-6.50 (1H, m), 7.02-7.05 (2H, m), 7.20-7.26 (1H, m), 7.36-7.42 (2H, m), 8.04 (1H, dd, J=1.8 Hz, 7.8 Hz) 2) 1-(2-Furoyl)-1,2,3,4-tetrahydro-4-quinolinol

By the similar procedures as in REFERENCE EXAMPLE 1-2), 0.90 g (yield, 89%) of 1-(2-furoyl)-1,2,3,4-tetrahydro-4-quinolinol was obtained as colorless oil from 1.00 g (4.14 mmols) of 1-(2-furoyl)- 2,3-dihydro-4(1H)-quinolinone obtained in 1).
¹H-NMR (CDCl₃) δ: 2.04-2.11 (1H, m), 2.22-2.27 (1H, m), 3.76-3.85 (1H, m), 4.17-4.26 (1H, m), 4.86-4.88 (1H, m), 6.42-6.44 (1H, m), 6.84 (1H, d, J=3.6 Hz), 7.04-7.17 (3H, m), 7.37 (1H, m), 7.46-7.49 (1H, m).

### REFERENCE EXAMPLE 4

### 1-(3,4-Dimethoxybenzoyl)-1,2,3,4-tetrahydroquinoxaline

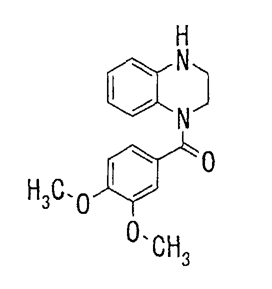

After 660 m g (4.92 mmols) of tetrahydroquinoxaline synthesized by the method known in the literature (Journal of Pharmaceutical Bulletin, 32(6), 2421 (1984)) was dissolved in pyridine (20 ml), 987 mg (4.92 mmols) of 3,4-dimethoxybenzoyl chloride was added to the solution. The mixture was stirred at room temperature for 16 hours. After the reaction solution was concentrated, ethyl acetate and water were added to the concentrate followed by extraction. The organic layer was dried and concentrated and the residue was applied to alumina column chromatography (developing solvent: ethyl acetate/hexane = 1/1). The eluate was recrystallized from ethyl acetate-hexane to give 780 mg (yield, 53%) of the product, 1-(3,4-dimethoxybenzoyl)-1,2,3,4-tetrahydro-quinoxaline as white crystals.
¹H-NMR (CDCl₃) δ: 3.55-3.60 (2H, m), 3.76 (3H, s), 3.88 (3H, s), 3.97-4.01 (2H, m), 4.06 (1H, brs), 6.36-6.41 (1H, m), 6.58-6.61 (2H, m), 6.76 (1H, d, J=8.4 Hz), 6.84-6.90 (1H, m), 7.02-7.07 (2H, m).

### REFERENCE EXAMPLE 5

### 6-Chloro-1-(3,4-dimethoxybenzoyl)-1,2,3,4-tetrahydro-4-quinolinol

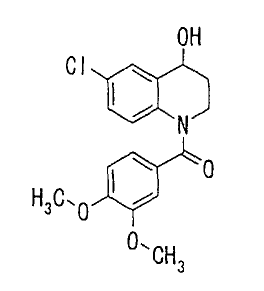

1) 6-Chloro-1-(3,4-dimethoxybenzoyl)-2,3-dihydro-4(1H)-quinolinone
   By the similar procedures as in REFERENCE EXAMPLE 1-1), 5.80 g (yield, 100%) of 6-chloro-1-(3,4-dimethoxybenzoyl)-2,3-dihydro-4(1H)-quino(inone was obtained as light yellow crystals from 3.00 (16.5 mmols) of 6-chloro-2,3-dihydro-4(1H)-quinolone.
   ¹H-NMR (CDCl₃) δ: 2.89 (2H, t, J=6.6 Hz), 3.85 (3H, s), 3.91 (3H, s), 4.33 (2H, t, J=6.6 Hz), 6.78 (1H, d, J=8.4 Hz), 6.87 (1H, d, J=8.7 Hz), 7.01 (1H, dd, J=8.4,2.1 Hz), 7.11 (1H, d, J=1.8 Hz), 7.23 (1H, dd, J=8.7,2.1 Hz), 7.97 (1H, d, J=1.8 Hz).
2) 6-Chloro-1-(3,4-dimethoxybenzoyl)-1,2,3,4-tetrahydro-4-quinolinol
   By the similar procedures as in REFERENCE EXAMPLE 1 -2), 4.50 g (yield, 89%) of 6-chloro-1-(3,4-dimethoxybenzoyl)-1,2,3,4-tetrahydro-4-quinolinol was obtained as light yellow crystals from 5.00 g (14.5 mmols) of 6-chloro-1-(3,4-dimethoxy-benzoyl)-2,3-dihydro-4(1H)-quinolinone obtained in 1).
   ¹H-NMR (CDCl₃) δ: 2.01-2.09 (1H, m), 2.23-2.30 (1H, m), 3.73-3.81 (2H, m), 3.81 (3H, s), 3.89 (3H, s), 4.08-4.16 (1H, m), 4.82-4.87 (1H, m), 6.75 (1H, d, J=8.4 Hz), 6.85 (1H, d, J=9.0 Hz), 6.92-7.03 (3H, m), 7.45 (1H, d, J=2.7 Hz)

### REFERENCE EXAMPLE 6

### 7-Chloro-1-(3,4-dimethoxybenzoyl)-1,2,3,4-tetrahydro-4-quinolinol

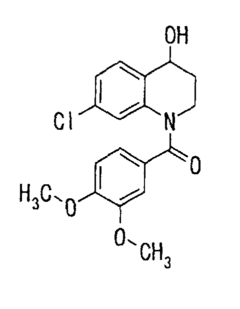

1) 7-Chloro-1-(3,4-dimethoxybenzoyl)-2,3-dihydro-4(1H)-quinolinone
   By the similar procedures as in REFERENCE EXAMPLE 1 -1), 3.50 g (yield, 92%) of 7-chloro-1-(3,4-dimethoxybenzoyl)-2,3-dihydro-4(1H)-quinolinone was obtained as light yellow crystals from 2.00 g (11.0 mmols) of 7-chloro-2,3-dihydro-4(1H)-quinolone.
   ¹H-NMR (CDCl₃) δ: 2.86 (2H, t, J=6.3 Hz), 3.86 (3H, s), 3.92 (3H, s), 4.31 (2H, t, J=6.3 Hz), 6.82 (1H, d, J=8.1 Hz), 7.03-7.14 (4H, m), 7.95 (1H, d, J=8.4 Hz)
2) 7-Chloro-1-(3,4-dimethoxybenzoyl)-1,2,3,4-tetrahydro-4-quinolinol
   By the similar procedures as in REFERENCE EXAMPLE 1 -2), 2.20 g (yield, 73%) of 7-chloro-1-(3,4-dimethoxybenzoyl)-1,2,3,4-tetrahydro-4-quinolinol was obtained as light yellow crystals from 3.00 g (8.70 mmols) of 7-chloro-1-(3,4-dimethoxy-benzoyl)-2,3-dihydro-4(1H)-quinolinone obtained in 1).
   ¹H-NMR (CDCl₃) δ: 2.01-2.09 (1H, m), 2.14-2.23 (1H, m), 3.72-3.83 (2H, m), 3.83 (3H, s), 3.91 (3H, s), 4.06-4.14 (1H, m), 4.82-4.87 (1H, m), 6.79 (1H, d, J=8.4 Hz), 6.99-7.09 (4H, m), 7.38 (1H, d, J=7.8 Hz).

### REFERENCE EXAMPLE 7

### 1-(5-Bromo-2-furoyl)-1,2,3,4-tetrahydro-4-quinolinol

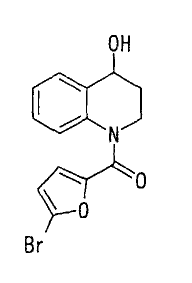

1) 1-(5-Bromo-2-furoyl)-2,3-dihydro-4(1H)-quinolinone
   By the similar procedures as in REFERENCE EXAMPLE 1 -1), 1.00 g (yield, 46%) of 1-(5-bromo-2-furoyl)-2,3-dihydro 4(1H)-quinolinone was obtained as white crystals from 1.00 g (6.8 mmols) of 2,3-dihydro-4(1H)-quinolone and 1.42 g (6.8 mmols) of 5-bromo-2-furoyl chloride.
   ¹H-NMR (CDCl₃) δ: 2.88 (2H, t, J=6.3 Hz), 4.36 (2H, t, J=6.3 Hz), 6.43 (1H, d, J=3.3 Hz), 6.96 (1H, d, J=3.3 Hz), 7.10 (1H, d, J=8.1 Hz), 7.23-7.28 (2H, m), 7.39-7.45 (1H, m), 8.03 (1H, dd, J=8.1,1.5 Hz)
2) 1-(5-Bromo-2-furoyl)-1,2,3,4-tetrahydro-4-quinolinol
   By the similar procedures as in REFERENCE EXAMPLE 1 -2), 0.81 g (yield, 90%) of 1-(5-bromo-2-furoyl)-1,2,3,4-tetrahydro-4-quinolinol was obtained as colorless oil from 900 m g (2.8 mmols) of 1-(5-bromo-2-furoyl)-2,3-dihydro-4(1H)-quinolinone obtained in 1).
   ¹H-NMR (CDCl₃) δ: 2.00-2.10 (1H, m), 2.21-2.26 (1H, m), 3.76-3.85 (1H, m), 4.16-4.23 (1H, m), 6.36 (1H, d, J=3.3 Hz), 6.76 (1H, d, J=3.3 Hz), 7.09-7.21 (3H, m), 7.23-7.28 (2H, m), 7.46-7.49 (1H, m)

### REFERENCE EXAMPLE 8

### cis-1-(3,4-Dimethoxybenzoyl)-2-ethyl-1,2,3,4-tetrahydro-4-quinolinol

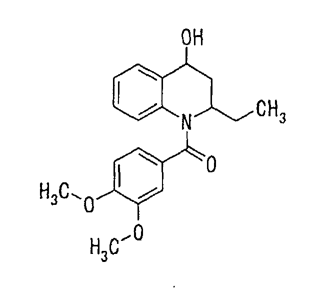

1) 1-(3,4-Dimethoxybenzoyl)-2-ethyl-2,3-dihydro-4(1H)-quinolinone
   By the similar procedures as in REFERENCE EXAMPLE 1 -1), 565 mg (yield, 73%) of 1-(3,4-dimethoxybenzoyl)-2-ethyl-2,3-dihydro-4(1H)-quinolinone was obtained as white crystals from 401 mg (2.29 mmols) of 2-ethyl-2,3dihydro-4(1H)-quinolinone.
   Melting point: 157-158°C (solvent for crystallization: ethyl acetate-hexane)
   FABMS (pos) 340.2 [M+H⁺]
2) cis-1-(3,4-Dimethoxybenzoyl)-2-ethyl-1,2,3,4-tetrahydro-4-quinolinol
   By the similar procedures as in REFERENCE EXAMPLE 1 -2), 447 mg (yield, 86%) of cis-1-(3,4-dimethoxybenzoyl)-2-ethyl-1,2,3,4-tetrahydro-4-quinolinol was obtained as white crystals from 520 mg (1.53 mmols) of 1-(3,4-dimethoxy-benzoyl)-2-ethyl-2,3-dihydro-4(1H)-quinolinone obtained in 1).
   Melting point: 168-169°C (solvent for crystallization: ethyl acetate-hexane)

### EXAMPLE 3

### cis-1-Isonicotinoyl-2-methyl-N-phenyl-1,2,3,4-tetrahydro-4-quinolinamine

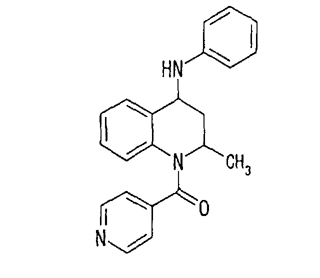

Under ice cooling 500 mg (2.10 mmols) of isonicotinic acid and DMF (0.1 ml) were dissolved in THF (10 ml), and 0.55 ml (6.3 mmols) of oxalyl chloride was dropwise added to the solution. After stirring at room temperature for 2 hours, the reaction mixture was concentrated and a pyridine solution (20 ml) of 500 mg (2.10 mmols) of cis-2-methyl-N-phenyl-1,2,3,4-tetrahydro-4-quinolinamine was added to the residue under ice cooling. After stirring at room temperature overnight, the reaction solution was diluted with ethyl acetate, washed with water, dried and then concentrated. The residue was applied to silica gel column chromatography (developing solvent: ethyl acetate/hexane = 1/1). The eluate was recrystallized from THF-hexane to give 178 mg (yield, 24%) of the product,
cis-1-isonicotinoyl-2-methyl-N-phenyl-1,2,3,4-tetrahydro-4-quinolinamine as white crystals.

| Elemental analysis as C₂₂H₂₁N₃O • 0.25H₂O | | | |
|---|---|---|---|
| Calcd. | C, 75.95; | H, 6.23; | N, 12.08 |
| Found | C, 76.12; | H, 6.15; | N, 12.23 |

Melting point: 210°C (solvent for crystallization: tetrahydrofuran-hexane)

### EXAMPLE 4

### cis-2-Methyl-N-phenyl-1-(2-pyridinylcarbonyl)-1,2,3,4-tetrahydro-4-quinolinamine

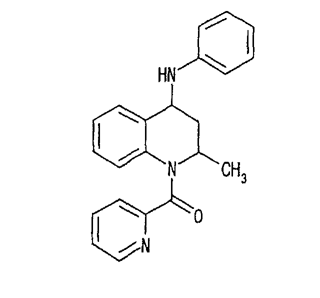

In a pyridine solution (20 ml), 500 mg (2.1 mmols) of cis-2-methyl-N-phenyl-1,2,3,4-tetrahydro-4-quinolinamine was dissolved, and 411 mg (2.31 mmols) of picolinoyl chloride hydrochloride was added to the solution under ice cooling. After stirring at room temperature overnight, the reaction mixture was diluted with ethyl acetate. The dilution was washed with water, dried and then concentrated. The residue was applied to silica gel column chromatography (developing solvent: ethyl acetate/hexane = 1/1). The eluate was recrystallized from ethyl acetate-hexane to give 134 mg (yield, 19%) of the product, cis-2-methyl-N-phenyl-1-(2-pyridinyl-carbonyl)-1,2,3,4-tetrahydro-4-quinolinamine as white crystals.
Melting point: 157°C (solvent for crystallization: ethyl acetate-hexane)
FABMS (pos) 343.1 [M+H⁺]

### EXAMPLE 5

### cis-1-(1,3-Benzoxol-5-ylcarbonyl)-2-methyl-N-phenyl-1,2,3,4-tetrahydro-4-quinolinamine

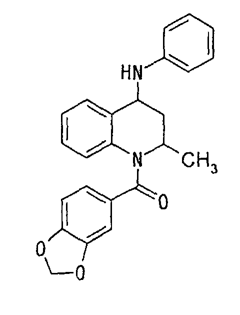

By the similar procedures as in EXAMPLE 4, 537 mg (yield, 66%) of cis-1-(1,3-benzodioxol-5-ylcarbonyl)-2-methyl-N-phenyl-1,2,3,4-tetrahydro-4-quinolinamine was obtained as white crystals from 500 mg (2.1 mmols) of the product, cis-2-methyl-N-phenyl-1,2,3,4-tetrahydro-4-quinolinamine and 453.5 mg (2.73 mmols) of 3,4-methylenedioxybenzoylchloride.
Melting point: 175°C (solvent for crystallization: ethyl acetate-hexane)

| Elemental analysis as C₂₄H₂₂N₂O₃ • 0.5H₂O | | | |
|---|---|---|---|
| Calcd. | C, 72.89; | H, 5.86; | N, 7.08 |
| Found | C, 73.07; | H, 6.01; | N, 7.04 |

### EXAMPLE 6

### cis-2-Methyl-N-phenyl-1-(3,4,5-trimethoybenzoyl)-1,2,3,4-tetrahydro-4-quinolinamine

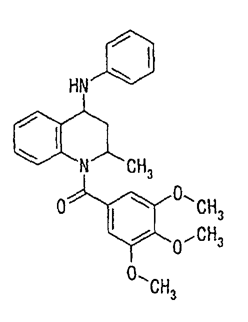

By the similar procedures as in EXAMPLE 3, 1.07 g (yield, 74%) of the product, cis-2-methyl-N-phenyl-1-(3,4,5-trimethoxybenzoyl)-1,2,3,4-tetrahydro-4-quinolinamine was obtained as white crystals from 800 mg (3.36 mmols) of cis-2-methyl-N-phenyl-1,2,3,4-tetrahydro-4-quinolinamine and 898 mg (4.23 mmols) of 3,4,5-trimethoxybenzoic acid.
Melting point: 300°C or hither (solvent for crystallization: ethyl acetate-hexane)
FABMS (pos) 433.2 [M+H⁺]

### EXAMPLE 7

### trans-1-(1,3-Benzodioxol-5-ylcarbonyl)-2-methyl-N-phenyl-1,2,3,4-tetrahydro-4-quinolinamine

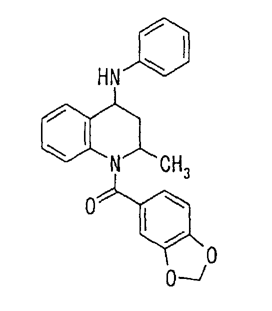

By the similar procedures as in EXAMPLE 4, 637 mg (yield, 49%) of the product, trans-1-(1,3-benzodioxol-5-ylcarbonyl)-2-methyl-N-phenyl-1,2,3,4-tetrahydro-4-quinolina mine was obtained as white crystals from 800 mg (3.36 mmols) of trans-2-methyl-N-phenyl-1,2,3,4-tetrahydro-4-quinolinamine and 726 mg (4.37 mmols) of 3,4-methylenedioxybenzoylchloride.
Melting point: 175°C (solvent for crystallization: ethyl acetate-hexane)

| Elemental analysis as C₂₄H₂₂N₂O₃ | | | |
|---|---|---|---|
| Calcd. | C, 74.59; | H, 5.74; | N, 7.25 |
| Found | C, 74.49; | H, 5.62; | N, 7.36 |

### EXAMPLE 8

### trans-2-Methyl-N-phenyl-1-(3,4,5-trimethoxybenzoyl)-1,2,3,4-tetrahydro-4-quinolinamine

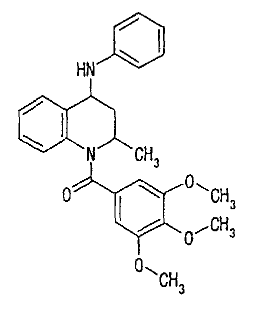

By the similar procedures as in EXAMPLE 3, 745 mg (yield, 51%) of the product, trans-2-methyl-N-phenyl-1-(3,4,5-trimethoxybenzoyl)-1,2,3,4-tetrahydro-4-quinolinamine was obtained as white crystals from 800 mg (3.36 mmols) of trans-2-methyl-N-phenyl-1,2,3,4-tetrahydro-4-quinolinamine and 898 mg (4.23 mmols) of 3,4,5-trimethoxybenzoic acid.
Melting point: 205°C (solvent for crystallization: ethyl acetate-hexane)

| Elemental analysis as C₂₆H₂₈N₂O₄ | | | |
|---|---|---|---|
| Calcd. | C, 72.20; | H, 6.53; | N, 6.48 |
| Found | C, 72.28; | H, 6.68; | N, 6.65 |

### EXAMPLE 9

### 1-(2-Furoyl)-N-phenyl-1,2,3,4-tetrahydro-4-quinolinamine

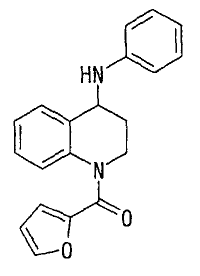

In chloroform (10 ml), 200 mg (0.82 mmols) of 1-(2-furoyl)-1,2,3,4-tetra-hydro-4-quinolinol obtained in REFERENCE EXAMPLE 3 was dissolved, and 576 mg (2.88 mmols) of iodotrimethylsilane was added to the solution under ice cooling. The mixture was then stirred for 3 hours. The reaction solution was concentrated, and THF (10 ml) was added to the residue. Then, 153 mg (1.64 mmols) of aniline and 324 mg (1.64 mmols) of barium carbonate were added. Stirring was continued for 48 hours at room temperature. After the insoluble matters were removed by filtration, the filtrate was concentrated, and the concentrate was applied to silica gel column chromatography (developing solvent: ethyl acetate/hexane = 1/3) to give 120 mg (yield, 46%) of the product, 1-(2-furoyl)-N-phenyl-1,2,3,4-tetrahydro-4-quinolinamine as colorless oil.
FABMS (pos) 318.1 [M⁺]

### EXAMPLE 10

### 1-(2-Furoyl)-4-phenoxy-1,2,3,4-tetrahydroquinolinone

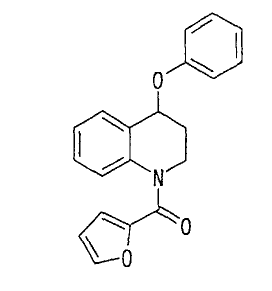

In THF (5 ml), 200 mg (0.82 mmols) of 1-(2-furoyl)-1,2,3,4-tetra-hydro-4-quinolinol obtained in REFERENCE EXAMPLE 3, 77 mg (0.82 mmols) of phenol and 216 mg (0.82 mmols) of triphenylphosphine were dissolved, and 143 mg (0.82 mmols) of diethyl azodicarboxylate was added to the solution followed by stirring at room temperature at room temperature for 16 hours. After the reaction solution was concentrated, the residue was applied to alumina column chromatography (developing solvent: ethyl acetate/hexane = 2/8) to give 72 mg (yield, 27%) of the product, 1-(2-furoyl)-4-phenoxy-1,2,3,4-tetrahydroquinolinone as colorless oil.
FABMS (pos) 320.1 [M+H⁺]

### EXAMPLE 11

### trans-1-Isonicotinoyl-2-methyl-N-phenyl-1,2,3,4-tetrahydro-4-quinolinamine

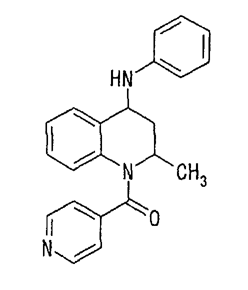

In THF (10 ml), 284 mg (2.31 mmols) of isonicotinic acid was dissolved, and 0.88 ml (10.1 mmols) of oxalyl chloride was dropwise added to the solution under ice cooling. After stirring at room temperature for an hour, the reaction mixture was concentrated and THF (15 ml) was added to dissolve the residue. Triethylamine (15 ml) and 500 mg (2.1 mmols) of trans-2-methyl-N-phenyl-1,2,3,4-tetrahydro-4-quinolinamine were added to the solution followed by stirring at room temperature overnight. The reaction solution was diluted with ethyl acetate, washed with water, dried and then concentrated. The resulting residue was applied to silica gel column chromatography (developing solvent; ethyl acetate/hexane = 1/1). The eluate was recrystallized from ethyl acetate-hexane to give 284 mg (yield, 25%) of the product,
cis-1-isonicotinoyl-2-methyl-N-phenyl-1,2,3,4-tetrahydro-4-quinolinamine as white crystals.
Melting point: 121°C (solvent for crystallization: ethyl acetate-hexane)
FABMS (pos) 344.1 [M+H⁺]

### EXAMPLE 12

### N-(4-Bromophenyl)-1-(3,4-dimethoxybenzoyl)-2-methyl-1,2,3,4-tetrahydro-4-quinolinamine

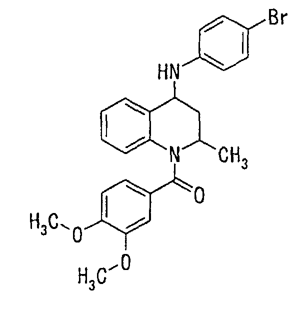

In chloroform (10 ml), 200 mg (0.61 mmols) of cis-1-(3,4-dimethoxybenzoyl)-2-methyl-1,2,3,4-tetrahydro-4-quinolinol obtained in REFERENCE EXAMPLE 1 was dissolved. Under ice cooling, 427 mg (2.14 mmols) of iodotrimethylsilane was added to the solution followed by stirring for 2 hours. After the reaction solution was concentrated, THF (10 ml) was added to dissolve the residue. Then, 315 mg (1.83 mmols) of p-bromoaniline and 241 mg (1.22 mmols) of barium carbonate were added to the solution. The mixture was stirred at room temperature for 24 hours. After the insoluble matters were removed by filtration, the filtrate was concentrated and the concentrate was applied to silica gel column chromatography (developing solvent: ethyl acetate/hexane = 1/3). The eluate was recrystallized from diethyl ether-hexane to give 80 mg (yield, 27%) of the product, N-(4-bromophenyl)-1-(3,4-dimethoxy-benzoyl)-2-methyl-1,2,3,4-tetrahydro-4-quinolinamine as white crystals (cis-trans = 1:6).
Melting point: 173-175°C (solvent for crystallization: diethyl ether-hexane)
FABMS (pos) 480.1 [M+H⁺]

### EXAMPLE 13

### trans-1-(3,4-Dimethoxybenzoyl)-2-methyl-N-(6-methyl-2-pyridinyl)-1,2,3,4-tetrahydro-4-quinolinamine

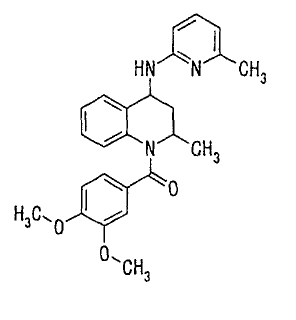

By the similar procedures as in EXAMPLE 12, 20 mg (yield, 8%) of the product, trans-1-(3,4-dimethoxybenzoyl)-2-methyl-N-(6-methyl-2-pyridinyl)-1,2,3,4-tetra-hydro-4-quinolinamine was obtained as white crystals from 200 mg (0.61 mmols) of cis-1-(3,4-dimethoxybenzoyl)-2-methyl-1,2,3,4-tetrahydro-4-quinolinol obtained in REFERENCE EXAMPLE 1 and 198 mg (1.83 mmols) of 2-amino-6-methylpyridine.
Melting point: 184-186°C (solvent for crystallization: diethyl ether-hexane)
FABMS (pos) 418.2 [M+H⁺]

### EXAMPLE 14

### N-(4-chlorophenyl)-1-(3,4-dimethoxybenzoyl)-1,2,3,4-tetrahydro-4-quinolinamine

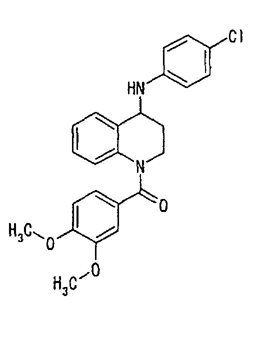

By the similar procedures as in EXAMPLE 12, 174 mg (yield, 43%) of the product, N-(4-chlorophenyl)-1-(3,4-dimethoxybenzoyl)-1,2,3,4-tetrahydro-4-quinolinamine was obtained as white crystals from 300 mg (0.96 mmols) of 1-(3,4-dimethoxybenzoyl)-1,2,3,4-tetrahydro-4-quinolinol obtained in REFERENCE EXAMPLE 2 and 367 mg (2.88 mmols) of 4-chloroaniline.
Melting point: 131°C (solvent for crystallization: ethyl acetate-hexane)
FABMS (pos) 445.1 [M+H⁺]

### EXAMPLE 15

### 1-(2-Furoyl)-N-methyl-N-phenyl-1,2,3,4-tetrahydro-4-quinolinamine

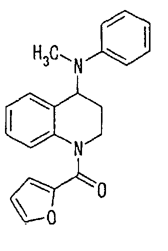

After 122 mg (0.50 mmols) of 1-(2-furoyl)-1,2,3,4-tetrahydro-4-quinolinol obtained in REFERENCE EXAMPLE 3 was dissolved in chloroform (10 ml), 350 mg (1.75 mmols) of iodotrimethylsilane was added to the solution under ice cooling. The mixture was stirred for 3 hours. The reaction solution was concentrated. THF (10 ml) was added to the residue, and 429 mg (2.00 mmols) of N-methylaniline and 197 mg (1.00 mmols) of barium carbonate were further added thereto followed by stirring at room temperature for 30 hours. After the insoluble matters were removed by filtration, the filtrate was concentrated and the concentrate was applied to silica gel column chromatography (developing solvent: ethyl acetate/hexane = 3/7) to give 110 mg (yield, 66%) of the product, 1-(2-furoyl)-N-methyl-N-phenyl-1,2,3,4-tetrahydro-4-quinolinamine as colorless oil.
FABMS (pos) 333.2 [M+H⁺]

### EXAMPLE 16

1-(2-Furoyl)-N-ethyl-N-phenyl-1,2,3,4-tetrahydro-4-quinolinamine

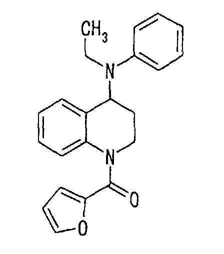

By the similar procedures as in EXAMPLE 15, 240 mg (yield, 56%) of the product, 1-(2-furoyl)-N-ethyl-N-phenyl-1,2,3,4-tetrahydro-4-quinolinamine was obtained as colorless oil from 300 mg (1.23 mmols) of 1-(2-furoyl)-1,2,3,4-tetrahydro-4-quinolinol obtained in REFERENCE EXAMPLE 3 and 447 mg (3.69 mmols) of N-ethylaniline.
FABMS (pos) 347.2 [M+H⁺]

### EXAMPLE 17

### 1-(3,4-Dimethoxybenzoyl)-N-phenyl-1,2,3,4-tetrahydro-4-quinolinamine

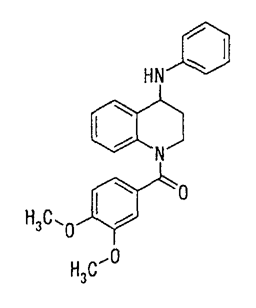

By the similar procedures as in EXAMPLE 12, 260 mg (yield, 70%) of 1-(3,4-dimethoxybenzoyl)-N-phenyl-1,2,3,4-tetrahydro-4-quinol inamine was obtained as white crystals from 300 mg (0.96 mmols) of 1-(3,4-dimethoxybenzoyl)-1,2,3,4-tetra-hydro-4-quinolinol obtained in REFERENCE EXAMPLE 2 and 267 mg (2.87 mmols) of aniline.
Melting point: 155-6°C (solvent for crystallization: diisopropyl ether-hexane)

| Elemental analysis as C₂₄H₂₄N₂O₃ | | | |
|---|---|---|---|
| Calcd. | C, 74.21; | H, 6.23; | N, 7.21 |
| Found | C, 73.71; | H, 6.21; | N, 7.21 |

### EXAMPLE 18

### 1-(3,4-Dimethoxybenzoyl)-N-methyl-N-phenyl-1,2,3,4-tetrahydro-4-quinolinamine

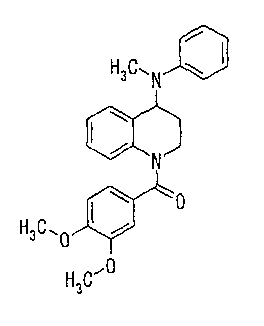

By the similar procedures as in EXAMPLE 12, 250 mg (yield, 65%) of the product, 1-(3,4-dimethoxybenzoyl)-N-methyl-N-phenyl-1,2,3,4-tetrahydro-4-quinolinamine was obtained as white crystals from 300 mg (0.96 mmols) of 1-(3,4-dimethoxy-benzoyl)-1,2,3,4-tetrahydro-4-quinolinol obtained in REFERENCE EXAMPLE 2 and 308 mg (2.87 mmols) ofN-methylaniline.
Melting point: 143-4°C (solvent for crystallization: ethyl acetate-diisopropyl ether)

| Elemental analysis as C₂₅H₂₆N₂O₃ | | | |
|---|---|---|---|
| Calcd. | C, 74.60; | H, 6.51; | N, 6.96 |
| Found | C, 74.32; | H, 6.52; | N, 6.80 |

The title compounds obtained in EXAMPLES 3 to 18 are summarized in TABLE 1, wherein Stereochemistry designates the stereochemistry between XAr¹ and R².

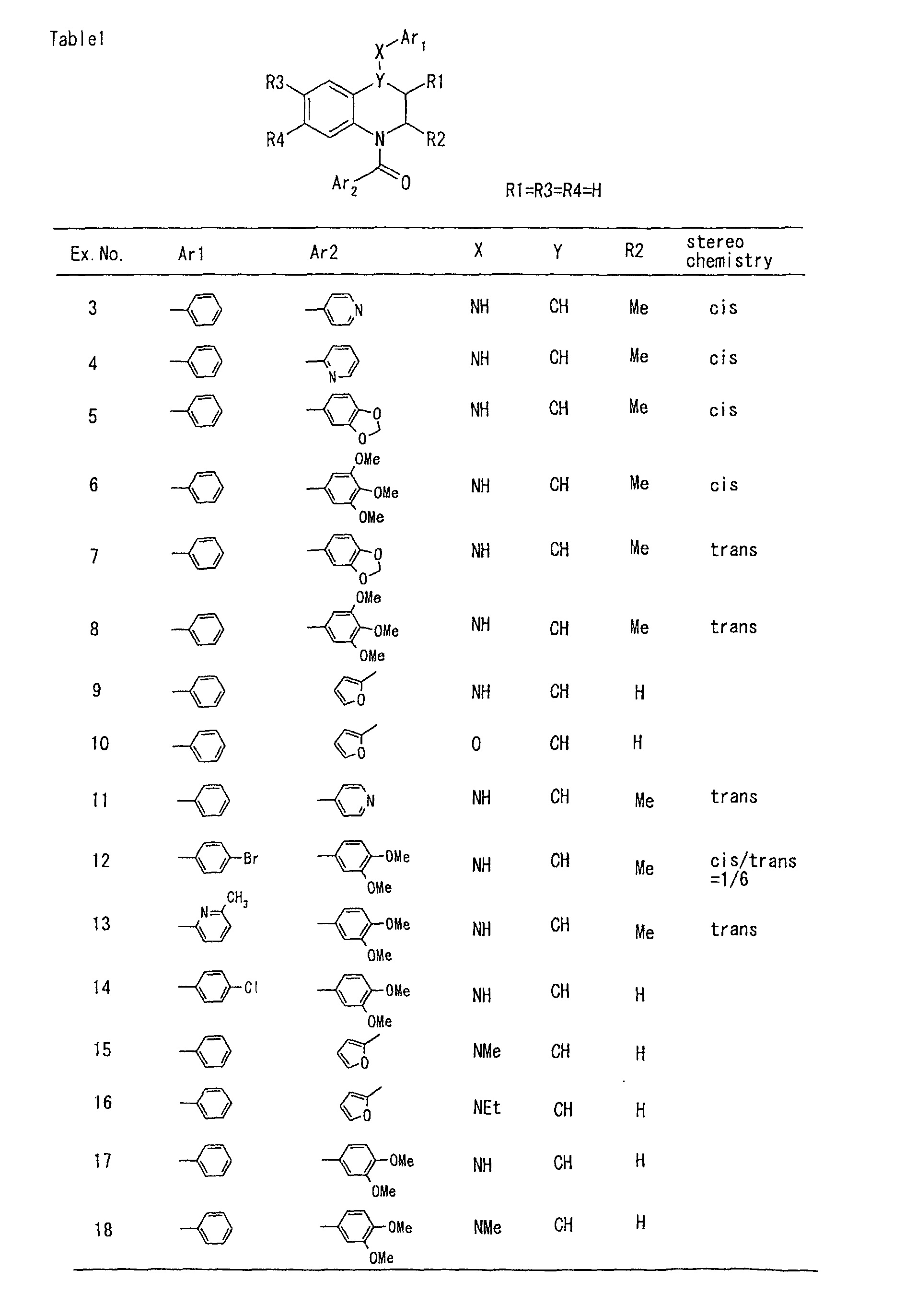

### EXAMPLE 19

### 4-(4-Bromophenoxy)-1-(3,4-dimethoxybenzoyl)-1,2,3,4-tetrahydro-4-quinolinamine

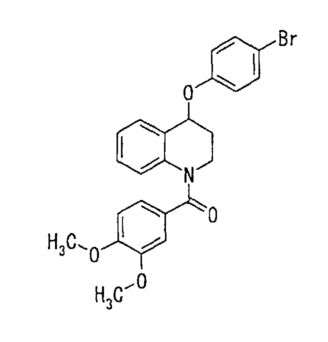

By the similar procedures as in EXAMPLE 10, 248 mg (yield, 66%) of the product, 4-(4-bromophenoxy)-1-(3,4-dimethoxybenzoyl)-1,2,3,4-tetrahydro-4-quinolinamine was obtained as white powders from 250 mg (0.80 mmols) of 1-(3,4-dimethoxybenzoyl)-1,2,3,4-tetrahydro-4-quinolinol obtained in REFERENCE EXAMPLE 2 and 138 mg (0.80 mmols) of 4-bromophenol.
Melting point: 56-7°C (solvent for crystallization: ethyl acetate-hexane)

| Elemental analysis as C₂₄H₂₂NO₄Br | | | |
|---|---|---|---|
| Calcd. | C, 61.55; | H, 4.73; | N, 2.99 |
| Found | C, 61.66; | H, 4.90; | N, 2.92 |

### EXAMPLE 20

### 1-Benzyl-4-(3,4-dimethoxybenzoyl)-1,2,3,4-tetrahydroquinoxaline

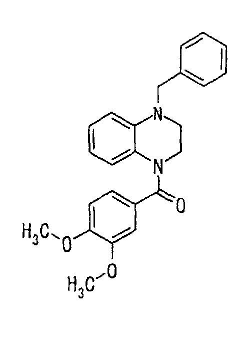

After 200 mg (0.67 mmols) of 1-(3,4-dimethoxybenzoyl)-1,2,3,4-tetrahydroquinoxaline synthesized in REFERENCE EXAMPLE 4 was dissolved in DMF (10 ml), 54 mg (1.34 mmols) of 60% sodium hydride was added to the solution followed by stirring at 60°C for 20 minutes. Then 344 mg (2.01 mmols) of benzyl bromide was added to the mixture. After stirring for 2 hours, ethyl acetate and water were added to the mixture for extraction. The organic layer was dried and concentrated. The residue was applied to alumina column chromatography(developing solvent: ethyl acetate/hexane = 3/7). The eluate was recrystallized from ethyl acetate-diisopropyl ether to give 182 mg (yield, 70%) of the product, 1-benzyl-4-(3,4-dimethoxybenzoyl)-1,2,3,4-tetrahydroquinoxaline as white crystals.
Melting point: 111-2°C (solvent for crystallization: ethyl acetate-diisopropyl ether)

| Elemental analysis as C₂₄H₂₄N₂O₃ | | | |
|---|---|---|---|
| Calcd. | C, 74.21; | H, 6.23; | N, 7.21 |
| Found | C, 73.97; | H, 6.18; | N, 7.15 |

### EXAMPLE 21

### 1-(3,4-Dimethoxybenzoyl)-N-(6-quinolinyl)-1,2,3,4-tetrahydro-4-quinolinamine

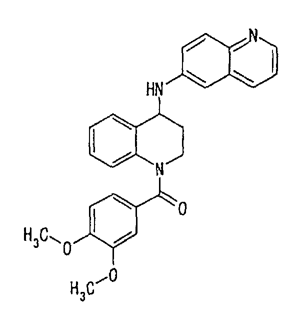

By the similar procedures as in EXAMPLE 12, 122 mg (yield, 22%) of 1-(3,4-dimethoxybenzoyl)-N-(6-quinolinyl)-1,2,3,4-tetrahydro-4-quinolinamine was obtained as white crystals 400 mg (1.28 mmols) of 1-(3,4-dimethoxybenzoyl)-1,2,3,4-tetrahydro-4-quinolinol obtained in REFERENCE EXAMPLE 2 and 554 mg (3.84 mmols) of 6-aminoquinolinone.
Melting point: 116-118°C (solvent for crystallization: THF-hexane)
FABMS (pos) 440.2 [M⁺]

### EXAMPLE 22

### 1-(3,4-Dimethoxybenzoyl)-N-(2-pyridinyl)-1,2,3,4-tetrahydro-4-quinol inamine

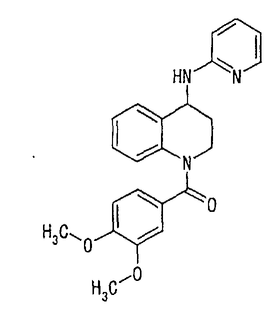

By the similar procedures as in EXAMPLE 12, 131 mg (yield, 26%) of the product, 1-(3,4-dimethoxybenzoyl)-N-(2-pyridinyl)-1,2,3,4-tetrahydro-4-quinolinamine was obtained as white crystals from 400 mg (1.28 mmols) of 1-(3,4-dimethoxybenzoyl)-1,2,3,4-tetrahydro-4-quinolinol obtained in REFERENCE EXAMPLE 2 and 361 mg (3.84 mmols) of 2-aminopyridine.
Melting point: 156-157°C (solvent for crystallization: diethyl ether-hexane)

| Elemental analysis as C₂₃H₂₃N₃O₃ | | | |
|---|---|---|---|
| Calcd. | C, 70.93; | H, 5.95; | N, 10.79 |
| Found | C, 70.64; | H, 6.00; | N, 10.79 |

### EXAMPLE 23

### N-(2-Chlorophenyl)-1-(3,4-dimethoxybenzoyl)-1,2,3,4-tetrahydro-4-quinolinamine

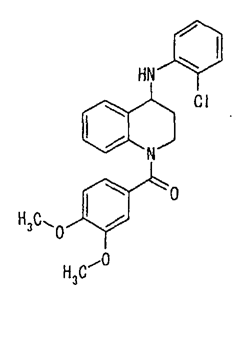

By the similar procedures as in EXAMPLE 12, 227 mg (yield, 56%) of the product, N-(2-chlorophenyl)- 1-(3,4-dimethoxybenzoyl)-1,2,3,4-tetrahydro-4-quinolinamine was obtained as white crystals from 300 mg (0.957 mmols) of 1-(3,4-dimethoxybenzoyl)-1,2,3,4-tetrahydro-4-quinolinol and 367 mg (2.87 mmols) of 2-chloroaniline.
Melting point: 92-93°C (solvent for crystallization: diethyl ether-hexane)

| Elemental analysis as C₂₄H₂₃N₂O₃Cl • 0.4H₂O | | | |
|---|---|---|---|
| Calcd. | C, 67.02; | H, 5.58; | N, 6.51 |
| Found | C, 67.32; | H, 5.86; | N, 6.22 |

### EXAMPLE 24

### 1-(3,4-Dimethoxybenzoyl)-N-(2-fluorophenyl)-1,2,3,4-tetrahydro-4-quinolinamine

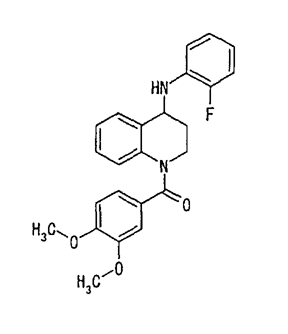

By the similar procedures as in EXAMPLE 12, 160 mg (yield, 41%) of the product, 1-(3,4-dimethoxybenzoyl)-N-(2-fluorophenyl)-1,2,3,4-tetrahydro-4-quinolinamine was obtained as white crystals from 300 mg (0.957 mmols) of 1-(3,4-dimethoxybenzoyl)-1,2,3,4-tetrahydro-4-quinolinol obtained in REFERENCE EXAMPLE 2 and 213 mg (1.92 mmols) of o-fluoroaniline.
Melting point: 133-134°C (solvent for crystallization: ethyl acetate-hexane)

| Elemental analysis as C₂₄H₂₃N₂O₃F | | | |
|---|---|---|---|
| Calcd. | C, 70.92; | H, 5.70; | N, 6.89 |
| Found | C, 70.80; | H, 5.71; | N, 6.61 |

### EXAMPLE 25

### 1-(3,4-Dimethoxybenzoyl)-N-methyl-N-(2-methylphenyl)-1,2,3,4-tetrahydro-4-quinolinamine

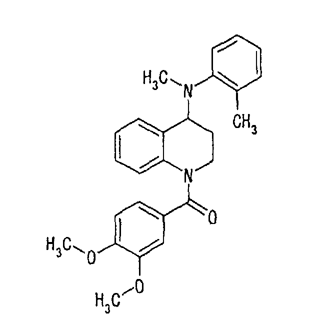

By the similar procedures as in EXAMPLE 12, 157 mg (yield, 39%) of the product, 1-(3,4-dimethoxybenzoyl)-N-methyl-N-(2-methylphenyl)-1,2,3,4-tetrahydro-4-quinolinamine was obtained as white crystals from 300 mg (0.957 mmols) of 1-(3,4-dimethoxybenzoyl)-1,2,3,4-tetrahydro-4-quinolinol obtained in REFERENCE EXAMPLE 2 and 233 mg (1.92 mmols) of N-methyl-2-toluidine.
Melting point: 170-171°C (solvent for crystallization: ethyl acetate-hexane)

| Elemental analysis as C₂₆H₂₈N₂O₃ | | | |
|---|---|---|---|
| Calcd. | C, 74.97; | H, 6.78; | N, 6.73 |
| Found | C, 75.10; | H, 6.81; | N, 6.58 |

### EXAMPLE 26

### N-(2,6-Dichlorophenyl)-1-(3,4-dimethoxybenzoyl)-1,2,3,4-tetrahydro-4-quinolinamine

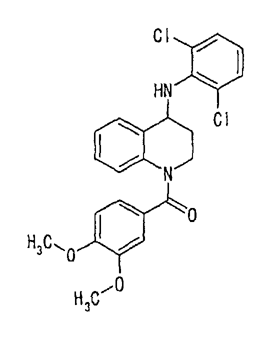

By the similar procedures as in EXAMPLE 12, 142 mg (yield, 32%) of the product, N-(2,6-dichlorophenyl)-1-(3,4-dimethoxybenzoyl)-1,2,3,4-tetrahydro-4-quinolinamine was obtained as white crystals from 300 mg (0.957 mmols) of 1-(3,4-dimethoxybenzoyl)-1,2,3,4-tetrahydro-4-quinolinol obtained in REFERENCE EXAMPLE 2 and 311 mg (1.92 mmols) of 2,6-dichloroaniline.
Melting point: 154-156°C (solvent for crystallization: ethyl acetate-hexane)

| Elemental analysis as C₂₄H₂₂N₂O₃Cl₂ | | | |
|---|---|---|---|
| Calcd. | C, 63.03; | H, 4.85; | N, 6.13 |
| Found | C, 62.81; | H, 4.84; | N, 5.91 |

### EXAMPLE 27

### N-(2-Chlorophenyl)-1-(3,4-dimethoxybenzoyl)-2-methyl-1,2,3,4-tetrahydro-4-quinolinamine

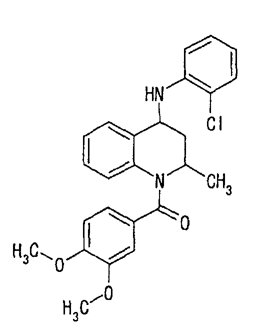

By the similar procedures as in EXAMPLE 12, 216 mg (yield, 41%) of the product, N-(2-chlorophenyl)-1-(3,4-dimethoxybenzoyl)-2-methyl-1,2,3,4-tetrahydro-4-quinolinamine was obtained as white crystals from 400 mg (0.61 mmols) of cis-1-(3,4-dimethoxybenzoyl)-2-methyl-1,2,3,4-tetrahydro-4-quinolinol obtained in REFERENCE EXAMPLE 1 and 467 mg (3.66 mmols) of o-chloroaniline (cis:trans = 1 : 1).
Melting point: 148-150°C (solvent for crystallization: diethyl ether-hexane)
FABMS (pos) 437.2 [M+H⁺]

### EXAMPLE 28

### cis-1-(3,4-Dimethoxybenzoyl)-N,2-dimethyl-N-phenyl-1,2,3,4-tetrahydro-4-quinolinamine

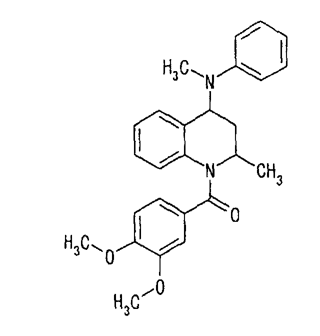

After 1.20 g (2.98 mmols) of cis-1-(3,4-dimethoxybenzoyl)-2-methyl-N-phenyl-1,2,3,4-tetrahydro-4-quinolinamine obtained in EXAMPLE 1-3 was dissolved in DMF (50 ml), 1.20 g (29.8 mmols) of 60% sodium hydride was added to the solution followed by stirring at room temperature for an hour. Methyl iodide (3.6 ml) was added to the reaction solution. After stirring at room temperature for 24 hours, ethyl acetate and water were added to the mixture for extraction. The organic layer was washed with water, dried and concentrated. The residue was recrystallized from ethyl acetate-hexane to give 849 mg (68%) of the product,
cis-1-(3,4-dimethoxybenzoyl)-2,N-dimethyl-N-phenyl-1,2,3,4-tetrahydro-4-quinolinamine was obtained as white crystals.
Melting point: 180-181°C (solvent for crystallization: ethyl acetate-hexane)
FABMS (pos) 416.2 [M⁺]

### EXAMPLE 29

### (+)-cis-1-(3,4-Dimethoxybenzoyl)-N,2-dimethyl-N-phenyl-1,2,3,4-tetrahydro-4-quinolinamine

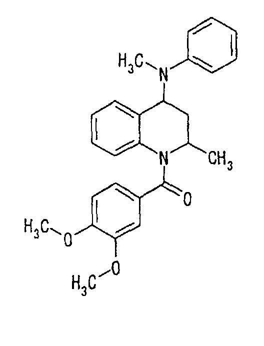

By optical resolution through fractionation on HPLC (CHIRALPAK AD, developing solvent: hexane/EtOH=1/1, UV 254nm detection), 548 mg of
cis-1-(3,4-dimethoxybenzoyl)-N,2-dimethyl-N-phenyl-1,2,3,4-tetrahydro-4-quinolinamine obtained in EXAMPLE 28 was optically resolved to give 234 mg of the product,
(+)-cis-1-(3,4-dimethoxybenzoyl)-N,2-dimethyl-N-phenyl-1,2,3,4-tetrahydro-4-quinolinamin as white powders.
[α]_{D}= +456.2° (0.5% EtOH)

### EXAMPLE 30

### (-)-cis-1-(3,4-Dimethoxybenzoyl)-N,2-dimethyl-N-phenyl-1,2,3,4-tetrahydro-4-quinolinamine

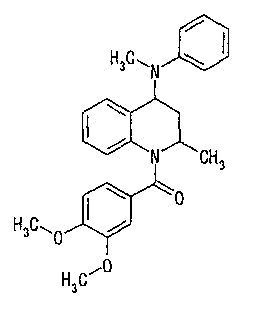

By optical resolution through fractionation on HPLC (CHIRALPAK AD, developing solvent: hexane/EtOH=1/1, UV 254nm detection), 548 mg of cis-1-(3,4-dimethoxybenzoyl)-N,2-dimethyl-N-phenyl-1,2,3,4-tetrahydro-4-quino linamine obtained in EXAMPLE 28 was optically resolved to give 201 mg of the product,
(-)-cis-1-(3,4-dimethoxybenzoyl)-N,2-dimethyl-N-phenyl-1,2,3,4-tetrahydro-4-quinolinamin as white powders.
[α]_{D} = -456.2° (0.5% EtOH)

### EXAMPLE 31

### N-(1,3-Benzodioxol-5-yl)-1-(3,4-dimethoxybenzoyl)-1,2,3,4-tetrahydro-4-quinolinamine

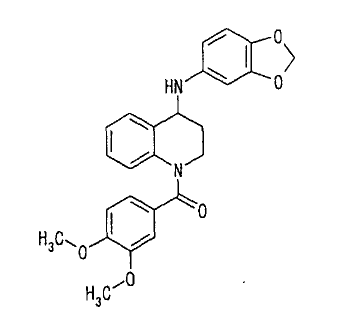

By the similar procedures as in EXAMPLE 12, 214 mg (yield, 49%) of the product, N-(1,3-benzodioxol-5-yl)-1-(3,4-dimethoxybenzoyl)-1,2,3,4-tetrahydro-4-quinolinamine was obtained as yellow crystals from 300 mg (0.957 mmols) of 1-(3,4-dimethoxybenzoyl)-1,2,3,4-tetrahydro-4-quinolinol obtained in REFERENCE EXAMPLE 2 and 395 mg (2.88 mmols) of 3,4-methylenedioxyaniline.
Melting point: 135-136°C (solvent for crystallization: ethyl acetate-hexane)

| Elemental analysis as C₂₅H₂₄N₂O₅ • 0.25H₂O | | | |
|---|---|---|---|
| Calcd. | C, 68.72; | H, 5.65; | N, 6.41 |
| Found | C, 68.51; | H, 5.52; | N, 6.21 |

### EXAMPLE 32

### N-(3-Chlorophenyl)-1-(3,4-dimethoxybenzoyl)-1,2,3,4-tetrahydro-4-quinolinamine

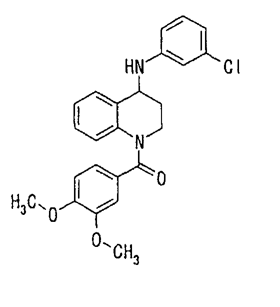

By the similar procedures as in EXAMPLE 12, 227 mg (yield, 56%) of the product, N-(3-chlorophenyl)-1-(3,4-dimethoxybenzoyl)-1,2,3,4-tetrahydro-4-quinolinamine was obtained as white crystals from 300 mg (0.957 mmols) of 1-(3,4-dimethoxybenzoyl)-1,2,3,4-tetrahydro-4-quinolinol obtained in REFERENCE EXAMPLE 2 and 367 mg (2.88 mmols) of 3-chloroanilin.
Melting point: 160°C (solvent for crystallization: diethyl ether-hexane)

| Elemental analysis as C₂₄H₂₃N₂O₃Cl | | | |
|---|---|---|---|
| Calcd. | C, 68.16; | H, 5.48; | N, 6.62 |
| Found | C, 67.95; | H, 5.46; | N, 6.53 |

### EXAMPLE 33

### 1-(3,4-Dimethoxybenzoyl)-N-(4-methylphenyl)-1,2,3,4-tetrahydro-4-quinolinamine

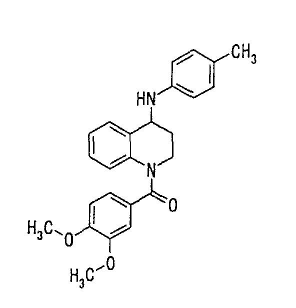

By the similar procedures as in EXAMPLE 12, 227 mg (yield, 56%) of the product, 1-(3,4-dimethoxybenzoyl)-N-(4-methylphenyl)-1,2,3,4-tetrahydro-4-quinolinamine was obtained as white crystals from 280 mg (0.89 mmols) of 1-(3,4-dimethoxybenzoyl)-1,2,3,4-tetrahydro-4-quinolinol obtained in REFERENCE EXAMPLE 2 and 286 mg (2.67 mmols) of 4-toluidine.
Melting point: 97-99°C (solvent for crystallization: diethyl ether-hexane)
FABMS (pos) 400.2 [M⁺]

### EXAMPLE 34

### N-(4-Bromophenyl)-1-(3,4-dimethoxybenzoyl)-1,2,3,4-tetrahydro-4-quinolinamine

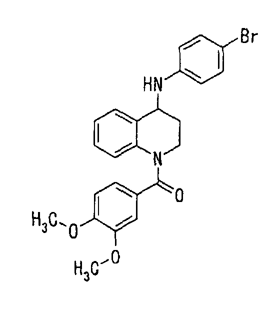

By the similar procedures as in EXAMPLE 12, 193 mg (yield, 22%) of the product, N-(4-bromophenyl)-1-(3,4-dimethoxybenzoyl)-1,2,3,4-tetrahydro-4-quinolinamine was obtained as white crystals from 600 mg (1.91 mmols) of 1-(3,4-dimethoxybenzoyl)-1,2,3,4-tetrahydro-4-quinolinol obtained in REFERENCE EXAMPLE 2 and 986 mg (5.73 mmols) of 4-bromoaniline.
Melting point: 130°C (solvent for crystallization: diethyl ether-hexane)

| Elemental analysis as C₂₄H₂₃N₂O₃Br • 0.4H₂O | | | |
|---|---|---|---|
| Calcd. | C, 60.74; | H, 5.05; | N, 5.90 |
| Found | C, 60.96; | H, 5.31; | N, 5.61 |

The title compounds obtained in EXAMPLE 19 to 34 are summarized in TABLE 2, wherein Stereochemistry designates the stereochemistry between XAr¹ and R².

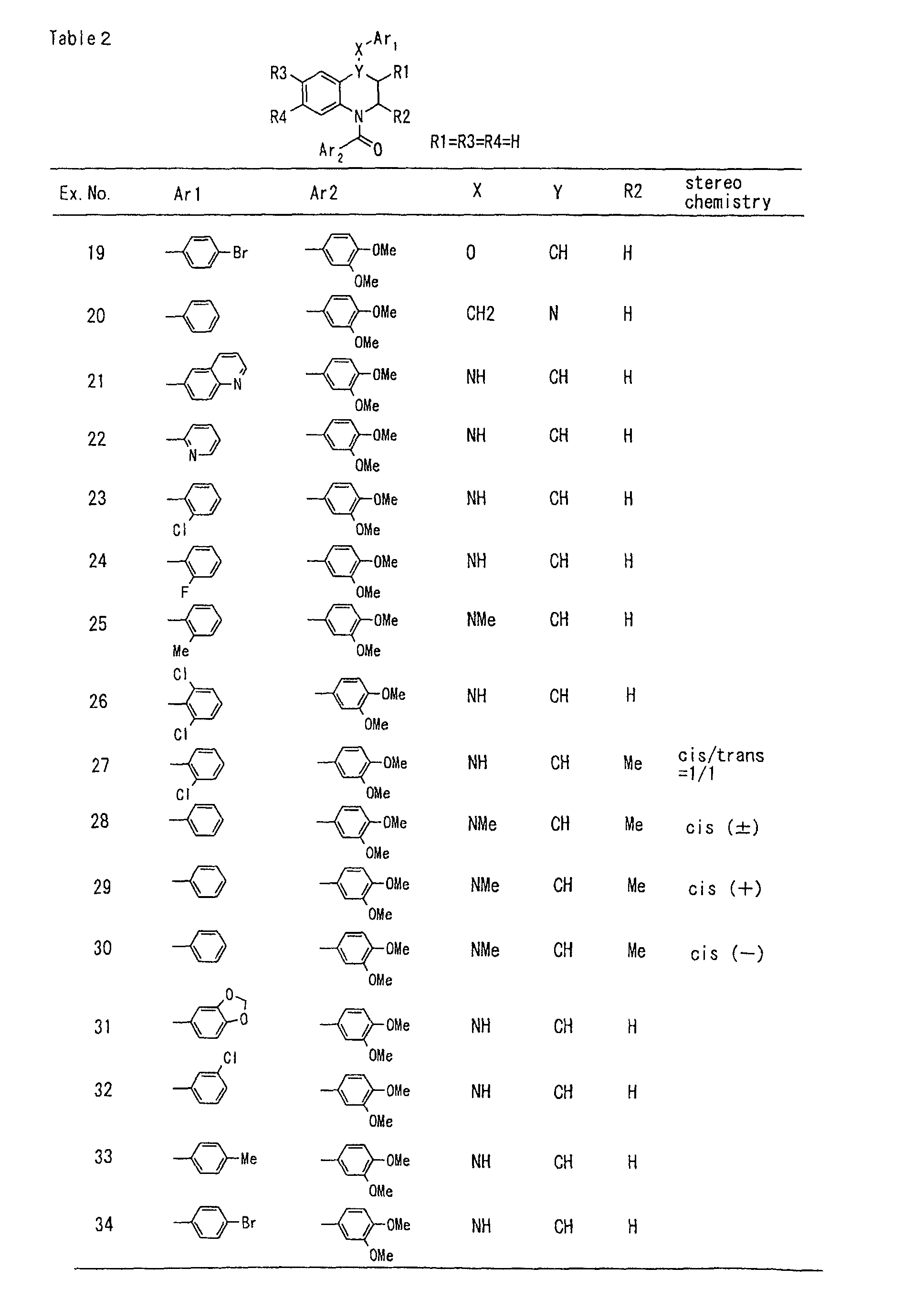

### EXAMPLE 35

### N-(2-Chlorophenyl)-1-(3,4-dimethoxybenzoyl)-N-methyl-1,2,3,4-tetrahydro-4-quinolinamine

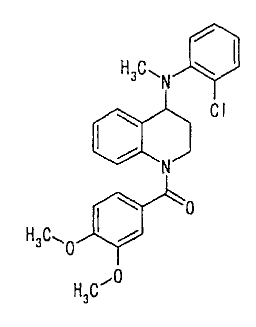

After 132 mg (0.312 mmols) ofN-(2-chlorophenyl)-1-(3,4-dimethoxybenzoyl)-1,2,3,4-tetrahydro-4-quinolinamine obtained in EXAMPLE 23 was dissolved in DMF (8 ml), 125 mg (3.12 mmols) of sodium hydride was added to the solution followed by stirring at room temperature for 45 minutes. Methyl iodide (0.38 ml) was added to the reaction solution. After stirring at room temperature for 18 hours, water and ethyl acetate were added and the mixture was then extracted. The organic layer was washed with water, dried and concentrated. The residue was subjected to silica gel column chromatography (developing solvent: ethyl acetate/hexane = 1/1). The eluate was recrystallized from ethyl acetate-hexane to give 105 mg (yield, 77%) of the product, N-(2-chlorophenyl)-1-(3,4-dimethoxybenzoyl)-N-methyl-1,2,3,4-tetrahydro-4-quinolinamine was obtained as white crystals.
Melting point: 146-147°C (solvent for crystallization: ethyl acetate-hexane)
FABMS (pos) 459.1 [M+Na⁺]

### EXAMPLE 36

### N-(4-Chlorophenyl)-1-(3,4-dimethoxybenzoyl)-N-methyl-1,2,3,4-tetrahydro-4-quinolinamine

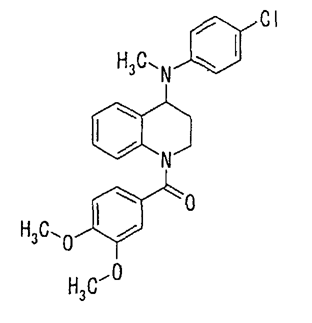

By the similar procedures as in EXAMPLE 12, 145 mg (yield, 35%) of the product, N-(4-chlorophenyl)-1-(3,4-dimethoxybenzoyl)-N-methyl-1,2,3,4-tetrahydro-4-quinolinamine was obtained as yellow crystals from 300 mg (0.957 mmols) of 1-(3,4-dimethoxybenzoyl)-1,2,3,4-tetrahydro-4-quinolinol obtained in REFERENCE EXAMPLE 2 and 408 mg (2.88 mmols) of 4-chloro-N-methylaniline.
Melting point: 72-74°C (solvent for crystallization: diethyl ether-hexane)
FABMS (pos) 436.2 [M⁺]

### EXAMPLE 37

### N-(4-Bromophenyl)-1-(3,4-dimethoxybenzoyl)-N-methyl-1,2,3,4-tetrahydro-4-quinolinamine

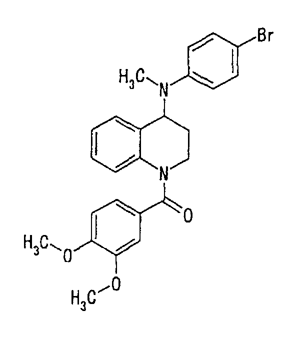

By the similar procedures as in EXAMPLE 35, 85 mg (yield, 52%) of the product, N-(4-bromophenyl)-1-(3,4-dimethoxybenzoyl)-N-methyl-1,2,3,4-tetrahydro-4-quinolinamine was obtained as white crystals from 150 mg (0.33 mmols) of N-(4-bromophenyl)-1-(3,4-dimethoxybenzoyl)-1,2,3,4-tetrahydro-4-quinolinamine.
Melting point: 73-74°C (solvent for crystallization: diethyl ether-hexane)
FABMS (pos) 480.0 [M⁺]

### EXAMPLE 38

### N-(3-Chloro-4-methylphenyl)-1-(3,4-dimethoxybenzoyl)-1,2,3,4-tetrahydro-4-quinolinamine

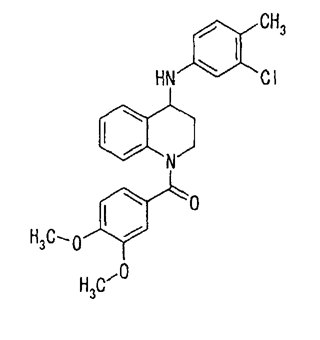

By the similar procedures as in EXAMPLE 12, 205 mg (yield, 37%) of the product, N-(3-chloro-4-methylphenyl)-1-(3,4-dimethoxybenzoyl)-1,2,3,4-tetrahydro-4-quinolinamine was obtained as white crystals from 400 mg (1.28 mmols) of 1-(3,4-dimethoxybenzoyl)-1,2,3,4-tetrahydro-4-quinolinol obtained in REFERENCE EXAMPLE 2 and 544 mg (3.84 mmols) of 4-amino-2-chlorotoluene.
Melting point: 94-96°C (solvent for crystallization: diethyl ether-hexane)
FABMS (pos) 459.0 [M+Na⁺]

### EXAMPLE 39

### 6-Chloro-N-(2-chlorophenyl)-1-(3,4-dimethoxybenzoyl)-1,2,3,4-tetrahydro-4-quinolinamine

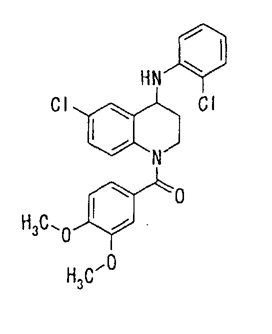

By the similar procedures as in EXAMPLE 12, 6 232 mg (yield, 44%) of 6-chloro-N-(2-chlorophenyl)-1-(3,4-dimethoxybenzoyl)-1,2,3,4-tetrahydro-4-quinolinamine was obtained as light yellow crystals from 400 mg (1.15 mmols) of 6-chloro-1-(3,4-dimethoxybenzoyl)-1,2,3,4-tetrahydro-4-quinolinol obtained in REFERENCE EXAMPLE 5 and 440 mg (3.45 mmols) of 2-chloroaniline.
Melting point: 87-89°C (solvent for crystallization: diethyl ether-hexane)
FABMS (pos) 479.0 [M+Na⁺]

### EXAMPLE 40

### 1-(5-Bromo-2-furoyl)-N-phenyl-1,2,3,4-tetrahydro-4-quinolinamine hydrochloride

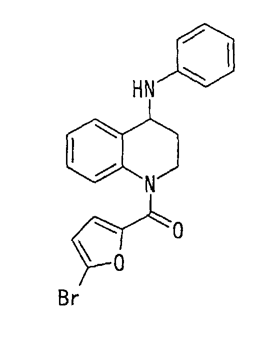

1-(5-Bromo-2-furoyl)-N-phenyl-1,2,3,4-tetrahydro-4-quinolinamine was obtained from 400 mg (1.24 mmols) of 1-(5-bromo-2-furoyl)-1,2,3,4-tetrahydro-4-quinolinol obtained in REFERENCE EXAMPLE 7 and 346 mg (3.72 mmols) of aniline, by the procedures similar to EXAMPLE 9. The resulting product was dissolved in diethyl ether and 0.5 ml of 4N hydrogen chloride-ethyl acetate solution was added to the solution followed by stirring. The precipitated crystals were taken out by filtration to give 271 mg (yield, 50%) of 1-(5-bromo-2-furoyl)-N-phenyl-1,2,3,4-tetrahydro-4-quinolinamine hydrochloride as white crystals.
FABMS (pos) 419.0 [M+Na⁺]

### EXAMPLE 41

### trans-1-(5-Bromo-2-furoyl)-2-methyl-N-phenyl-1,2,3,4-tetrahydro-4-quinolinamine

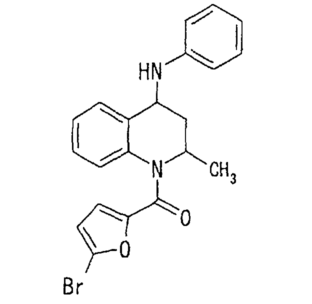

By the similar procedures as in EXAMPLE 4, the product, trans-1-(5-bromo-2-furoyl)-2-methyl-N-phenyl-1,2,3,4-tetrahydro-4-quinolinamine was obtained as white crystals from trans-2-methyl-N-phenyl-1,2,3,4-tetrahydro-4-quinolinamine and 5-bromo-2-furoylchloride.
Melting point: 117-118°C (solvent for crystallization: diethyl ether-hexane)

| Elemental analysis as C₂₁H₁₉N₂O₂Br | | | |
|---|---|---|---|
| Calcd. | C, 61.33; | H, 4.66; | N, 6.81 |
| Found | C, 61.39; | H, 4.60; | N, 6.75 |

### EXAMPLE 42

### 1-([1,1'-Biphenyl]-4-ylmethyl)-4-(3,4-dimethoxybenzoyl)-1,2,3,4-tetrahydroquinoxaline

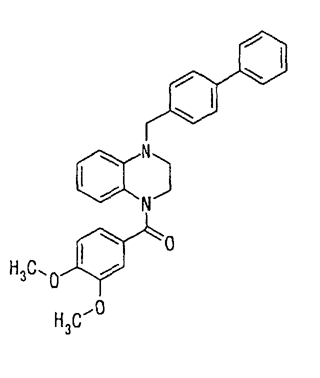

By the similar procedures as in EXAMPLE 20, 32 mg (yield, 28%) of the product, 1-([1,1'-biphenyl]-4-ylmethyl)-4-(3,4-dimethoxybenzoyl)-1,2,3,4-tetrahydroquinoxaline was obtained as white crystals from 75 mg (0.25 mmols) of 1-(3,4-dimcthoxybenzoyl)-1,2,3,4-tetrahydroquinoxaline synthesized in REFERENCE EXAMPLE 4 and 247 mg (1.00 mmols) of 4-bromomethylbiphenyl.
Melting point: 133-4°C (solvent for crystallization: ethyl acetate-diisopropyl ether)

| Elemental analysis as C₃₀H₂₈N₂O₃ | | | |
|---|---|---|---|
| Calcd. | C, 77.56; | H, 6.08; | N, 6.03 |
| Found | C, 77.29; | H, 5.89; | N, 5.93 |

### EXAMPLE 43

### N-(2,4-Dichlorophenyl)-1-(3,4-dimethoxybenzoyl)-2-methyl-1,2,3,4-tetrahydro-4-quinolinamine

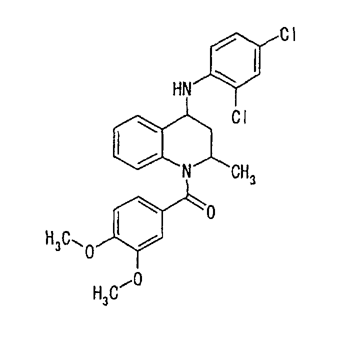

By the similar procedures as in EXAMPLE 12, 302 mg (yield, 53%) of the product, N-(2,4-dichlorophenyl)-1-(3,4-dimethoxybenzoyl)-2-methyl-1,2,3,4-tetrahydro-4-quinolinamine was obtained as white crystals from 200 mg (0.61 mmols) of cis-1-(3,4-dimethoxybenzoyl)-2-methyl-1,2,3,4-tetrahydro-4-quinolinol obtained in REFERENCE EXAMPLE 1 and 593 mg (3.66 mmols) of 2,4-dichloroaniline.
(cis/trans = 1/1.6)
Melting point: 165-167°C (solvent for crystallization: diethyl ether-hexane)
FABMS (pos) 493.0 [M+Na⁺]

### EXAMPLE 44

### 1-(3,4-Dimethoxybenzoyl)-N-(2-isopropylphenyl)-1,2,3,4-tetrahydro-4-quinolinamine hydrochloride

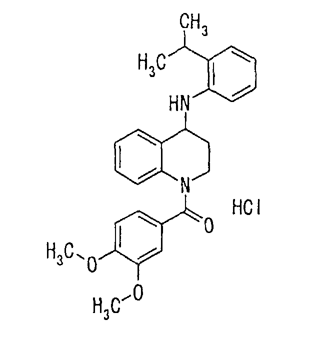

By the similar procedures as in EXAMPLE 12, 1-(3,4-dimethoxybenzoyl)-N-(2-isopropylphenyl)-1,2,3,4-tetrahydro-4-quinolinamine was obtained from 300 mg (0.957 mmols) of 1-(3,4-dimethoxybenzoyl)-1,2,3,4-tetrahydro-4-quinolinol obtained in REFERENCE EXAMPLE 2 and 260 mg (1.92 mmols) of o-isopropylamine. The product obtained was dissolved in diethyl ether and 0.5 ml of 4N hydrogen chloride-ethyl acetate solution was added to the solution followed by stirring. The precipitated crystals were taken out by filtration to give 57 mg (yield, 8%) of 1-(3,4-dimethoxybenzoyl)-N-(2-isopropylphenyl)-1,2,3,4-tetrahydro-4-quinolinamine hydrochloride as white crystals.
Melting point: 93-96°C (solvent for crystallization: diethyl ether)
FABMS (pos) 453.1 [M+Na⁺]

### EXAMPLE 45

### 1-(3,4-Dimethoxybenzoyl)-N-ethyl-2-methyl-N-phenyl-1,2,3,4-tetrahydro-4-quinolinamine hydrochloride

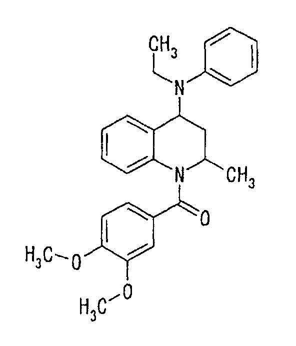

By the similar procedures as in EXAMPLE 44, 76.7 mg (yield, 22%) of the product, 1-(3,4-dimethoxybenzoyl)-N-ethyl-2-methyl-N-phenyl-1,2,3,4-tetrahydro-4-quinolinamine hydrochloride was obtained as white crystals from 250 mg (0.76 mmols) of cis-1-(3,4-dimethoxybenzoyl)-2-methyl-1,2,3,4-tetrahydro-4-quinolinol obtained in REFERENCE EXAMPLE 1 and 368 mg (3.04 mmols) of N-ethylaniline.
(cis/trans = 3/2)
FABMS (pos) 453.1 [M+Na⁺]

### EXAMPLE 46

### N-(2-Chlorophenyl)-1-(3,4-dimethoxybenzoyl)-N,2-dimethyl-1,2,3,4-tetrahydro-4-quinolinamine

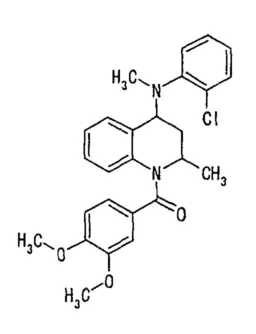

By the similar procedures as in EXAMPLE 12, 80 mg (yield, 15%) of the product, N-(2-chlorophenyl)-1-(3,4-dimethoxybenzoyl)-N,2-dimethyl-1,2,3,4-tetrahydro-4-quinolinamine from 400 mg (1.22 mmols) of cis-1-(3,4-dimethoxybenzoyl)-2-methyl-1,2,3,4-tetrahydro-4-quinolinol obtained in REFERENCE EXAMPLE 1 and 518 mg (3.66 mmols) of 2-chloro-N-methylaniline.
(cis/trans =3/1)
FABMS (pos) 473.2 [M+Na⁺]

### EXAMPLE 47

### 6-Chloro-N-(4-chlorophenyl)-1-(3,4-dimethoxybenzoyl)-1,2,3,4-tetrahydro-4-quinolinamine

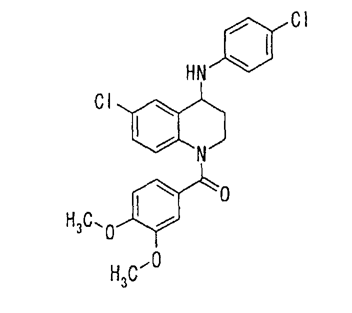

By the similar procedures as in EXAMPLE 12, 250 mg (yield, 48%) of the product, 6-chloro-N-(4-chlorophenyl)-1-(3,4-dimethoxybenzoyl)-1,2,3,4-tetrahydro-4-quinolinamine was obtained as light yellow crystals from 400 mg (1.15 mmols) of 6-chloro-1-(3,4-dimethoxybenzoyl)-1,2,3,4-tetrahydro-4-quinolinol obtained in REFERENCE EXAMPLE 5 and 440 mg (3.45 mmols) of 4-chloroaniline.
Melting point: 176-178°C (solvent for crystallization: ethyl acetate-hexane)
FABMS(pos) 479.1 [M+Na⁺]

### EXAMPLE 48

### 6-Chloro-1-(3,4-dimethoxybenzoyl)-N-phenyl-1,2,3,4-tetrahydro-4-quinolinamine

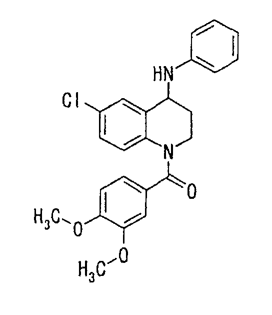

By the similar procedures as in EXAMPLE 12, 188 mg (yield, 34%) of the product, 6-chloro-1-(3,4-dimethoxybenzoyl)-N-phenyl-1,2,3,4-tetrahydro-4-quinolinamine was obtained as light yellow crystals from 400 mg (1.15 mmols) of 6-chloro-1-(3,4-dimethoxybenzoyl)-1,2,3,4-tetrahydro-4-quinolinol obtained in REFERENCE EXAMPLE 5 and 321 mg (3.45 mmols) of aniline.
Melting point: 188-189°C (solvent for crystallization: ethyl acetate-hexane)
FABMS (pos) 445.1 [M+Na⁺]

### EXAMPLE 49

### 7-Chloro-N-(4-chlorophenyl)-1-(3,4-dimethoxybenzoyl)-1,2,3,4-tetrahydro-4-quinolinamine

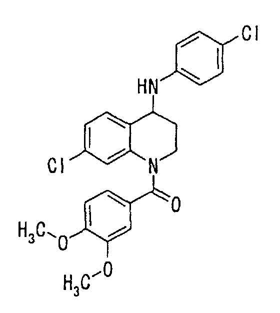

By the similar procedures as in EXAMPLE 12, 168 mg (yield, 32%) of the product, 7-chloro-N-(4-chlorophenyl)-1-(3,4-dimethoxybenzoyl)-1,2,3,4-tetrahydro-4-quinolinamine was obtained as light yellow crystals from 400 mg (1.15 mmols) of7-chloro-1-(3,4-dimethoxybenzoyl)-1,2,3,4-tetrahydro-4-quinolinol obtained in REFERENCE EXAMPLE 6 and 440 mg (3.45 mmols) of 4-chloroaniline.
FABMS (pos) 479.1 [M+Na⁺]

The title compounds obtained in EXAMPLES 35 through 49 are summarized in TABLE 3, wherein Stereochemistry designates the stereochemistry between. XAr¹ and R².

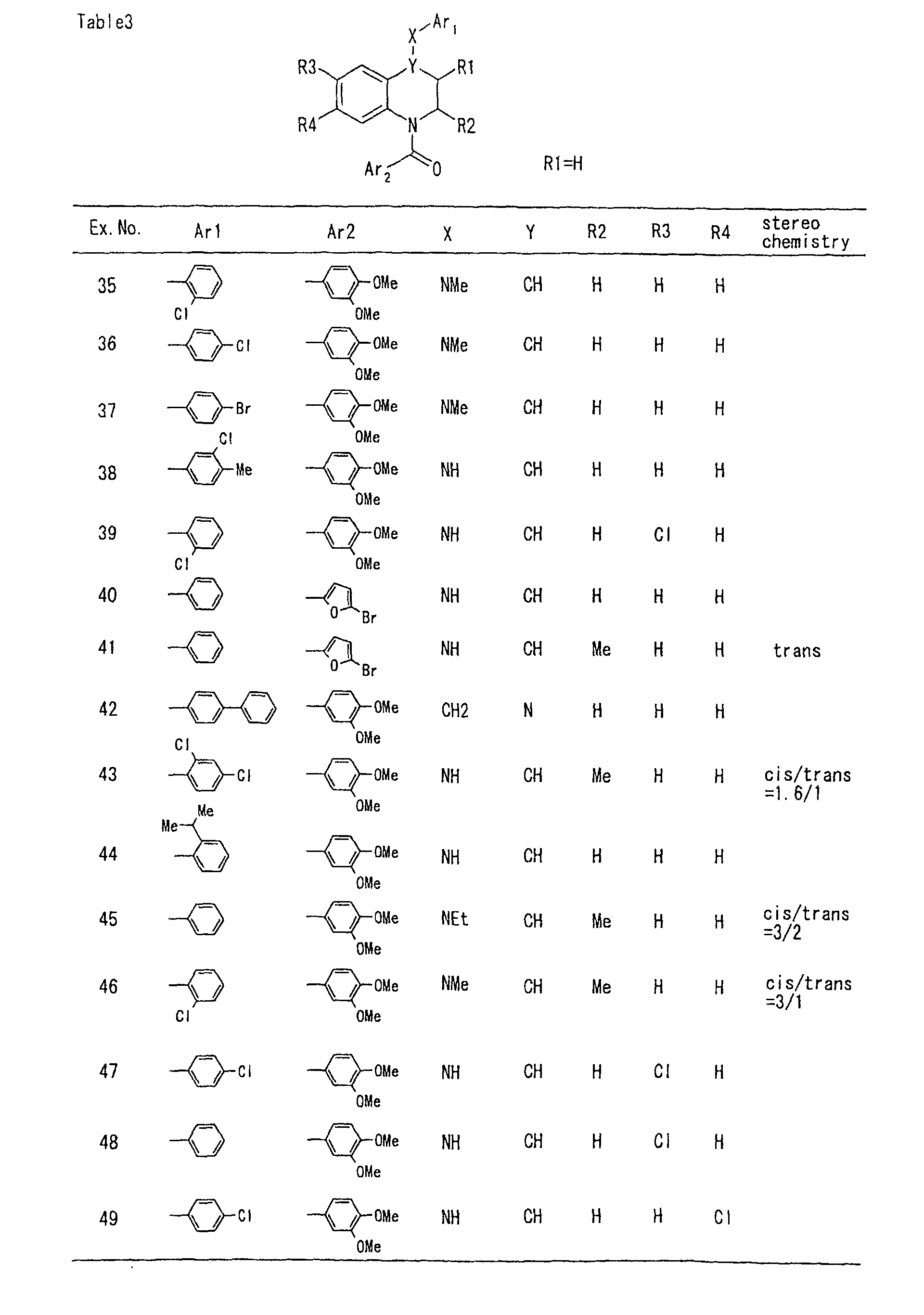

### EXAMPLE 50

### 7-Chloro-1-(3,4-dimethoxybenzoyl)-N-phenyl-1,2,3,4-tetrahydro-4-quinolinamine

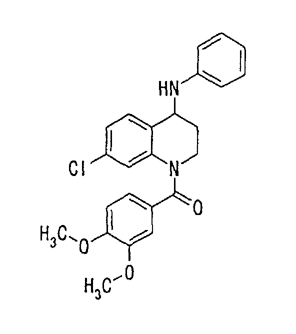

By the similar procedures as in EXAMPLE 12, 230 mg (yield, 44%) of the product, 7-chloro-1-(3,4-dimethoxybenzoyl)-N-phenyl-1,2,3,4-tetrahydro-4-quinolinamine was obtained as light yellow crystals from 400 mg (1.15 mmols) of 7-chloro-1-(3,4-dimethoxybenzoyl)-1,2,3,4-tetrahydro-4-quinolinol obtained in REFERENCE EXAMPLE 6 and 321 mg (3.45 mmols) of aniline.
Melting point: 125-126°C (solvent for crystallization: ethyl acetate-hexane)
FABMS (pos) 445.1 [M+Na⁺]

### EXAMPLE 51

### 7-Chloro-N-(2-chlorophenyl)-1-(3,4-dimethoxybenzoyl)-1,2,3,4-tetrahydro-4-quinolinamine hydrochloride

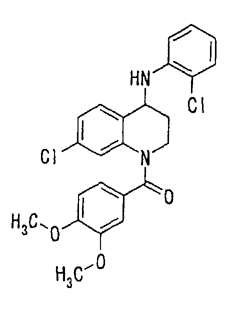

By the similar procedures as in EXAMPLE 12, the product, 7-chloro-N-(2-chlorophenyl)-1-(3,4-dimethoxybenzoyl)-1,2,3,4-tetrahydro-4-quinolinamine was obtained from 400 mg (1.15 mmols) of 7-chloro-1-(3,4-dimethoxybenzoyl)-1,2,3,4-tetrahydro-4-quinolinol obtained in REFERENCE EXAMPLE 6 and 440 mg (3.45 mmols) of 2-chloroaniline. The product obtained was dissolved in diethyl ether and 0.5 ml of 4N hydrogen chloride-ethyl acetate solution was added to the solution followed by stirring. The precipitated crystals were taken out by filtration to give 29 mg (yield, 5%) of 7-chloro-N-(2-chlorophenyl)-1-(3,4-dimethoxybenzoyl)-1,2,3,4-tetrahydro-4-quinolinamine hydrochloride as light yellow crystals.
Melting point: 74-76°C (solvent for crystallization: diethyl ether-hexane)
FABMS (pos) 479.1 [M+Na⁺]

### EXAMPLE 52

### cis-1-[(4'-Chloro[1,1'-biphenyl]-4-yl)carbonyl]-2-methyl-N-phenyl-1,2,3,4-tetrahydro-4-quinolinamine

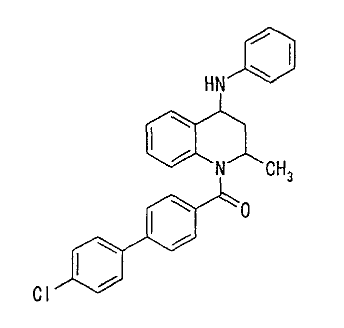

By the similar procedures as in EXAMPLE 2, 240 mg (yield, 45%) of the product, cis-1-[(4'-chloro[1,1'-biphenyl]-4-yl)carbonyl]-2-methyl-N-phenyl-1,2,3,4-tetrahydro-4-quinolinamine was obtained as white crystals from 477 mg (2.00 mmols) of cis-2-methyl-N-phenyl-1,2,3,4-tetrahydro-4-quinolinamine and 233 mg (1.00 mmols) of 4'-chlorobiphenylcarboxylic acid.
Melting point: 193-194°C (solvent for crystallization: ethyl acetate-hexane)

| Elemental analysis as C₂₉H₂₅N₂OCl | | | |
|---|---|---|---|
| Calcd. | C, 76.89; | H, 5.56; | N, 6.18 |
| Found | C, 76.87; | H, 5.58; | N, 6.14 |

### EXAMPLE 53

### trans-1-(2,4-Difluorobenzoyl)-2-methyl-N-phenyl-1,2,3,4-tetrahydro-4-quinolinamine

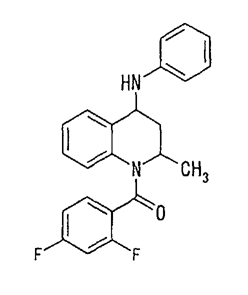

By the similar procedures as in EXAMPLE 2, trans-1-(2,4-difluorobenzoyl)-2-methyl-N-phenyl-1,2,3,4-tetrahydro-4-quinolinamine was obtained as white crystals from trans-2-methyl-N-phenyl-1,2,3,4-tetrahydro-4-quinolinamine and 2,4-difluorobenzoyl chloride.
Melting point: 143.6°C (solvent for crystallization: ethyl acetate-hexane)

### EXAMPLE 54

### 1-(3,4-Dimethoxybenzoyl)-2-ethyl-N-methyl-N-phenyl-1,2,3,4-tetrahydro-4-quinolinamine

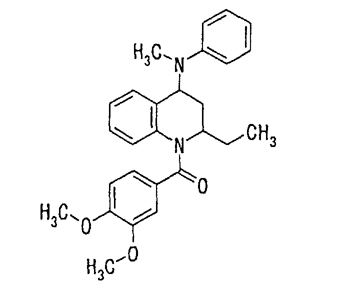

By the similar procedures as in EXAMPLE 12, 190 mg (yield, 38%) of the product, 1-(3,4-dimethoxybenzoyl)-2-ethyl-N-methyl-N-phenyl-1,2,3,4-tetrahydro-4-quinolinamine was obtained as white crystals from 400 mg (1.17 mmols) of cis-1-(3,4-dimethoxybenzoyl)-2-ethyl-1,2,3,4-tetrahydro-4-quinolinol obtained in REFERENCE EXAMPLE 8 and376 mg (3.51 mmols) of N-methylaniline.
(cis/trans= 1/3).
Melting point: 146-148°C (solvent for crystallization: diethyl ether-hexane) FABMS (pos) 453.2 [M+Na⁺]

### EXAMPLE 55

### Ethyl 6-(4- {[1-(3,4-Dimethoxybenzoyl)-2-methyl-1,2,3,4-tetrahydro-4-quinolinyl]-amino)phenoxy}hexanoate

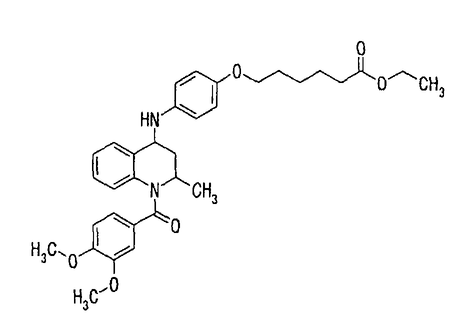

By the similar procedures as in EXAMPLE 12, 337 mg (yield, 39%) of the product, ethyl 6-(4-{[1-(3,4-dimethoxybenzoyl)-2-methyl-1,2,3,4-tetrahydro-4-quinolinyl]amino}phenoxy)hexanoate was obtained from 500 mg (1.60 mmols) of cis-1-(3,4-dimethoxybenzoyl)-2-methyl-1,2,3,4-tetrahydro-4-quinolinol obtained in REFERENCE EXAMPLE 1 and 1.50 g (6.40 mmols) of ethyl 6-(4-aminophenoxy)hexanoate. (cis/trans = 3/1)
¹H-NMR (CDCl₃) δ: 1.23-1.41 (9H, m), 1.47-1.55 (2H,m), 1.63-1.83 (3H, m), 2.33 (2H, t, J=7.2 Hz), 2.55 (0.25H, m), 2.61 (0.75H, m), 3.18 (0.75H, s), 3.67 (2.25H, s), 3.82-3.93 (2H, m), 3.85 (3H,s), 4.13 (2H, q, J=7.2 Hz), 4.39 (0.75H, m), 4.58 (0.25H, m), 4.89 (1H, m), 6.57-7.10 (10H, m), 7.34-7.43 (1H, m)

### EXAMPLE 56

### trans-2-Methyl-1-(3-methyl-2-furoyl)-N-phenyl-1,2,3,4-tetrahydro-4-quinolinamine

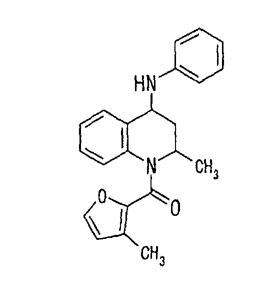

By the similar procedures as in EXAMPLE 2, 535 mg (yield, 39%) of trans-2-methyl-1-(3-methyl-2-furoyl)-N-phenyl-1,2,3,4-tetrahydro-4-quinolinamine was obtained as white crystals from 953 mg (4.00 mmols) oftrans-2-methyl-N-phenyl-1,2,3,4-tetrahydro-4-quinolinamine and 500 mg (4.00 mmols) of 3-methyl-2-furanecarboxylic acid.
¹H-NMR (CDCl₃) δ: 1.35 (3H, d, J=6.6 Hz), 1.93-2.00 (1H, m), 2.25 (3H, s), 2.43-2.52 (1H, m), 4.06 (1H, brs.), 4.72-4.78 (2H,m), 6.26 (1H, s), 6.64-6.79 (4H, m), 7.06-7.43 (6H, m)

The title compounds obtained in EXAMPLES 50 through 56 are summarized in TABLE 4, wherein Stereochemistry designates the stereochemistry between XAr¹ and R².

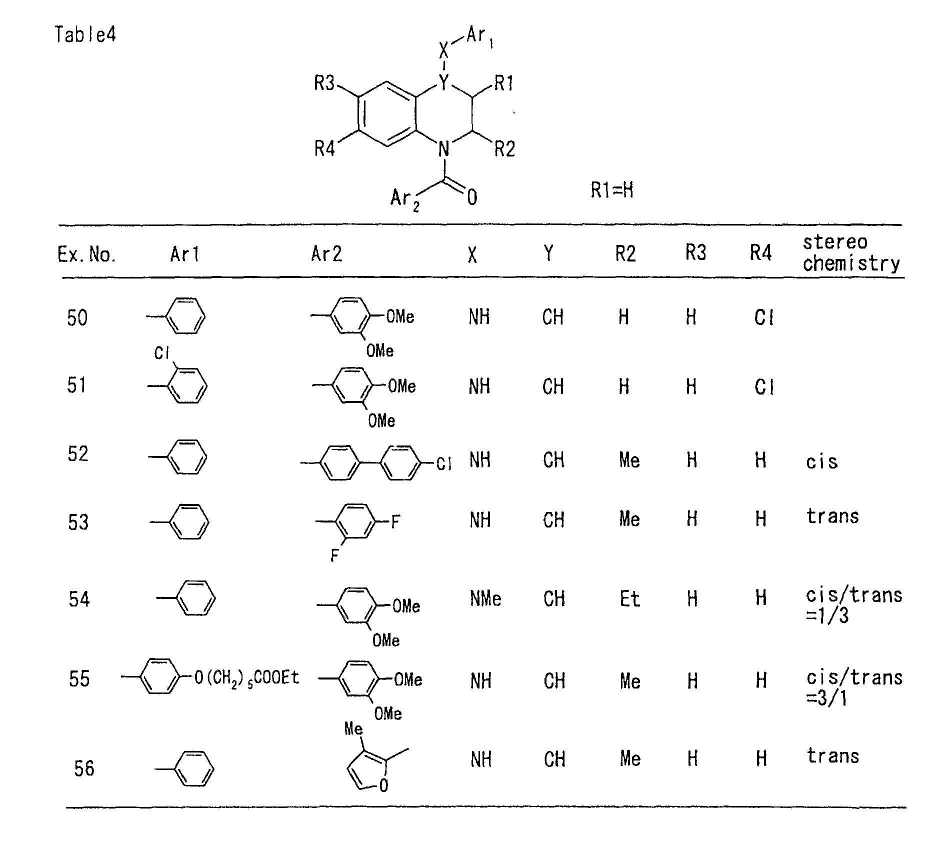

### EXPERIMENTAL EXAMPLE 1

### Activity of promoting the sAPP secretion in rat-derived pheochromocytoma PC12h cells

### (1) Materials

Substrain PC12h cells of rat-derived pheochromocytoma PC12h cells (Brain Res., 222 (191), p. 225-233) were handed out by Professor Hiroshi Hatanaka of Osaka University. Dulbecco's modified Eagle's medium (DMEM) was purchased from Nissui Seiyaku K.K., HEPES (N-2-hydroxyethylpiperazine-N'-2-ethanesulfonic acid) buffer (pH 7.4), horse serum (HS) and a penicillin (5000 U/ml)/streptomycin (5 mg/ml) mixture from Gibco, and fetal calf serum (FCS) from BioWhittaker, Inc. A flask for incubation and a 6-well multi-well plate coated with collagen manufactured by Falcon Co., Ltd. and Iwaki Glass Co., Ltd., respectively, were used. Anti-βAPP 22C11 antibody was purchased from Roche Diagnostics, Inc. Other reagents used were special grade chemicals commercially available.

### (2) Method

PC12h cells were grown in DMEM medium supplemented with 10% FCS/5% HS/penicillin streptomycin, the cells were taken out by pipetting. The cells were inoculated on the collagen-coated 6-well plate to become 50-70% confluent, which was allowed to stand for a day. On the following day, the well was rinsed twice with 1 ml of 10 mM HEPES/DMEM. After the medium was exchanged with 0.5 ml of 10 mM HEPES/DMEM medium, a compound was added thereto. The culture supernatant was recovered 3 hours after.

The culture supernatant was concentrated by ultrafiltration using Microcon 10 (Amicon, Inc.). A sample was applied to SDS-PAGE (10/20% gel, ATTO) and transferred (2 mA/cm², 60 mins.) onto a PVDF membrane (Bio-Rad Laboratories). Blocking was performed at 4°C overnight using 0.1% Tween 20/20 mM Tris-buffered saline (pH 7.4) containing 5% skimmed milk. Anti-βAPP 22C11 antibody was added to the medium in a 30-fold dilution, and reacted for an hour. As a secondary antibody there was used HRP (horse radish peroxidase)-bound anti-mouse antibody (Vector, Inc.) in a 10,000-fold dilution. ECL Plus (Amersham, Inc.) was used for detection, followed by digitalization in Fluor-S Max (Bio-Rad Laboratories) and quantification using NIH-image.

### (3) Results

The sAPP secretion-promoting activities observed 3 hours after the addition of Compounds 1-2, 2-2, 1-3 and 2-3 are shown in TABLE 5 and FIGS. 1 and 2. FIG. 1 shows the results of representative Western blotting observed 3 hours after 3 µM of each compound was added. In all of the compounds, the sAPP level in the medium increased, as compared to the control group. FIG. 2 shows a mean relative value in the 3 wells of the sAPP secretion levels by each compound to the control group, obtained after quantification of the images using NIH Image. The sAPP level increased to about 1.4 times by Compound 1-2, to times by Compound 2-2, to 3.3 times by Compound 1-3, and to 3 times by Compound 2-3, as compared to the control group.

**TABLE 5**

| Compound added | sAPP secretion level (relative value to the control group, when the control group is made 100) |
|---|---|
| Control group | 100 |
| 1-2 | 139.37 |
| 2-2 | 278.43 |
| 1-3 | 325.14 |
| 2-3 | 298.36 |

### EXPERIMENTAL EXAMPLE 2

### Activity of promoting the sAPP secretion in rat-derived pheochromocytoma PC12h cells

### (1) Materials

Substrain PC12h cells of rat-derived pheochromocytoma PC12h cells were handed out by Professor Hiroshi Hatanaka of Osaka University. Dulbecco's modified Eagle's medium (DMEM) was purchased from Nissui Seiyaku K.K., HEPES (N-2-hydroxyethylpiperazine-N'-2-ethanesulfonic acid) buffer (pH 7.4), horse serum (HS) and a penicillin (5000 U/ml)/streptomycin (5 mg/ml) mixture from Gibco, fetal calf serum (FCS) from BioWhittaker, Inc., transferrin, insulin and progesterone from Sigma, Inc. A flask for incubation and a collagen-coated 96-well multi-well plate used were manufactured by Falcon Co., Ltd. and Iwaki Glass Co., Ltd., respectively. LDH cytotoxic test Wako was purchased from Wako Pure Chemical Industries, Ltd. Other reagents used were special grade chemicals commercially available.

### (2) Method

PC12h cells were inoculated on the 96-well plate in 2 x 10⁴ cells/cm². On the following day, the medium was exchanged with HEPES/DME medium containing 5 mg/ml transferrin, 5 mg/ml insulin and 20 nM progesterone. The cells survived 72 hours after were counted. The cell number was determined by solubilizing the cells survived in PBS containing Tween 20 after removal of the medium and assaying in terms of the LDH activity.

### (3) Results

In PC12h cells, glutamate in the medium hampers uptake of cystine, or a material to make glutathione, into cells. Thus, when 1 mM or more glutamates are added to a medium and incubation is continued for 3 days or longer, it is known that PC12h cells become extinct due to apoptosis accompanied by a reduced level of glutathione in the cells. The compounds of the present invention used in EXPERIMENTAL EXAMPLE 1 all prevented this cell death caused by the addition of 1 mM glutamate concentration-dependently within the range of 0.1 to 3 µM, and the EC₅₀ value was 0.3 to 1 µM. In the compounds assayed, the prevention by Compound 2-3 was the strongest and the maximum prevention activity was noted with 1 µM, showing about 90% surviving rate (TABLE 6 and FIG. 3).

**TABLE 6**

| Concentration of Compound (µM) | Surviving Rate (%) | | | |
|---|---|---|---|---|
| | Compound 1-2 | Compound 2-2 | Compound 1-3 | Compound 2-3 |
| 0 | 5.55 | 4.49 | 4.58 | 4.59 |
| 0.1 | 6.54 | 5.93 | 4.89 | 7.70 |
| 0.3 | 16.75 | 16.41 | 7.44 | 38.57 |
| 1.0 | 68.32 | 67.94 | 49.17 | 86.63 |
| 3.0 | 82.12 | 75.29 | 77.51 | 86.34 |
| 10.0 | 68.30 | 66.96 | 63.24 | 56.90 |

The compounds of the present invention possess a potent activity of promoting the secretion of soluble beta amyloid precursor protein and suppresses the functional disorders or apoptosis of cells, in particular neurons, mediated by the secreted soluble beta amyloid precursor proteins having a neurotrophic factor like property. Examples of the target diseases for which they are useful include Alzheimer's disease, Parkinson's disease, prion disease, neuropathy (preferably diabetic neuropathy, etc.), senile dementia, neuronal disorders associated with cerebrovascular disorders, cerebrovascular dementia, neuronal disorders associated with cerebrovascular disorders, etc. The compounds can be used for the prevention or treatment of these diseases.

## Claims

1. A pharmaceutical composition comprising a compound having a soluble beta amyloid precursor protein secretion promoting activity and an apoptosis inhibiting activity, or a salt thereof, or a prodrug thereof.

2. The pharmaceutical composition according to claim 1, comprising a compound containing:
1) a partial structure represented by formula (a): 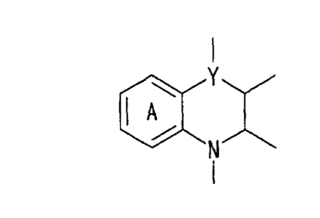 [wherein ring A represents a benzene ring which may optionally be substituted, and Y represents CH or N];
2) a partial structure represented by formula (b): 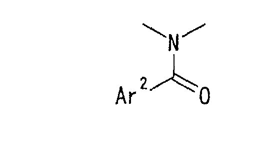 [wherein Ar² represents an aromatic group which may optionally be substituted]; or,
3) a partial structure represented by formula (c): 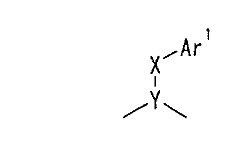 [wherein Ar¹ represents an aromatic group which may optionally be substituted; X represents oxygen atom, CR³R⁴ or NR⁵ (wherein R³, R⁴ or R⁵, which may be the same or different, each represents hydrogen atom or a lower alkyl group which may optionally be substituted), and Y has the same significance as defined above]; or a salt thereof, or a prodrug thereof.

3. An agent for promoting soluble beta amyloid precursor protein secretion comprising a compound represented by formula (I): 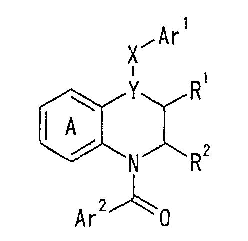 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; ring A represents a benzene ring which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y represents CH or N] or a salt thereof, or a prodrug thereof.

4. The agent according to claim 3, wherein R¹ is hydrogen atom.

5. The agent according to claim 3, wherein R² is hydrogen atom or a lower alkyl group which may optionally be substituted.

6. The agent according to claim 3, wherein R² is hydrogen atom or a C₁₋₆ alkyl group.

7. The agent according to claim 3, wherein Ar¹ is a phenyl group which may optionally be substituted or a quinolyl group which may optionally be substituted.

8. The agent according to claim 3, wherein Ar¹ is a phenyl group which may optionally be substituted.

9. The agent according to claim 3, wherein Ar¹ is a phenyl group which may optionally be substituted with a halogen atom, a C₁₋₃ alkylenedioxy group, an optionally halogenated C₁₋₆ alkyl group or an optionally halogenated C₁₋₆ alkoxy group.

10. The agent according to claim 3, wherein Ar² is a monocyclic aromatic group which may optionally be substituted.

11. The agent according to claim 3, wherein Ar² is a phenyl, furyl, thienyl or pyridyl group, each of which may optionally be substituted.

12. The agent according to claim 3, wherein Ar² is a phenyl, furyl, thienyl or pyridyl group, each of which may optionally be substituted with a halogen atom, a C₁₋₃ alkylenedioxy group, an optionally halogenated C₁₋₆ alkyl group or an optionally halogenated C₁₋₆ alkoxy group.

13. The agent according to claim 3, wherein the ring A is unsubstituted benzene ring.

14. The agent according to claim 3, wherein X is NR⁵ (R⁵ has the same significance as defined in claim 3) and Y is CH.

15. The agent according to claim 3, wherein R¹ is hydrogen atom, R² is methyl, Ar¹ is a phenyl group which may optionally be substituted, Ar² is 3,4-dimethoxyphenyl group, ring A is unsubstituted benzene ring, X is NR⁵ (R⁵ has the same significance as defined in claim 3) and Y is CH.

16. An agent for inhibiting apoptosis comprising a compound represented by formula (I): 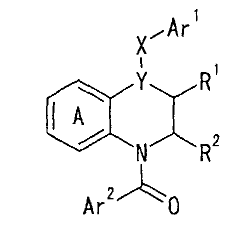 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; ring A represents a benzene ring which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y represents CH or N] or a salt thereof, or a prodrug thereof.

17. The agent according to claim 3 or 16, which is an agent for improving neuronal dysfunction.

18. The agent according to claim 3 or 16, which is an agent for the prevention/treatment of a neurodegenerative disease.

19. The agent according to claim 18, wherein the neurodegenerative disease is Alzheimer's disease, Parkinson's disease, prion disease or neuropathy.

20. Use of a compound having a soluble beta amyloid precursor protein secretion promoting activity and an apoptosis inhibiting activity, or a salt thereof, or a prodrug thereof, for the production of a pharmaceutical having an activity of improving neuronal dysfunction.

21. Use of a compound having a soluble beta amyloid precursor protein secretion promoting activity and an apoptosis inhibiting activity, or a salt thereof, or a prodrug thereof, for the production of an agent for the prevention/treatment of a neurodegenerative disease.

22. Use of a compound having a soluble beta amyloid precursor protein secretion promoting activity and an apoptosis inhibiting activity, or a salt thereof, or a prodrug thereof, for the production of an agent for the prevention/treatment of Alzheimer's disease, Parkinson's disease, prion disease or neuropathy.

23. Use of a compound containing:
1) a partial structure represented by formula (a): 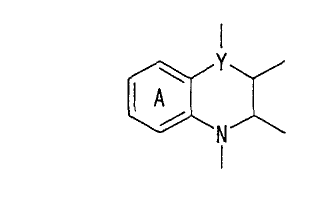 [wherein ring A represents a benzene ring which may optionally be substituted and Y represents CH or N];
2) a partial structure represented by formula (b): 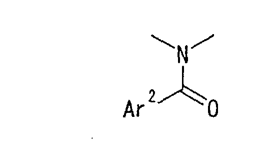 [wherein Ar² represents an aromatic group which may optionally be substituted]; or,
3) a partial structure represented by formula (c): 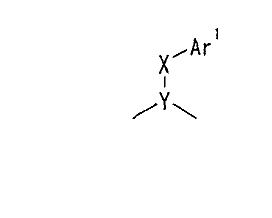 [wherein Ar¹ represents an aromatic group which may optionally be substituted; X represents oxygen atom, CR³R⁴ or NR⁵ (wherein R³, R⁴ or R⁵, which may be the same or different, each represents hydrogen atom or a lower alkyl group which may optionally be substituted), and Y has the same significance as defined above]; or a salt thereof, or a prodrug thereof, for the production of a pharmaceutical having a soluble beta amyloid precursor protein secretion promoting activity and an apoptosis inhibiting activity.

24. Use of a compound represented by formula (I): 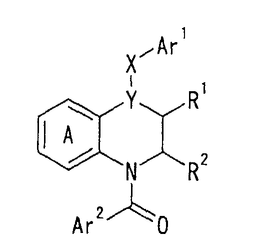 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; ring A represents a benzene ring which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y represents CH or N] or a salt thereof, or a prodrug thereof, for the production of a pharmaceutical having a soluble beta amyloid precursor protein secretion promoting activity and/or an apoptosis inhibiting activity.

25. A method of improving neuronal dysfunction, which comprises administering to a mammal a compound having a soluble beta amyloid precursor protein secretion promoting activity and an apoptosis inhibiting activity, or a salt thereof, or a prodrug thereof.

26. A method for the prevention/treatment of a neurodegenerative disease, which comprises administering to a mammal a compound having a soluble beta amyloid precursor protein secretion promoting activity and an apoptosis inhibiting activity, or a salt thereof, or a prodrug thereof.

27. The method for the prevention/treatment according to claim 26, wherein the neurodegenerative disease is Alzheimer's disease, Parkinson's disease, prion disease or neuropathy.

28. A method for promoting soluble beta amyloid precursor protein secretion and/or inhibiting apoptosis, which comprises administering to a mammal a compound containing:
1) a partial structure represented by formula (a): 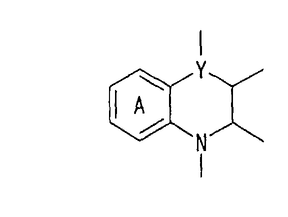 [wherein ring A represents an optionally substituted benzene ring and Y represents CH or N];
2) a partial structure represented by formula (b): 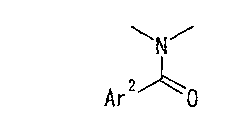 [wherein Ar² represents an aromatic group which may optionally be substituted]; or,
3) a partial structure represented by formula (c): 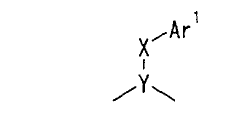 [wherein Ar¹ represents an aromatic group which may optionally be substituted; X represents oxygen atom, CR³R⁴ or NR⁵ (wherein R³, R⁴ or R⁵, which may be the same or different, each represents hydrogen atom or a lower alkyl group which may optionally be substituted), and Y has the same significance as defined above]; or a salt thereof, or a prodrug thereof.

29. A method for promoting soluble beta amyloid precursor protein secretion and/or inhibiting apoptosis, which comprises administering to a mammal a compound represented by formula (I): 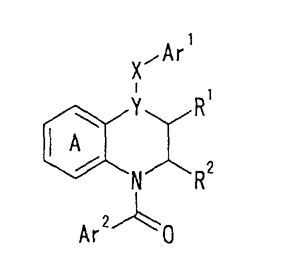 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; ring A represents a benzene ring which may optionally be substituted; X represents oxygen atom, CR³R⁴ or NR⁵ (wherein R³, R⁴ or R⁵, which may be the same or different, each represents hydrogen atom or a lower alkyl group which may optionally be substituted); and Y represents CH or N] or a salt thereof, or a prodrug thereof.

30. A compound represented by formula (II); 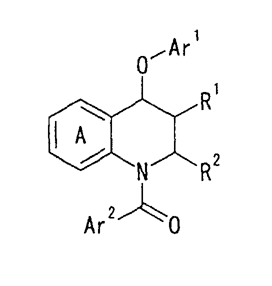 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; and ring A represents a benzene ring which may optionally be substituted] or a salt thereof, or a prodrug thereof.

31. The compound or a salt thereof, or a prodrug thereof, according to claim 30, wherein R¹ is hydrogen atom, R² is methyl, Ar¹ is a phenyl group which may optionally be substituted, Ar² is 3,4-dimethoxyphenyl group, and ring A is unsubstituted benzene ring.

32. A pharmaceutical composition comprising the compound or a salt thereof, or a prodrug thereof, according to claim 30.

33. A process of manufacturing a compound represented by formula (II): 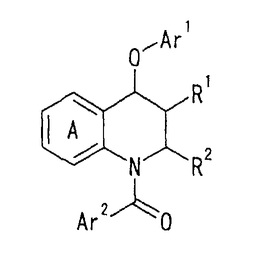 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; each of Ar¹ and Ar² represents an aromatic group which may optionally be substituted; and ring A represents a benzene ring which may optionally be substituted] or a salt thereof, or a prodrug thereof, which comprises condensation-reacting a compound represented by formula: 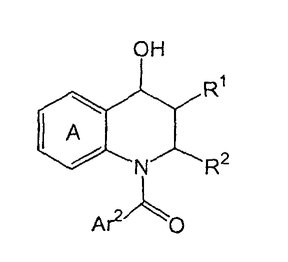 [wherein R¹, R², Ar² and ring A have the same significance as defined above] or a salt thereof, with a compound represented by formula:
Ar¹OH
[wherein Ar¹ has the same significance as defined above] or a salt thereof.

34. A compound represented by formula (III): 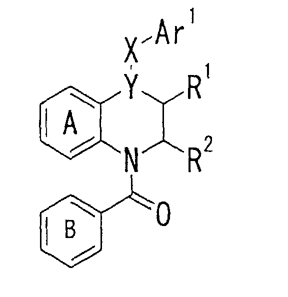 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; Ar¹ represents an aromatic group which may optionally be substituted; ring A represents a benzene ring which may optionally be substituted; ring B represents a benzene ring, which may optionally be substituted with an optionally halogenated C₁₋₆ alkoxy group, hydroxy group or a C₁₋₃ alkylenedioxy group; X represents CR³R⁴ or NR⁵ (wherein R³, R⁴ or R⁵, which may be the same or different, each represents hydrogen atom or a lower alkyl group which may optionally be substituted); and Y represents CH or N (provided that the compounds wherein X is NH, Y is CH and Ar¹ is unsubstituted phenyl group are excluded)] or a salt thereof, or a prodrug thereof.

35. The compound or a salt thereof, or a prodrug thereof, according to claim 34, wherein R¹ is hydrogen atom.

36. The compound or a salt thereof, or a prodrug thereof, according to claim 34, wherein R² is hydrogen atom or a lower alkyl group which may optionally be substituted.

37. The compound or a salt thereof, or a prodrug thereof, according to claim 34, wherein Ar¹ is a monocyclic aromatic group which may optionally be substituted.

38. The compound or a salt thereof, or a prodrug thereof, according to claim 34, wherein Ar¹ is a phenyl group which may optionally be substituted.

39. The compound or a salt thereof, or a prodrug thereof, according to claim 34, wherein ring A is unsubstituted benzene ring.

40. The compound or a salt thereof, or a prodrug thereof, according to claim 34, wherein X is NR⁵ (wherein R⁵ has the same significance as defined in claim 34) and Y is CH.

41. The compound or a salt thereof, or a prodrug thereof, according to claim 34, wherein R¹ is hydrogen atom, R² is methyl, Ar¹ is a phenyl group which may optionally be substituted, ring A is unsubstituted benzene ring, X is NR⁵ (wherein R⁵ has the same significance as defined in claim 34) and Y is CH N (provided that those wherein X is NH, Y is CH and Ar¹ is unsubstituted phenyl group are excluded).

42. A pharmaceutical composition comprising the compound or a salt thereof, or a prodrug thereof, according to claim 34.

43. A process of manufacturing a compound represented by formula: 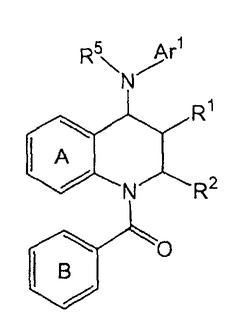 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; R⁵ represents hydrogen atom or a lower alkyl group which may optionally be substituted; ring A represents a benzene ring which may optionally be substituted; ring B represents a benzene ring, which may optionally be substituted with an optionally halogenated C₁₋₆ alkoxy group, hydroxy group or a C₁₋₃ alkylenedioxy group; and, Ar¹ represents an aromatic group which may optionally be substituted (provided that the compounds wherein R⁵ is H and Ar¹ is unsubstituted phenyl group are excluded)] or a salt thereof, or a prodrug thereof, which comprises substituting a compound represented by formula: 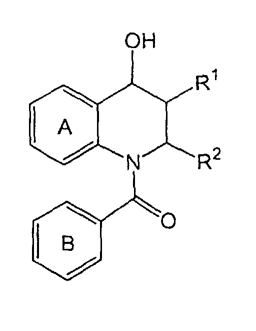 [wherein R¹, R², ring A and ring B have the same significance as defined above] or a salt thereof, with a compound represented by formula:
Ar¹ - NR⁵H
[wherein Ar¹ and R⁵ have the same significance as defined above] or a salt thereof.

44. A process of manufacturing a compound represented by formula: 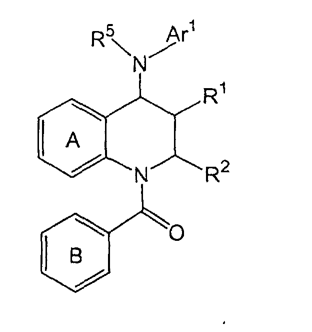 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; R⁵ represents hydrogen atom or a lower alkyl group which may optionally be substituted; ring A represents a benzene ring which may optionally be substituted; ring B represents a benzene ring, which may optionally be substituted with an optionally halogenated C₁₋₆ alkoxy group, hydroxy group or a C₁₋₃ alkylenedioxy group; and, Ar¹ represents an aromatic group which may optionally be substituted (provided that the compounds wherein R⁵ is H and Ar¹ is unsubstituted phenyl group are excluded)] or a salt thereof, or a prodrug thereof, which comprises reacting a compound represented by formula: 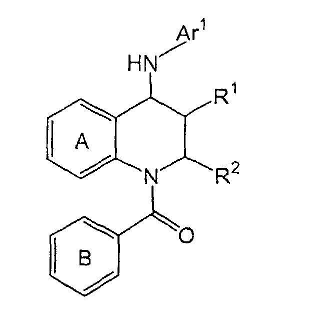 [wherein R¹, R², Ar¹, ring A and ring B have the same significance as defined above] or a salt thereof, with a compound represented by formula:
R⁵Z
[wherein R⁵ has the same significance as defined above and Z represents a reactive substituent] or a salt thereof.

45. A process of manufacturing a compound represented by formula (III): 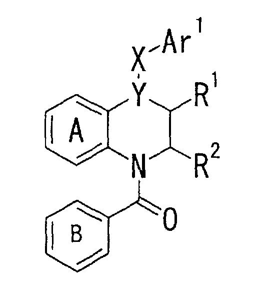 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; Ar¹ represents an aromatic group which may optionally be substituted; X represents CR³R⁴ or NR⁵ (wherein R³, R⁴ or R⁵, which may be the same or different, each represents hydrogen atom or a lower alkyl group which may optionally be substituted); Y represents CH or N (provided that the compounds wherein X is NH, Y is CH and Ar¹ is unsubstituted phenyl group are excluded); ring A represents a benzene ring which may optionally be substituted; and ring B represents a benzene ring, which may optionally be substituted with an optionally halogenated C₁₋₆ alkoxy group, hydroxy group or a C₁₋₃ alkylenedioxy group] or a salt thereof, or a prodrug thereof, which comprises reacting a compound represented by formula: 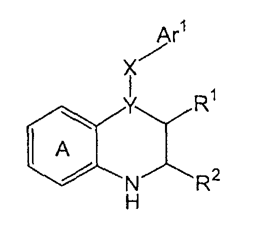 [wherein R¹, R², Ar¹, X, Y and ring A have the same significance as defined above] or a salt thereof, with a compound represented by formula: 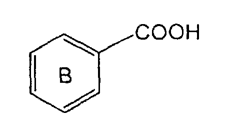 [wherein ring B has the same significance as defined above] or a salt thereof.

46. A process of manufacturing a compound represented by formula: 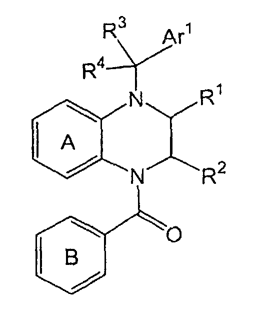 [wherein R¹ and R², which may be the same or different, each represents hydrogen atom or a lower alkyl group which may optionally be substituted, or R¹ and R² are combined to form a 4- to 7-membered ring together with a carbon atom adjacent thereto; R³ and R⁴, which may be the same or different, each represents hydrogen atom or a lower alkyl group which may optionally be substituted; ring A represents a benzene ring which may optionally be substituted; ring B represents a benzene ring, which may optionally be substituted with an optionally halogenated C₁₋₆ alkoxy group, hydroxy group or a C₁₋₃ alkylenedioxy group; and Ar¹ represents an aromatic group which may optionally be substituted] or a salt thereof, or a prodrug thereof, which comprises reacting a compound represented by formula: 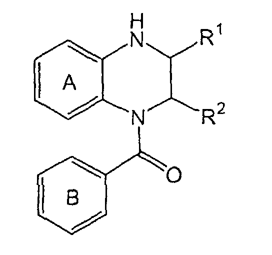 [wherein R¹, R², ring A and ring B have the same significance as defined above] or a salt thereof, with a compound represented by formula:
Ar¹CR³R⁴Z
[wherein Ar¹, R³ and R⁴ have the same significance as defined above; and Z represents a reactive substituent] or a salt thereof.
